# EUROPEAN PATENT SPECIFICATION

(11) **EP 3 445 388 B1**
(45) Date of publication and mention of the grant of the patent: **17.04.2024**
(21) Application number: 17727373.7
(22) Date of filing: 18.04.2017
(51) Int. Cl.: A61K 38/46, C12N 9/22, C12N 15/10, C12N 15/113

(54) **MATERIALS AND METHODS FOR TREATMENT OF HEMOGLOBINOPATHIES**
MATERIALIEN UND VERFAHREN ZUR BEHANDLUNG VON HÄMOGLOBINOPATHIEN
SUBSTANCES ET MÉTHODES POUR LE TRAITEMENT D'HÉMOGLOBINOPATHIES

(30) Priority: 18.04.2016 US 201662324024 P; 01.09.2016 US 201662382522 P; 02.12.2016 US 201662429428 P
(43) Date of publication of application: 27.02.2019
(73) Proprietor: Vertex Pharmaceuticals Incorporated, Boston, MA 02210 (US)
(72) Inventor: COWAN, Chad, Albert, Cambridge, MA 02139 (US); LUNDBERG, Ante, Sven, Cambridge, MA 02139 (US); CHAKRABORTY, Tirtha, Cambridge, MA 02139 (US); LIN, Michelle, I-ching, Cambridge, MA 02139 (US); MISHRA, Bibhu, Prasad, Cambridge, MA 02139 (US); PAIK, Elizabeth, Jae-eun, Cambridge, MA 02139 (US); KERNYTSKY, Andrew, Cambridge, MA 02139 (US); BORLAND, Todd, Douglass, Cambridge, MA 02139 (US)
(74) Representative: Vossius & Partner Patentanwälte Rechtsanwälte mbB
(86) International application number: PCT/IB2017/000577
(87) International publication number: WO 2017/182881

(56) References cited:
- WO-A1-2015/183025
- WO-A2-2015/168547
- US-A1- 2015 044 772
- US-A1- 2015 166 969
- US-A1- 2015 307 867
- US-A1- 2016 029 604
- MATTHEW C. CANVER ET AL: "BCL11A enhancer dissection by Cas9-mediated in situ saturating mutagenesis", NATURE, vol. 527, no. 7577, 1 November 2015 (2015-11-01), pages 192-197, XP55274680, ISSN: 0028-0836, DOI: 10.1038/nature15521
- CANVER MATTHEW C ET AL: "Customizing the genome as therapy for the beta-hemoglobinopathies", 6 April 2016 (2016-04-06), BLOOD, VOL. 127, NR. 21, PAGE(S) 2536-2545, XP002771817, ISSN: 0006-4971(print) page 2539, column 1, paragraph 3 - page 2539, column 2, paragraph 4; figure 1 page 2541, column 1, paragraph 3
- BAUER DANIEL E ET AL: "Hemoglobin switching's surprise: the versatile transcription factor BCL11A is a master repressor of fetal hemoglobin", CURRENT OPINION IN GENETICS & DEVELOPMENT, vol. 33, 14 September 2015 (2015-09-14), pages 62-70, XP029324656, 14.09.2015 ISSN: 0959-437X, DOI: 10.1016/J.GDE.2015.08.001
- STUART H. ORKIN: "Recent advances in globin research using genome-wide association studies and gene editing", ANNALS OF THE NEW YORK ACADEMY OF SCIENCES, vol. 1368, no. 1, 11 February 2016 (2016-02-11), pages 5-10, XP055274553, US ISSN: 0077-8923, DOI: 10.1111/nyas.13001
- LI HOJUN ET AL: "Genome Editing in Erythroid Progenitor Cells Mediated By Crispr/Cas9", BLOOD, vol. 124, no. 21, December 2014 (2014-12), XP002771899, & 56TH ANNUAL MEETING OF THE AMERICAN-SOCIETY-OF-HEMATOLOGY; SAN FRANCISCO, CA, USA; DECEMBER 06 -09, 2014
- DANIEL E BAUER: "Blood Journal | Fine-Mapping and Genome Editing Reveal An Essential Erythroid Enhancer At The HbF-Associated BCL11A Locus", BLOOD, vol. 122, no. 21, 15 November 2013 (2013-11-15), pages 437-437, XP055203587, ISSN: 0006-4971
- BAUER DANIEL E ET AL: "Crispr-Cas9 Saturating Mutagenesis Reveals an Achilles Heel in the BCL11A Erythroid Enhancer for Fetal Hemoglobin Induction (by Genome Editing)", BLOOD, vol. 126, no. 23, December 2015 (2015-12), XP002771900, & 57TH ANNUAL MEETING OF THE AMERICAN-SOCIETY-OF-HEMATOLOGY; ORLANDO, FL, USA; DECEMBER 05 -08, 2015
- D. E. BAUER ET AL: "An Erythroid Enhancer of BCL11A Subject to Genetic Variation Determines Fetal Hemoglobin Level", SCIENCE, vol. 342, no. 6155, 11 October 2013 (2013-10-11), pages 253-257, XP055203754, ISSN: 0036-8075, DOI: 10.1126/science.1242088
- BLOBEL GERD A ET AL: "An international effort to cure a global health problem: A report on the 19th Hemoglobin Switching Conference", EXPERIMENTAL HEMATOLOGY, vol. 43, no. 10, 1 October 2015 (2015-10-01), pages 821-837, XP029284406, ISSN: 0301-472X, DOI: 10.1016/J.EXPHEM.2015.06.008
- LI HOJUN ET AL: "Efficient CRISPR-Cas9 mediated gene disruption in primary erythroid progenitor cells.", HAEMATOLOGICA JUN 2016, vol. 101, no. 6, 11 March 2016 (2016-03-11), pages e216-e219, XP002773432, ISSN: 1592-8721

## Description

### Field

The present description provides materials and methods for treating patients with hemoglobinopathies, both ex *vivo* and *in vivo.* In addition, the present description provides materials and methods for deleting, modulating, or inactivating a transcriptional control sequence of a B-cell lymphoma 11A (BCL1 1A) gene in a cell by genome editing.

### Sequence Listing

This patent contains a Sequence Listing in computer readable form (filename: 160077PCT Sequence Listing; 14,446,299 bytes - ASCII text file; created April 7, 2017).

### Background

Hemoglobinopathies include anemias of genetic origin, which result in decreased production and/or increased destruction of red blood cells. These disorders also include genetic defects, which result in the production of abnormal hemoglobins with an associated inability to maintain oxygen concentration. Many of these disorders are referred to as β-hemoglobinopathies because of their failure to produce normal β-globin protein in sufficient amounts or failure to produce normal β-globin protein entirely. For example, β-thalassemias result from a partial or complete defect in the expression of the β-globin gene, leading to deficient or absent adult hemogloblin (HbA). Sickle cell anemia results from a point mutation in the β-globin structural gene, leading to the production of an abnormal hemoglobin (HbS) (Atweh, Semin. Hematol. 38(4):367-73 (2001)). Hemoglobinopathies result in a reduction in the oxygen carrying capacity of the blood, which can lead to symptoms such as weariness, dizziness, and shortness of breath, particularly when exercising.

For patients diagnosed with a hemoglobinopathy, currently only a few symptomatic treatments are available, such as a blood transfusion, to increase blood oxygen levels.

Genome engineering refers to the strategies and techniques for the targeted, specific modification of the genetic information (genome) of living organisms. Genome engineering is a very active field of research because of the wide range of possible applications, particularly in the areas of human health; the correction of a gene carrying a harmful mutation, for example, or to explore the function of a gene. Early technologies developed to insert a transgene into a living cell were often limited by the random nature of the insertion of the new sequence into the genome. Random insertions into the genome may result in disrupting normal regulation of neighboring genes leading to severe unwanted effects. Furthermore, random integration technologies offer little reproducibility, as there is no guarantee that the sequence would be inserted at the same place in two different cells. Recent genome engineering strategies, such as ZFNs, TALENs, HEs and MegaTALs, enable a specific area of the DNA to be modified, thereby increasing the precision of the correction or insertion compared to early technologies. These newer platforms offer a much larger degree of reproducibility, but still have their limitations.

Despite efforts from researchers and medical professionals worldwide who have been trying to address hemoglobinopathies, there still remains a critical need for developing safe and effective treatments for hemoglobinopathies.

### Summary

The present disclosure presents an approach to address the genetic basis of hemoglobinopathies. By using genome engineering tools to create permanent changes to the genome that can delete, modulate, or inactivate a transcriptional control sequence of the BCL11A gene with a single treatment, the resulting therapy may ameliorate the effects of hemoglobinopathies.

The present invention is defined by the enclosed claims.

Provided herein are cellular, *ex vivo* and *in vivo* methods for creating permanent changes to the genome by deleting, modulating, or inactivating a transcriptional control sequence of the BCL11A gene, which can be used to treat hemoglobinopathies. Also provided herein are components, kits, and compositions for performing such methods. Also described are cells produced by such methods. Examples of hemoglobinopathies can be sickle cell anemia and thalassemia (α, β, δ, γ, and combinations thereof).

Provided herein is a method for editing a B-cell lymphoma 11A (BCL1 1A) gene in a human cell by genome editing, the method comprising the step of introducing into the human cell one or more deoxyribonucleic acid (DNA) endonucleases to effect one or more single-strand breaks (SSBs) or double-strand breaks (DSBs), within or near the BCL11A gene or other DNA sequence that encodes a regulatory element of the BCL1 1A gene, that results in a permanent deletion, modulation, or inactivation of a transcriptional control sequence of the BCL11A gene. The transcriptional control sequence can be located within a second intron of the BCL1 1A gene. The transcriptional control sequence can be located within a +58 DNA hypersensitive site (DHS) of the BCL11A gene.

Also described herein is an ex *vivo* method for treating a patient (e.g., a human) with a hemoglobinopathy, the method comprising the steps of: creating a patient specific induced pluripotent stem cell (iPSC); editing within or near a BCL11A gene or other DNA sequence that encodes a regulatory element of the BCL11A gene of the iPSC; differentiating the genome-edited iPSC into a hematopoietic progenitor cell; and implanting the hematopoietic progenitor cell into the patient.

The step of creating a patient specific induced pluripotent stem cell (iPSC) can comprise: isolating a somatic cell from the patient; and introducing a set of pluripotency-associated genes into the somatic cell to induce the somatic cell to become a pluripotent stem cell. The somatic cell can be a fibroblast. The set of pluripotency-associated genes can be one or more of the genes selected from the group consisting of OCT4, SOX2, KLF4, Lin28, NANOG and cMYC.

The step of editing within or near a BCL11A gene or other DNA sequence that encodes a regulatory element of the BCL11A gene of the iPSC can comprise introducing into the iPSC one or more deoxyribonucleic acid (DNA) endonucleases to effect one or more single-strand breaks (SSBs) or double-strand breaks (DSBs) within or near the BCL11A gene or other DNA sequence that encodes a regulatory element of the BCL11A gene that results in a permanent deletion, modulation, or inactivation of a transcriptional control sequence of the BCL11A gene.

The step of differentiating the genome-edited iPSC into a hematopoietic progenitor cell can comprise one or more of the following: treatment with a combination of small molecules, delivery of transcription factors (e.g., master transcription factors), or delivery of mRNA encoding transcription factors (e.g., master transcription factors).

The step of implanting the hematopoietic progenitor cell into the patient can comprise implanting the hematopoietic progenitor cell into the patient by transplantation, local injection, systemic infusion, or combinations thereof.

Also described herein is an *ex vivo* method for treating a patient (e.g., a human) with a hemoglobinopathy, the method comprising the steps of: isolating a mesenchymal stem cell from the patient; editing within or near a BCL11A gene or other DNA sequence that encodes a regulatory element of the BCL11A gene of the mesenchymal stem cell; differentiating the genome-edited mesenchymal stem cell into a hematopoietic progenitor cell; and implanting the hematopoietic progenitor cell into the patient.

The mesenchymal stem cell can be isolated from the patient's bone marrow or peripheral blood. The step of isolating a mesenchymal stem cell from the patient can comprise aspiration of bone marrow and isolation of mesenchymal cells using density gradient centrifugation media.

The step of editing within or near the BCL11A gene or other DNA sequence that encodes a regulatory element of the BCL11A gene of the mesenchymal stem cell can comprise introducing into the mesenchymal stem cell one or more deoxyribonucleic acid (DNA) endonucleases to effect one or more single-strand breaks (SSBs) or double-strand breaks (DSBs) within or near the BCL11A gene or other DNA sequence that encodes a regulatory element of the BCL11A gene that results in a permanent deletion, modulation, or inactivation of a transcriptional control sequence of the BCL11A gene.

The step of differentiating the genome-edited mesenchymal stem cell into a hematopoietic progenitor cell can comprise one or more of the following: treatment with a combination of small molecules, delivery of transcription factors (e.g., master trascription factors) or delivery of mRNA encoding transcription factors (e.g., master transcription factors).

The step of implanting the hematopoietic progenitor cell into the patient can comprise implanting the hematopoietic progenitor cell into the patient by transplantation, local injection, systemic infusion, or combinations thereof.

Also described herein is an *ex vivo* method for treating a patient (e.g., a human) with a hemoglobinopathy, the method comprising the steps of: isolating a hematopoietic progenitor cell from the patient; editing within or near a BCL11A gene or other DNA sequence that encodes a regulatory element of the BCL11A gene of the hematopoietic progenitor cell; and implanting the genome-edited hematopoietic progenitor cell into the patient.

The method can further comprise treating the patient with granulocyte colony stimulating factor (GCSF) prior to the step of isolating a hematopoietic progenitor cell from the patient. The step of treating the patient with granulocyte colony stimulating factor (GCSF) can be performed in combination with Plerixaflor.

The step of isolating a hematopoietic progenitor cell from the patient can comprise isolating CD34+ cells.

The step of editing within or near a BCL11A gene or other DNA sequence that encodes a regulatory element of the BCL11A gene of the hematopoietic progenitor cell can comprise introducing into the hematopoietic progenitor cell one or more deoxyribonucleic acid (DNA) endonucleases to effect one or more single-strand breaks (SSBs) or double-strand breaks (DSBs) within or near the BCL11A gene or other DNA sequence that encodes a regulatory element of the BCL11A gene that results in a permanent deletion, modulation, or inactivation of a transcriptional control sequence of the BCL11A gene.

The step of implanting the genome-edited hematopoietic progenitor cell into the patient can comprise implanting the genome-edited hematopoietic progenitor cell into the patient by transplantation, local injection, systemic infusion, or combinations thereof.

Also described herein is an *in vivo* method for treating a patient (e.g., a human) with a hemoglobinopathy, the method comprising the step of editing a BCL11A gene in a cell of the patient.

The step of editing a BCL11A gene in a cell of the patient can comprise introducing into the cell one or more deoxyribonucleic acid (DNA) endonucleases to effect one or more single-strand breaks (SSBs) or double-strand breaks (DSBs) within or near the BCL11A gene or other DNA sequence that encodes a regulatory element of the BCL11A gene that results in a permanent deletion, modulation, or inactivation of a transcriptional control of the BCL11A gene. The cell can be a bone marrow cell, a hematopoietic progenitor cell, a CD34+ cell, or combinations thereof.

The one or more DNA endonucleases can be a Cas1, Cas1B, Cas2, Cas3, Cas4, Cas5, Cas6, Cas7, Cas8, Cas9 (also known as Csn1 and Csx12), Cas100, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, or Cpf1 endonuclease; a homolog thereof, a recombination of the naturally occurring molecule thereof, codon-optimized thereof, or modified versions thereof, and combinations thereof.

The method can comprise introducing into the cell one or more polynucleotides encoding the one or more DNA endonucleases. The method can comprise introducing into the cell one or more ribonucleic acids (RNAs) encoding the one or more DNA endonucleases. The one or more polynucleotides or one or more RNAs can be one or more modified polynucleotides or one or more modified RNAs. The one or more DNA endonucleases can be one or more proteins or polypeptides. The one or more proteins or polypeptides can be flanked at the N-terminus, the C-terminus, or both the N-terminus and C-terminus by one or more nuclear localization signals (NLSs). The one or more proteins or polypeptides can be flanked by two NLSs, one NLS located at the N-terminus and the second NLS located at the C-terminus. The one or more NLSs can be a SV40 NLS.

The method can further comprise introducing into the cell one or more guide ribonucleic acids (gRNAs). The one or more gRNAs can be single-molecule guide RNA (sgRNAs). The one or more gRNAs or one or more sgRNAs can be one or more modified gRNAs, one or more modified sgRNAs, or combinations thereof. The one or more modified sgRNAs can comprise three 2'-O-methyl-phosphorothioate residues at or near each of its 5' and 3' ends. The modified sgRNA can be the nucleic acid sequence of SEQ ID NO: 71,959. The one or more DNA endonucleases can be pre-complexed with one or more gRNAs, one or more sgRNAs, or combinations thereof to form one or more ribonucleoproteins (RNPs). The weight ratio of sgRNA to DNA endonuclease in the RNP can be 1:1. The sgRNA can comprise the nucleic acid sequence of SEQ ID NO: 71,959, the DNA endonuclease can be a S. *pyogenes* Cas9 comprising a N-terminus SV40 NLS and a C-terminus SV40 NLS, and the weight ratio of sgRNA to DNA endonuclease can be 1:1. US2015044772 which relates to a chimeric fusion Cas protein discloses the nucleic acid sequence of SEQ ID NO: 71,959. Said sequence is also disclosed in WO2015183025 which relates to a method for analyzing a genotype using a CRISPR/Cas system.

The method can further comprise introducing into the cell a polynucleotide donor template comprising a wild-type BCL11A gene or cDNA comprising a modified transcriptional control sequence.

The method can further comprise introducing into the cell one guide ribonucleic acid (gRNA) and a polynucleotide donor template comprising a wild-type BCL11A gene or cDNA comprising a modified transcriptional control sequence. The one or more DNA endonucleases can be one or more Cas9 or Cpf1 endonucleases that effect one single-strand break (SSB) or double-strand break (DSB) at a locus within or near the BCL11A gene or other DNA sequence that encodes a regulatory element of the BCL11A gene that facilitates insertion of a new sequence from the polynucleotide donor template into the chromosomal DNA at the locus that results in a permanent insertion, modulation, or inactivation of the transcriptional control sequence of the chromosomal DNA proximal to the locus. The gRNA can comprise a spacer sequence that is complementary to a segment of the locus. Proximal can mean nucleotides both upstream and downstream of the locus.

The method can further comprise introducing into the cell one or more guide ribonucleic acid (gRNAs) and a polynucleotide donor template comprising a wild-type BCL11A gene or cDNA comprising a modified transcriptional control sequence. The one or more DNA endonucleases can be one or more Cas9 or Cpf1 endonucleases that effect or create a pair of single-strand breaks (SSBs) and/or double-strand breaks (DSBs), the first break at a 5' locus and the second break at a 3' locus, within or near the BCL11A gene or other DNA sequence that encodes a regulatory element of the BCL11A gene, that facilitates insertion of a new sequence from the polynucleotide donor template into the chromosomal DNA between the 5' locus and the 3' locus that results in a permanent insertion, modulation, or inactivation of the transcriptional control sequence of the chromosomal DNA between the 5' locus and the 3' locus. One guide RNA can create a pair of SSBs or DSBs. The one guide RNA can comprise a spacer sequence that is complementary to either the 5' locus or the 3' locus. Alternatively, the method may comprise a first guide RNA and a second guide RNA. The first guide RNA can comprise a spacer sequence that is complementary to a segment of the 5' locus and the second guide RNA can comprise a spacer sequence that is complementary to a segment of the 3' locus. The donor template can be either single or double stranded. The modified transcriptional control sequence can be located within a second intron of the BCL11A gene. The modified transcriptional control sequence can be located within a +58 DNA hypersensitive site (DHS) of the BCL11A gene.

The one or two gRNAs can be one or two single-molecule guide RNA (sgRNAs). The one or two gRNAs or one or two sgRNAs can be one or two modified gRNAs or one or two modified sgRNAs. The one modified sgRNA can comprise three 2'-O-methyl-phosphorothioate residues at or near each of its 5' and 3' ends. The one modified sgRNA can be the nucleic acid sequence of SEQ ID NO: 71,959. The one or more Cas9 endonucleases can be pre-complexed with one or two gRNAs or one or two sgRNAs to form one or more ribonucleoproteins (RNPs). The one or more Cas9 endonuclease can be flanked at the N-terminus, the C-terminus, or both the N-terminus and C-terminus by one or more nuclear localization signals (NLSs). The one or more Cas9 endonucleases can be flanked by two NLSs, one NLS located at the N-terminus and the second NLS located at the C-terminus. The one or more NLSs can be a SV40 NLS. The weight ratio of sgRNA to Cas9 endonuclease in the RNP can be 1:1. The one sgRNA can comprise the nucleic acid sequence of SEQ ID NO: 71,959, the Cas9 endonuclease can be a *S*. *pyogenes* Cas9 comprising a N-terminus SV40 NLS and a C-terminus SV40 NLS, and the weight ratio of sgRNA to Cas9 endonuclease can be 1:1.

The insertion can be by homology directed repair (HDR).

The SSB, DSB, 5' locus, and/or 3' locus can be located within a second intron of the BCL11A gene. The SSB, DSB, 5' locus, and/or 3' locus can be located within a +58 DNA hypersensitive site (DHS) of the BCL11A gene.

The method can further comprise introducing into the cell one or more guide ribonucleic acids (gRNAs). The one or more DNA endonucleases can be one or more Cas9 or Cpf1 endonucleases that effect or create a pair of single-strand breaks (SSBs) or double-strand breaks (DSBs), the first SSB or DSB at a 5' locus and a second SSB or DSB at a 3' locus, within or near the BCL11A gene or other DNA sequence that encodes a regulatory element of the BCL11A gene that causes a deletion of the chromosomal DNA between the 5' locus and the 3' locus that results in a permanent deletion, modulation, or inactivation of the transcriptional control sequence of the chromosomal DNA between the 5' locus and the 3' locus. The first guide RNA can comprise a spacer sequence that is complementary to a segment of the 5' locus and the second guide RNA comprises a spacer sequence that is complementary to a segment of the 3' locus. One guide RNA can create a pair of SSBs or DSBs. The one guide RNA can comprise a spacer sequence that is complementary to either the 5' locus or the 3' locus. Alternatively, the method may comprise a first guide RNA and a second guide RNA. The first guide RNA can comprise a spacer sequence that is complementary to a segment of the 5' locus and the second guide RNA can comprise a spacer sequence that is complementary to a segment of the 3' locus.

The one or more gRNAs can be one or more single-molecule guide RNA (sgRNAs). The one or more gRNAs or one or more sgRNAs can be one or more modified gRNAs or one or more modified sgRNAs. The one modified sgRNA can comprise three 2'-O-methyl-phosphorothioate residues at or near each of its 5' and 3' ends. The one modified sgRNA can be the nucleic acid sequence of SEQ ID NO: 71,959. The one or more Cas9 endonucleases can be pre-complexed with one or more gRNA or one or more sgRNA to form one or more ribonucleoproteins (RNPs). The one or more Cas9 endonuclease can be flanked at the N-terminus, the C-terminus, or both the N-terminus and C-terminus by one or more nuclear localization signals (NLSs). The one or more Cas9 endonucleases can be flanked by two NLSs, one NLS located at the N-terminus and the second NLS located at the C-terminus. The one or more NLSs can be a SV40 NLS. The weight ratio of sgRNA to Cas9 endonuclease in the RNP can be 1:1. The one sgRNA can comprise the nucleic acid sequence of SEQ ID NO: 71,959, the Cas9 endonuclease can be a *S*. *pyogenes* Cas9 comprising a N-terminus SV40 NLS and a C-terminus SV40 NLS, and the weight ratio of sgRNA to Cas9 endonuclease can be 1:1.

The 5' locus and/or 3' locus can be located within a second intron of the BCL11A gene. The 5' locus and/or 3' locus can be located within a +58 DNA hypersensitive site (DHS) of the BCL11A gene.

The Cas9 or Cpf1 mRNA, gRNA, and donor template can be formulated into separate lipid nanoparticles or co-formulated into a lipid nanoparticle.

The Cas9 or Cpf1 mRNA can be formulated into a lipid nanoparticle, and the gRNA and donor template can be delivered to the cell by an adeno-associated virus (AAV) vector.

The Cas9 or Cpf1 mRNA can be formulated into a lipid nanoparticle, and the gRNA can be delivered to the cell by electroporation and donor template can be delivered to the cell by an adeno-associated virus (AAV) vector.

The one or more RNP can be delivered to the cell by electroporation.

The editing within or near a BCL11A gene or other DNA sequence that encodes a regulatory element of the BCL11A gene can reduce BCL11A gene expression.

The BCL11A gene can be located on Chromosome 2: 60,451,167 - 60,553,567 (Genome Reference Consortium - GRCh38).

Also provided herein are one or more guide ribonucleic acids (gRNAs) for editing a BCL11A gene in a cell from a patient with a hemoglobinopathy. The one or more gRNAs can comprise a spacer sequence selected from the group consisting of nucleic acid sequences in SEQ ID NOs: 1 - 71,947 of the Sequence Listing. The one or more gRNAs can be one or more single-molecule guide RNAs (sgRNAs). The one or more gRNAs or one or more sgRNAs can be one or more modified gRNAs or one or more modified sgRNAs. The one or more modified sgRNAs can comprise three 2'-O-methyl-phosphorothioate residues at or near each of its 5' and 3' ends. The one or more modified sgRNAs can comprise the nucleic acid sequence of SEQ ID NO: 71,959. Also provided herein is a single-molecule guide RNA (sgRNA) comprising the nucleic acid sequence of SEQ ID NO: 71,959.

It is understood that the inventions described in this specification are not limited to the examples summarized in this Summary. Various other aspects are described and exemplified herein.

### Brief Description of the Drawings

Various aspects of materials and methods for treatment of hemoglobinopathies disclosed and described in this specification can be better understood by reference to the accompanying figures, in which:
Figures 1A-C show plasmids comprising a codon optimized gene for *S*. *pyogenes* Cas9 endonuclease.
Figure 1A is a plasmid (CTx-1) comprising a codon optimized gene for *S*. *pyogenes* Cas9 endonuclease. The CTx-1 plasmid also comprises a gRNA scaffold sequence, which includes a 20 bp spacer sequence from the sequences listed in SEQ ID NOs: 1 - 29,482 of the Sequence Listing.
Figure 1B is a plasmid (CTx-2) comprising a different codon optimized gene for *S*. *pyogenes* Cas9 endonuclease. The CTx-2 plasmid also comprises a gRNA scaffold sequence, which includes a 20 bp spacer sequence from the sequences listed in SEQ ID NOs: 1 - 29,482 of the Sequence Listing.
Figure 1C is a plasmid (CTx-3) comprising yet another different codon optimized gene for *S*. *pyogenes* Cas9 endonuclease. The CTx-3 plasmid also comprises a gRNA scaffold sequence, which includes a 20 bp spacer sequence from the sequences listed in SEQ ID NOs: 1 - 29,482 of the Sequence Listing.
Figures 2A-B depict the type II CRISPR/Cas system.

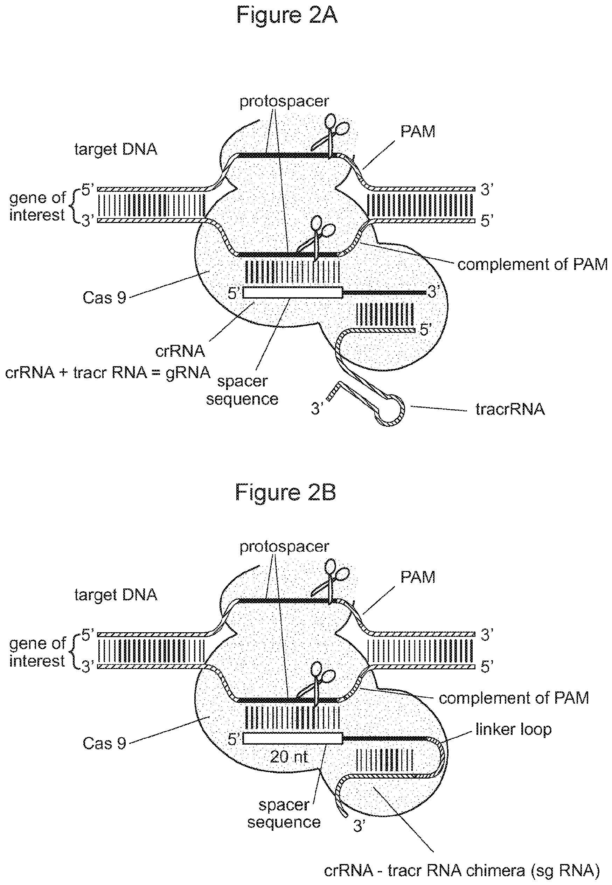

Figure 2A depicts the type II CRISPR/Cas system including gRNA.
Figure 2B depicts the type II CRISPR/Cas system including sgRNA.

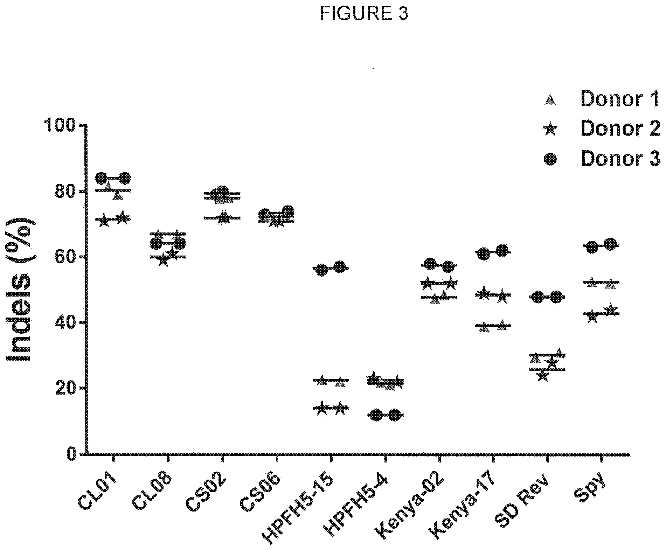

Figure 3 shows the rate of DNA editing in CD34+ hematopoietic stem and progenitor cells (HSPCs) and each of the different resulting HPFH genotypes.

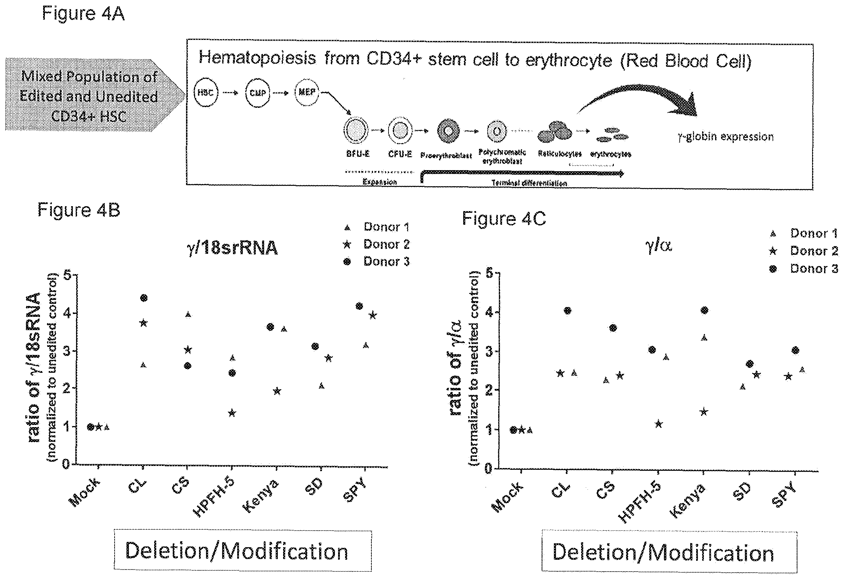

Figures 4A-C show the upregulation of γ-globin expression in erythrocytes differentiated from *Bulk* edited human CD34+ HSPCs from mobilized peripheral blood (mPB).
Figure 4A depicts hematopoiesis from human CD34+ HSPCs to erythrocytes.
Figure 4B shows the ratio of y/18sRNA for each of the deletion/modification.
Figure 4C shows the ratio of γ/α for each of the deletion/modification.

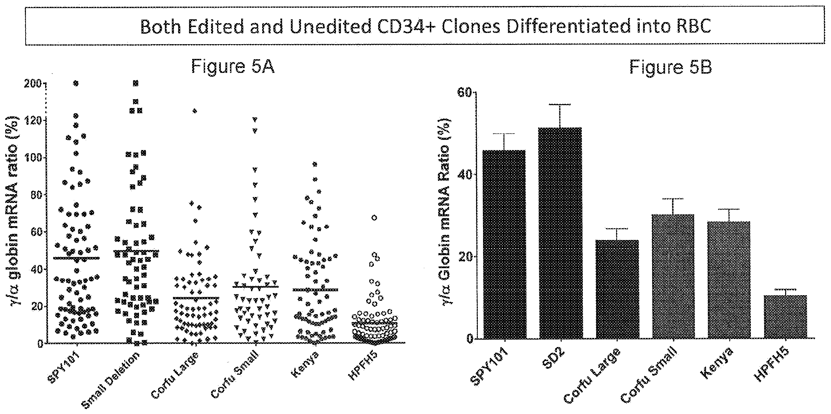

Figures 5A-B show the upregulation of γ-globin expression in erythrocytes differentiated from all gene-edited colonies from human CD34+ HSPCs.
Figure 5A shows the γ/α globin mRNA ratio (%) for each of the gene-edited colonies.
Figure 5B shows the average γ/α globin mRNA ratio (%) for each of the gene-modifications.

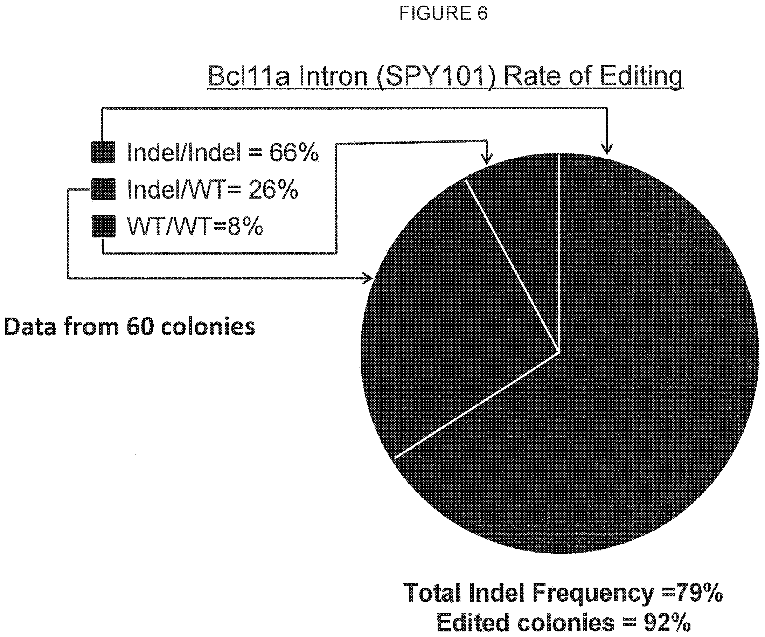

Figure 6 shows the BCL11A Intron (SPY101) rate of DNA editing in human CD34+ HSPC derived erythroid colonies.

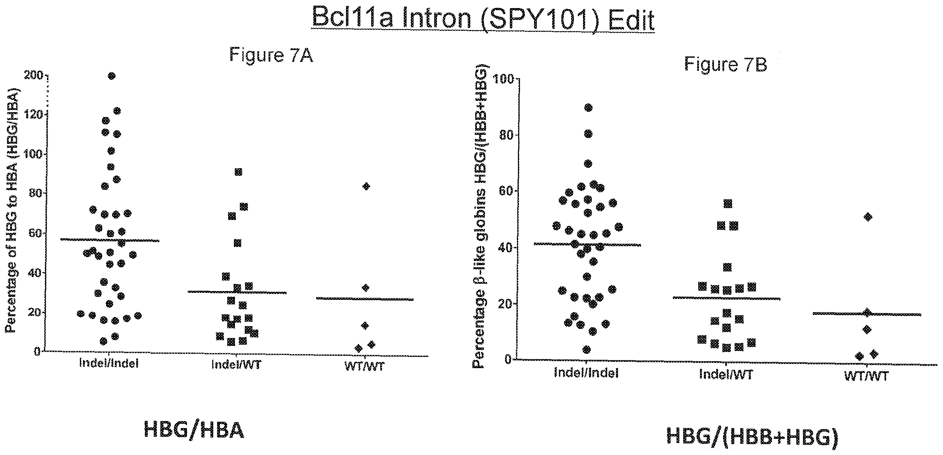

Figures 7A-B show the correlation between the SPY101 genotype and γ-globin expression in single cell colonies differentiated from gene-edited human mPB CD34+ HSPCs.
Figure 7A shows the percentage of γ-globin to α-globin (HBG/HBA) for each of the gene-edited colonies.
Figure 7B shows the percentage of β-like globins (HBG/(HBB+HBG)) for each of the gene-edited colonies.

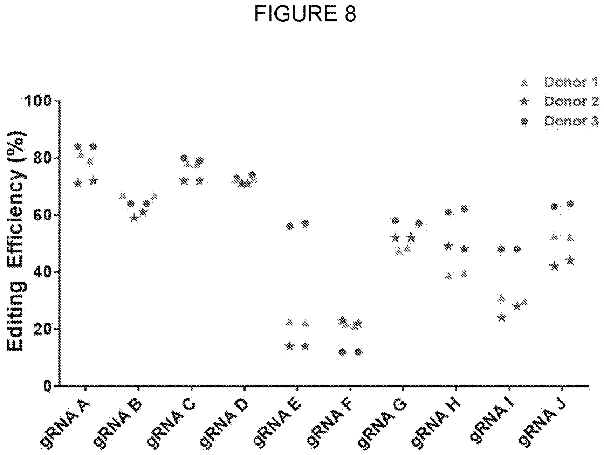

Figure 8 shows on-target editing efficacy of several gRNAs in human mPB CD34+ cells.

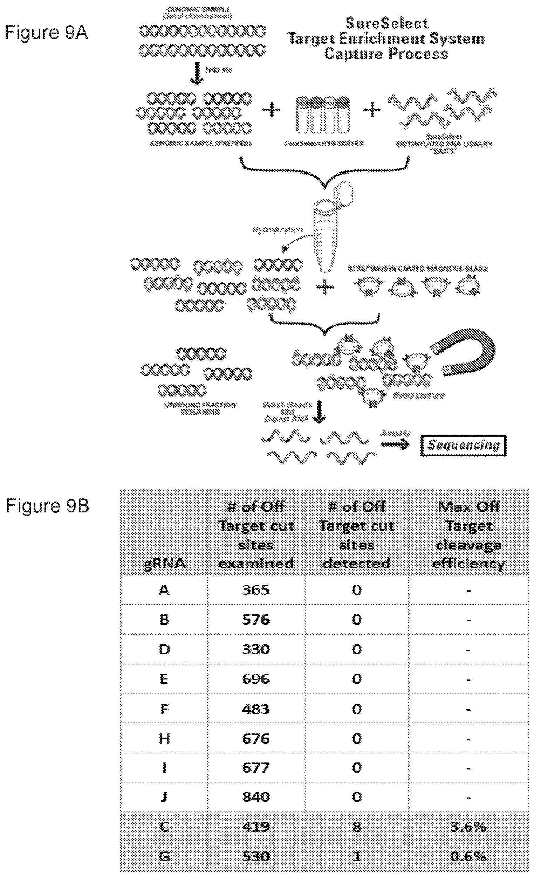

Figures 9A-B show the hybrid-capture assay used to detect off-target editing and results generated using the hybrid-capture assay from edited human mPB CD34+ HSPCs.
Figure 9A shows a schematic of a hybrid-capture assay used to detect editing activity at potential off-target sites.
Figure 9B shows observed off-target activity via hybrid capture sequencing.

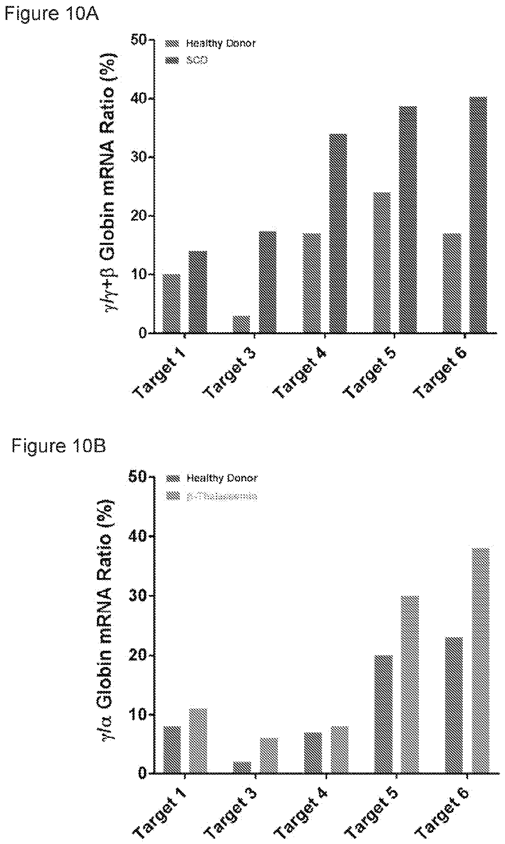

Figures 10A-B show ratios of globin mRNA levels measured in cells from SCD patients, a β-thalassemia patient, and healthy donors.
Figure 10A shows ratios of globin mRNA levels measured in cells from SCD patients compared to healthy donors.
Figure 10B shows ratios of globin mRNA levels measured in cells from a β-thalassemia patient compared to healthy donors.

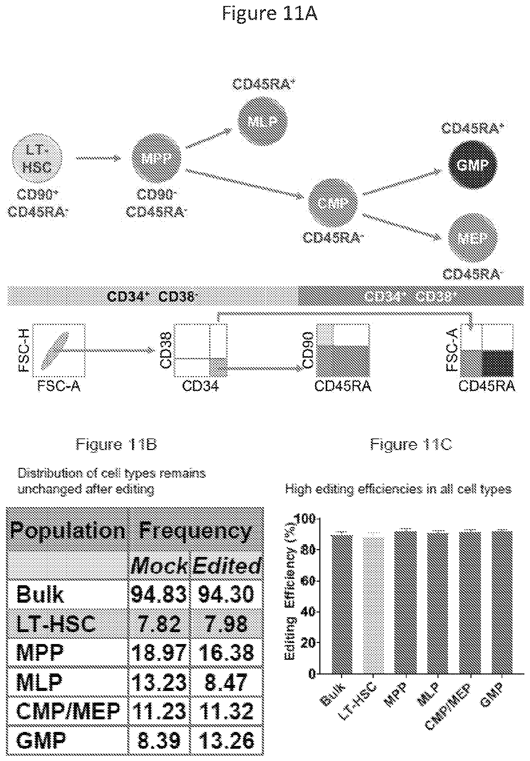

Figures 11A-C show the flow cytometry strategy used to detect various gene-edited cell populations and results generated using the flow cytometry strategy.
Figure 11A shows subpopulations of human mPB CD34+ HSPCs, associated surface markers, and flow cytometry gating strategy.
Figure 11B shows a similar distribution of cell types in the mock and edited conditions.
Figure 11C shows similar high editing efficiencies across the subpopulations compared to bulk.

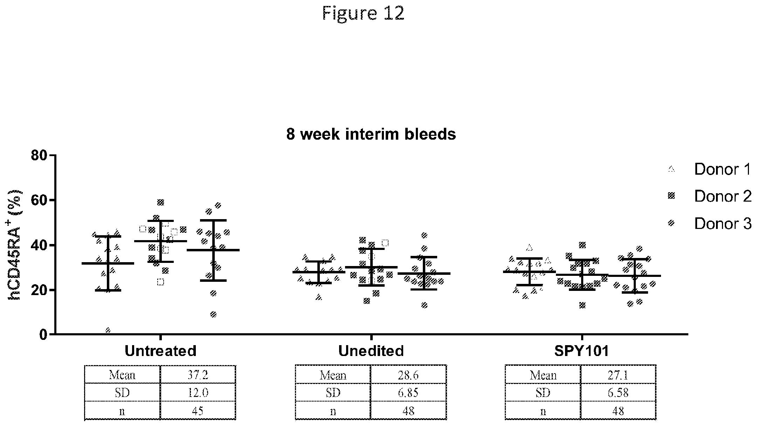

Figure 12 shows shows analysis of human CD45RA+ cell populations in NSG mice 8 weeks post-engraftment of human mPB CD34+ HSPCs. Data points represent individual animals and depict the percentage of live cells that were human CD45RA+ live cells.

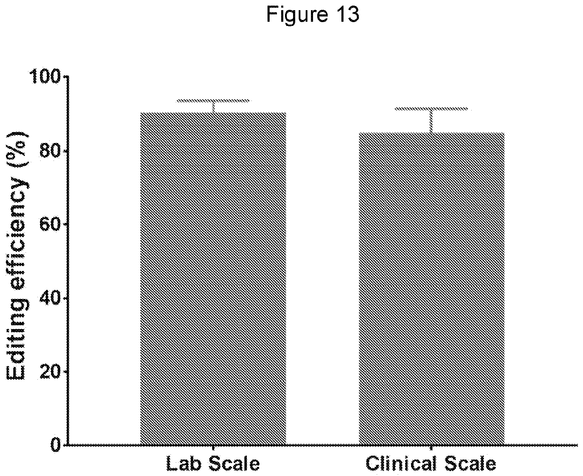

Figure 13 shows average editing efficacy of a SPY101 gRNA and Cas9 protein in human mPB CD34+ HSPCs at laboratory and clinically relevant scales.

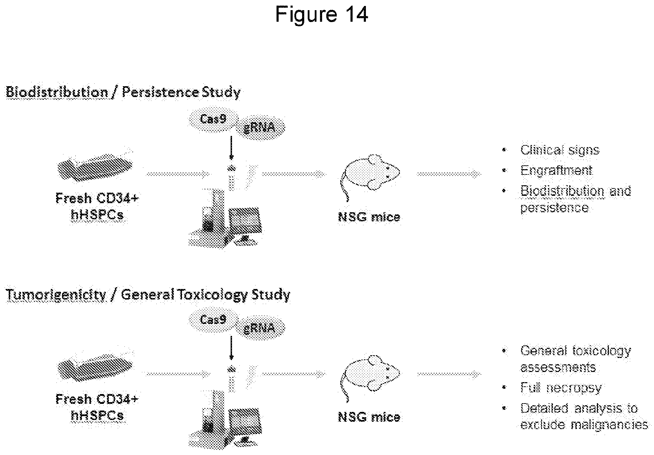

Figure 14 shows an overview of GLP/Toxicology study design.

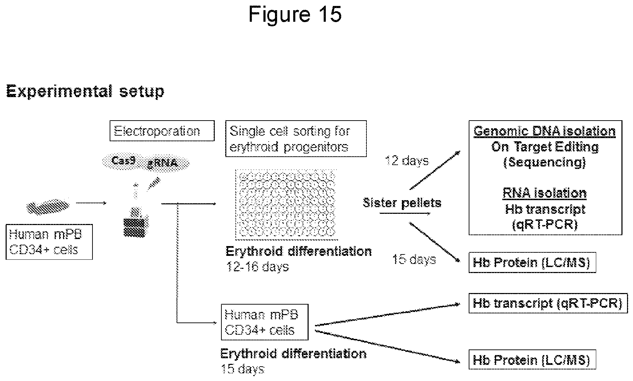

Figure 15 shows an overview of an experimental approach for bulk and single cell colony analysis of hemoglobin mRNA and protein levels in erythroid cell populations derived from CRISPR/Cas9 gene edited human mPB CD34+ HSPCs.

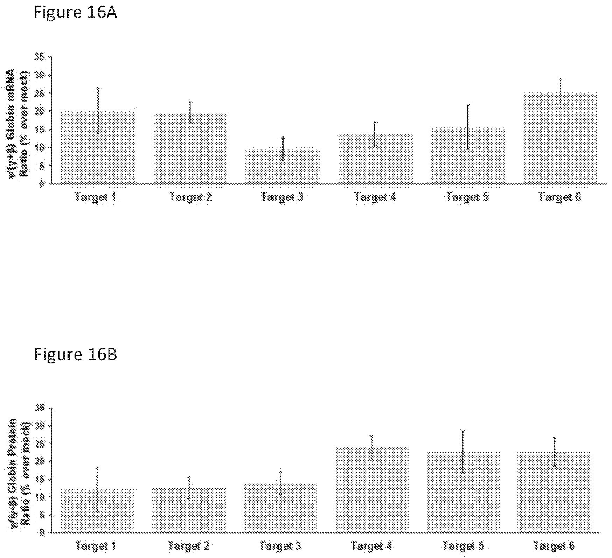

Figures 16A-B show γ-globin mRNA and protein upregulation in bulk differentiated human mPB CD34+ HSPCs modified with different targeted edits.
Figure 16A shows γ-globin mRNA upregulation in bulk differentiated human mPB CD34+ HSPCs modified with different targeted edits.
Figure 16B shows γ-globin protein upregulation in bulk differentiated human mPB CD34+ HSPCs modified with different targeted edits.

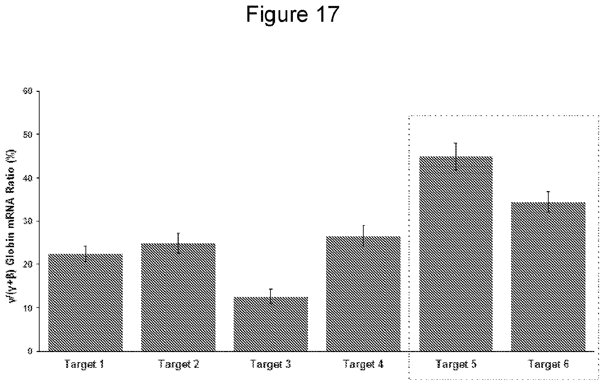

Figure 17 shows average γ-globin upregulation in individual colonies of differentiated human mPB CD34+ HSPCs modified with different target edits.

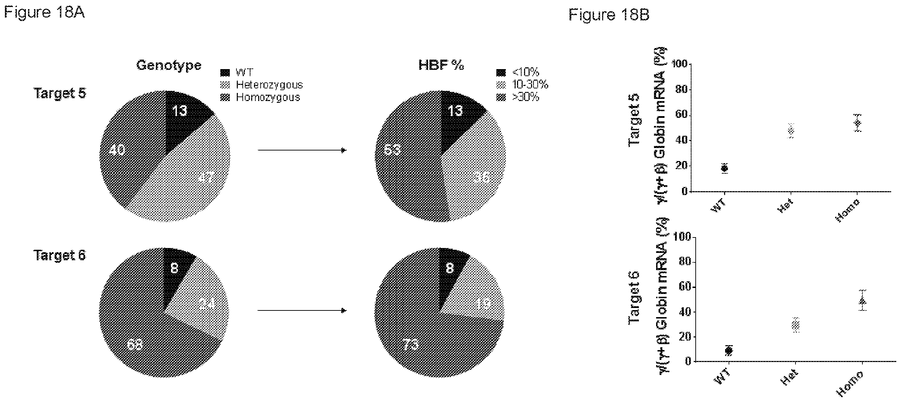

Figures 18A-B show a genotype to phenotype correlation in Target 5 and Target 6 edited colonies of erythroid differentiated human mPB CD34+ HSPCs.
Figure 18A includes charts on the left-hand side that show % of colonies with each genotype, and charts on the right side that show percent of colonies with each level of γ-globin upregulation (expressed as γ/(γ+β) globin mRNA ratio).
Figure 18B shows mRNA transcript levels, for groups of colonies with similar genotypes.

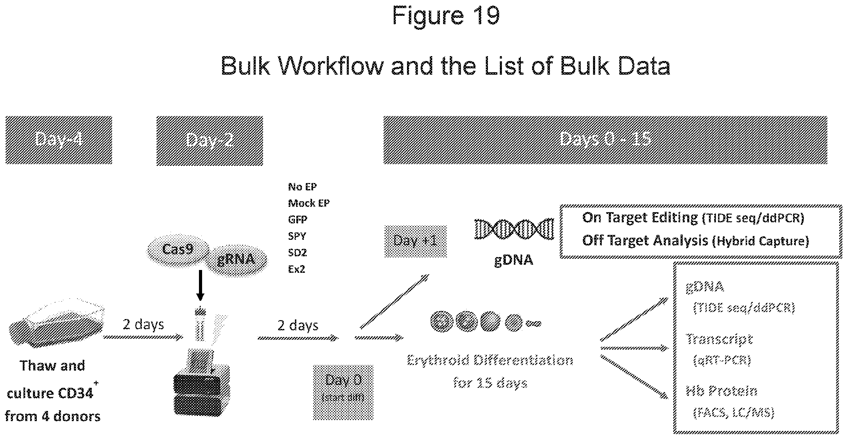

Figure 19 shows an overview of an experimental approach for bulk analysis of editing efficiency from genomic DNA, hemoglobin expression by mRNA, and protein in erythroid differentiated cell populations derived from CRISPR/Cas9 gene edited human mPB CD34+ HSPCs.

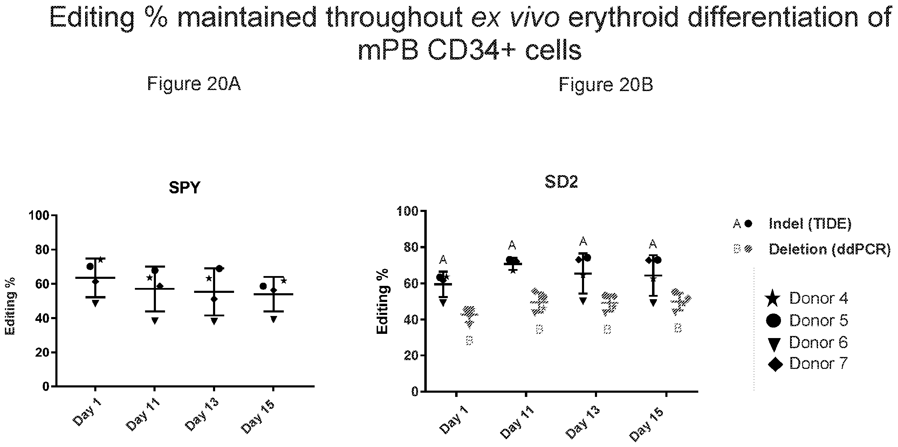

Figures 20A-B show the percentage of gene editing maintained throughout ex vivo erythroid differentiation of mPB CD34+ HSPCs edited with SPY101 gRNA or SD2 gRNA.
Figure 20A shows the percentage of gene editing maintained throughout ex vivo erythroid differentiation of mPB CD34+ HSPCs edited with SPY101 gRNA.
Figure 20B shows the percentage of gene editing maintained throughout ex vivo erythroid differentiation of mPB CD34+ HSPCs edited with SD2 gRNA.

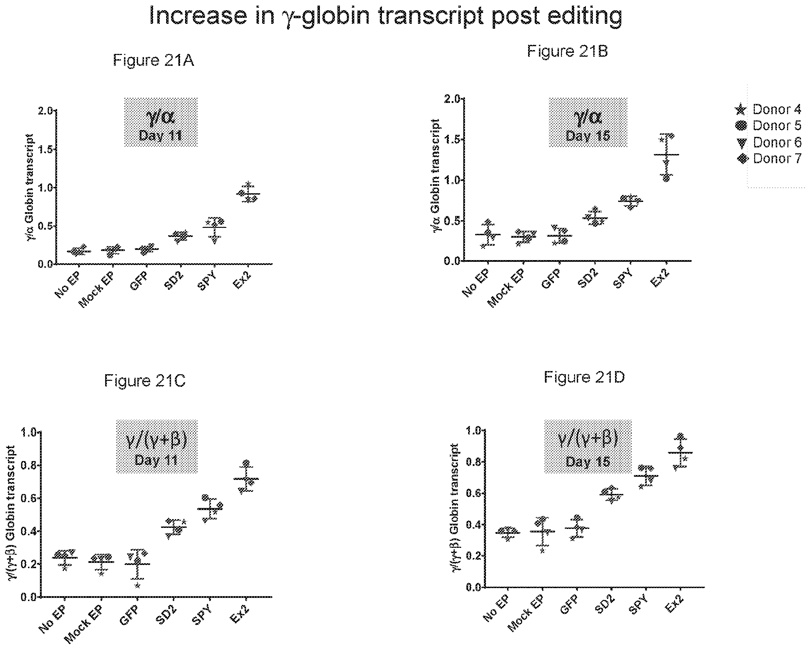

Figures 21A-D show the increase in γ-globin transcript depicted as γ/α or γ/(γ+β) in gene-edited mPB CD34+ HSPCs on days 11 or 15 post-erythroid differentiation.
Figure 21A shows the increase in γ-globin transcript (γ/α) in gene-edited mPB CD34+ HSPCs on day 11 post-differentiation.
Figure 21B shows the increase in γ-globin transcript (γ/α) in gene-edited mPB CD34+ HSPCs on day 15 post-differentiation.
Figure 21C shows the increase in γ-globin transcript (γ/(γ+β)) in gene-edited mPB CD34+ HSPCs on day 11 post-differentiation.
Figure 21D shows the increase in γ-globin transcript (γ/(γ+β)) in gene-edited mPB CD34+ HSPCs on day 15 post-differentiation.

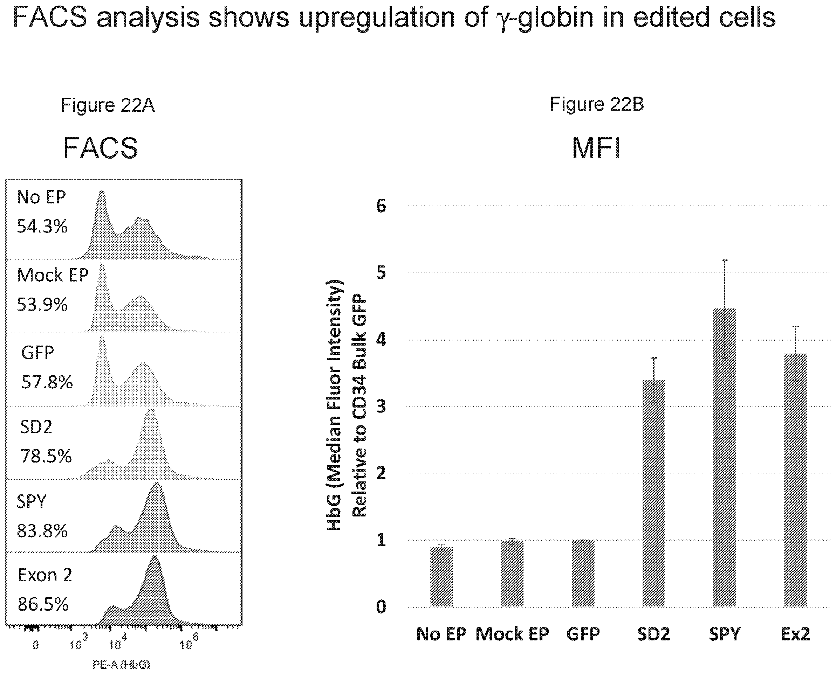

Figures 22A-B is FACS analysis and Median Flourescence Intensity (MFI) analysis showing the upregulation of γ-globin in gene-edited mPB CD34+ HSPCs on day 15 post-erythroid differentiation.
Figure 22A is FACS analysis showing the upregulation of γ-globin in gene-edited mPB CD34+ HSPCs 15 days post erythroid differentiation.
Figure 22B is MFI analysis showing the average upregulation of γ-globin in gene-edited mPB CD34+ cells from 4 donors post erythroid differentiation.

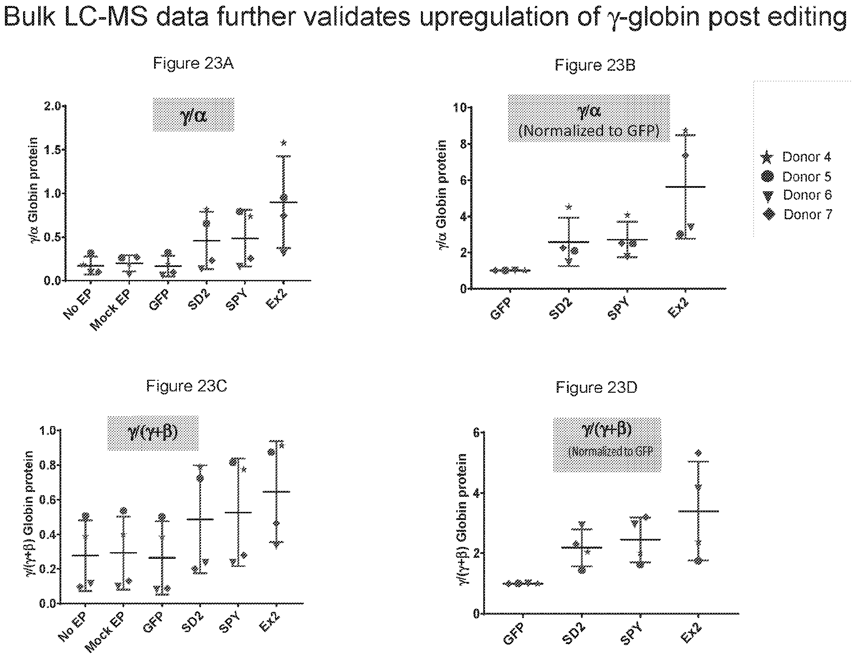

Figure 23A-D is bulk liquid-chromatography mass-spectrometry (LC-MS) data showing the upregulation of γ-globin, depicted as γ/α or γ/(γ+β) in gene-edited mPB CD34+ HSPCs on day 15 post-erythroid differentiation.
Figure 23A is bulk liquid-chromatography mass-spectrometry (LC-MS) data showing the upregulation of γ-globin (γ/α) in gene-edited mPB CD34+ HSPCs on day 15 post-differentiation.
Figure 23B is bulk liquid-chromatography mass-spectrometry (LC-MS) data showing the upregulation of γ-globin (γ/α) in gene-edited mPB CD34+ HSPCs on day 15 post-differentiation normalized to γ-globin (γ/α) in mPB CD34+ HSPCs transfected with GFP gRNA.
Figure 23C is bulk liquid-chromatography mass-spectrometry (LC-MS) data showing the upregulation of γ-globin (γ/(γ+β)) in gene-edited mPB CD34+ HSPCs on day 15 post-differentiation.
Figure 23D is bulk liquid-chromatography mass-spectrometry (LC-MS) data showing the upregulation of γ-globin (γ/(γ+β)) in gene-edited mPB CD34+ HSPCs on day 15 post-differentiation normalized to γ-globin (γ/α) in mPB CD34+ HSPCs transfected with GFP gRNA.

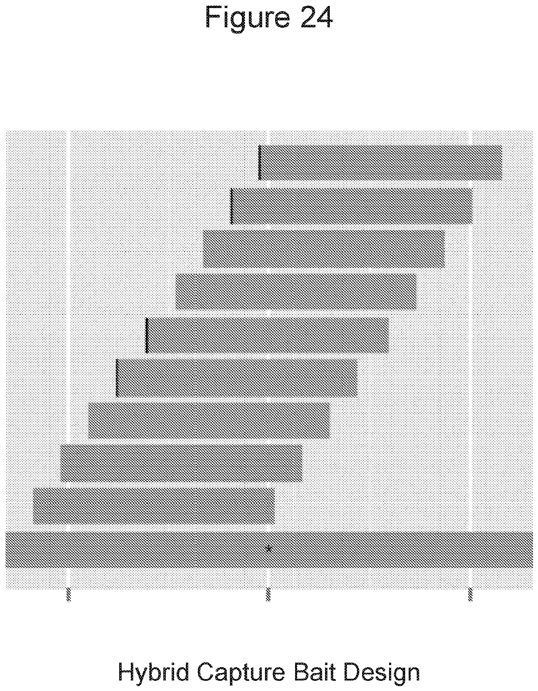

Figure 24 depicts the hybrid capture bait design.

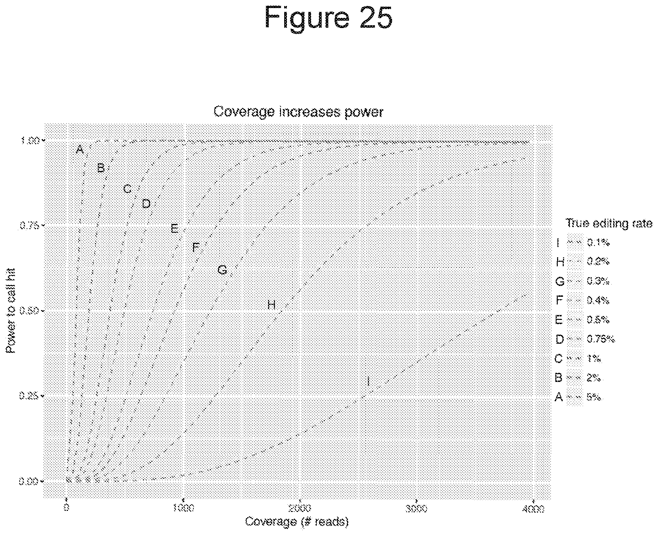

Figure 25 shows a graph depicting the hybrid capture method's power to detect indels.

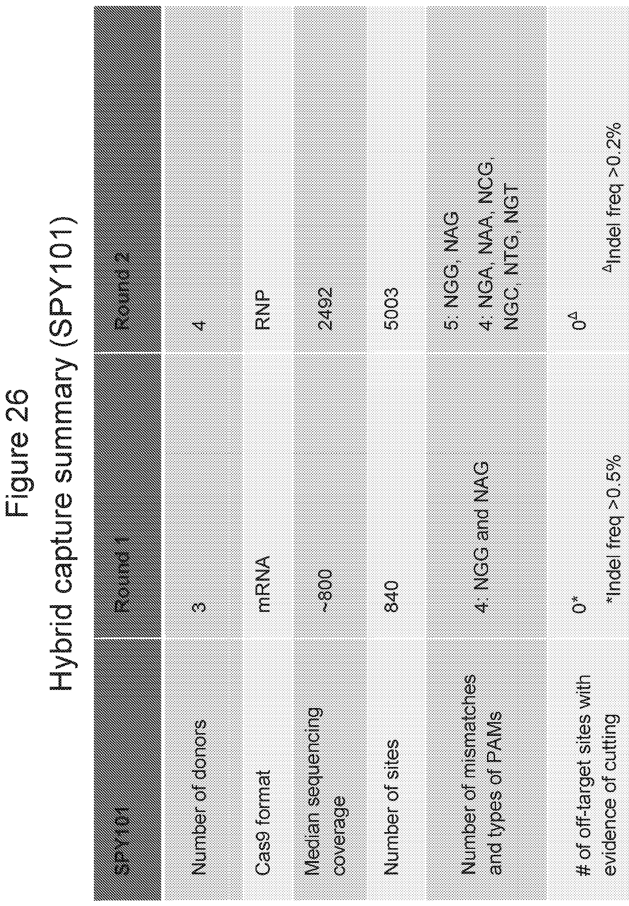

Figure 26 shows a summary of the data generated from hybrid capture experiments using SPY101 gRNA.

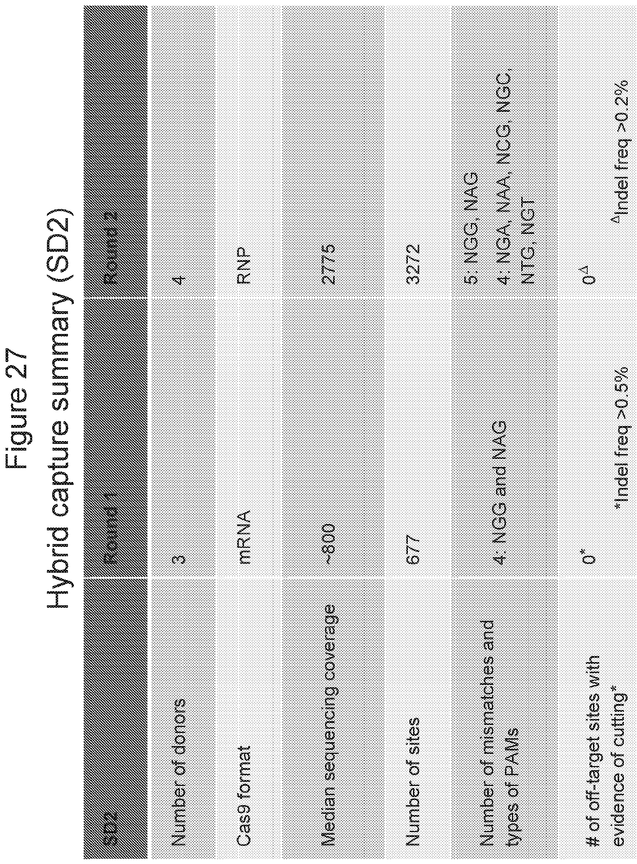

Figure 27 shows a summary of the data generated from hybrid capture experiments using SD2 gRNA.

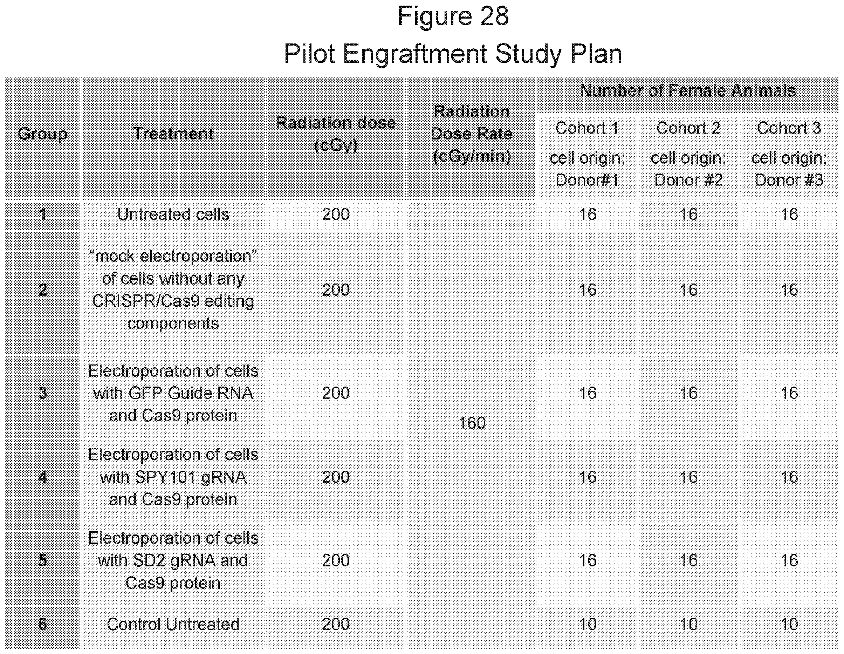

Figure 28 shows a study plan for the engraftment experiments.

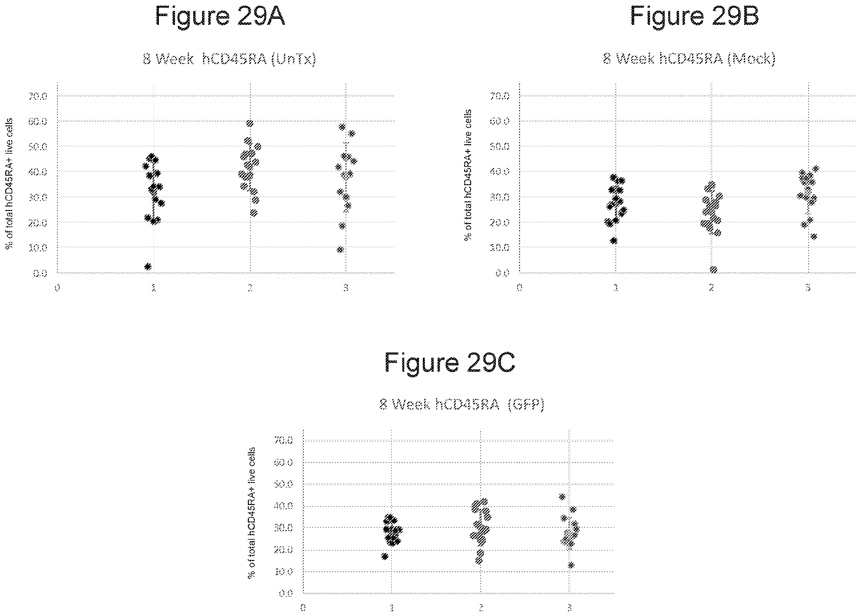

Figures 29A-E show 8 week interim bleed analysis data for untreated mice, and mice injected with mock edited cells, GFP gRNA edited cells, SPY101 gRNA edited cells, or SD2 gRNA edited cells.
Figure 29A shows 8 week interim bleed analysis data for untreated (UnTx) mice.
Figure 29B shows 8 week interim bleed analysis data for mice injected with mock-edited cells.
Figure 29C shows 8 week interim bleed analysis data for mice injected with GFP gRNA edited cells.

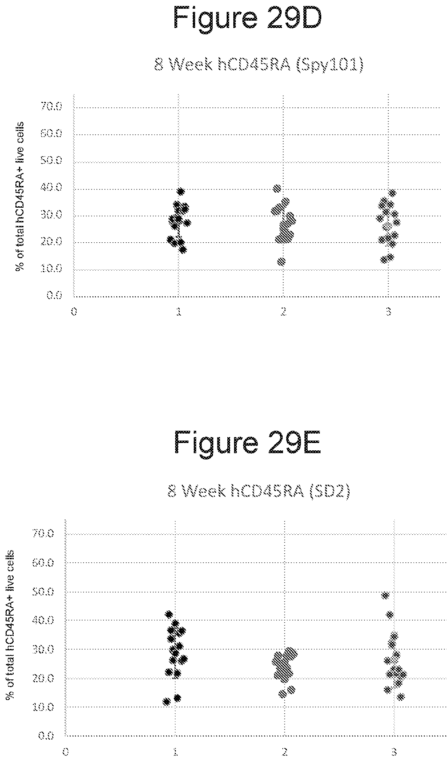

Figure 29D shows 8 week interim bleed analysis data for mice injected with SPY101 gRNA edited cells.
Figure 29E shows 8 week interim bleed analysis data for mice injected with SD2 gRNA edited cells.

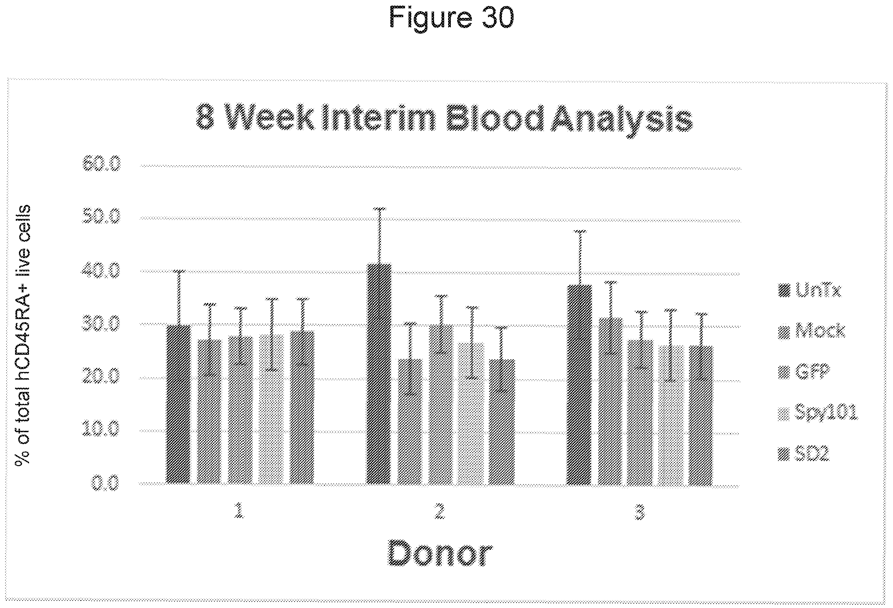

Figure 30 shows average 8 week interim bleed analysis data.

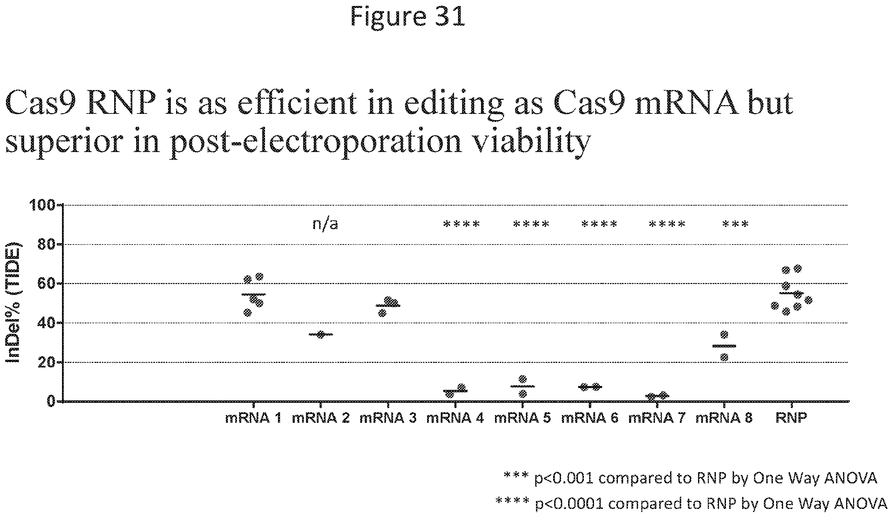

Figure 31 shows the Indel% for human mPB CD34+ HSPCs electroporated with various Cas9 mRNAs and SPY101 gRNA (mRNA1-8) compared to human mPB CD34+ HSPCs electroporated with Cas9 protein complexed with SPY101 gRNA (a ribonucleoprotein complex, RNP).

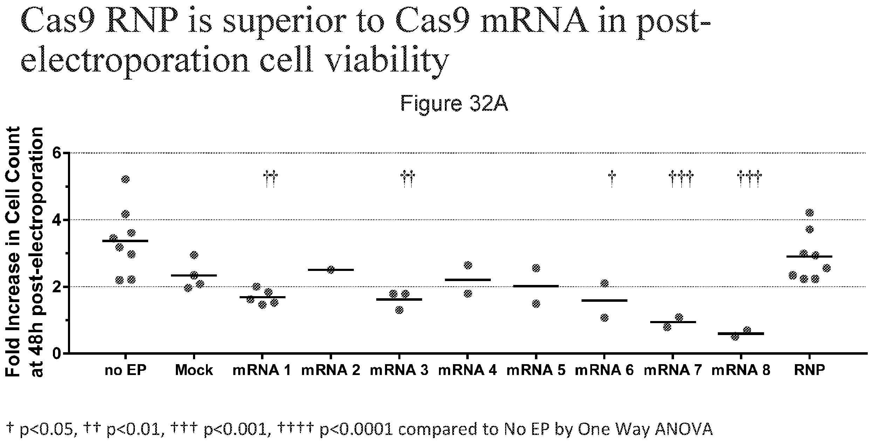

Figures 32A-B show the normalized cell count and cell viability of human mPB CD34+ HSPCs electroporated with various Cas9 mRNAs and SPY101 gRNA (mRNA 1-8) compared to human mPB CD34+ HSPCs electroporated with Cas9 protein complexed with SPY101 gRNA (RNP).
Figure 32A shows the fold increase in cell count at 48 hours post-electroporation for human mPB CD34+ HSPCs electroporated with various Cas9 mRNAs and SPY101 gRNA (mRNA 1-8) compared to human mPB CD34+ HSPCs electroporated with Cas9 protein complexed with SPY101 gRNA (RNP).

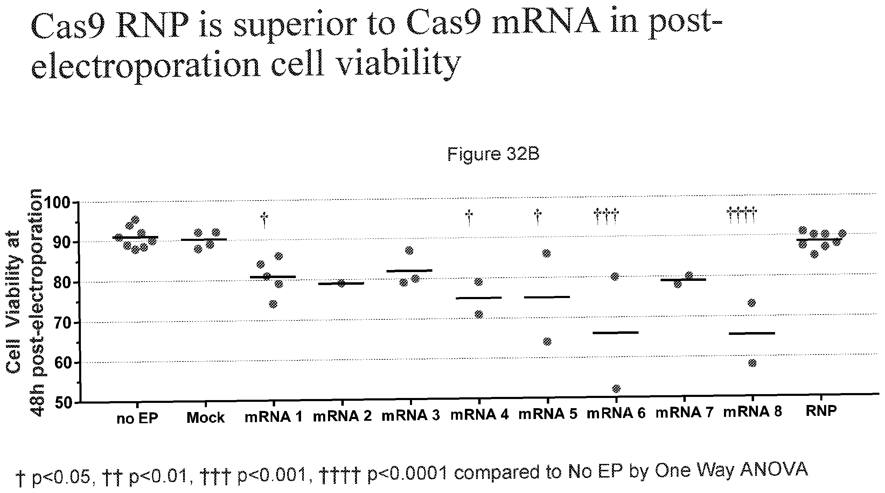

Figure 32B shows the cell viability at 48 hours post-electroporation for human mPB CD34+ HSPCs electroporated with various Cas9 mRNAs and SPY101 gRNA (mRNA 1-8) compared to human mPB CD34+ HSPCs electroporated with Cas9 protein complexed with SPY101 gRNA (RNP).

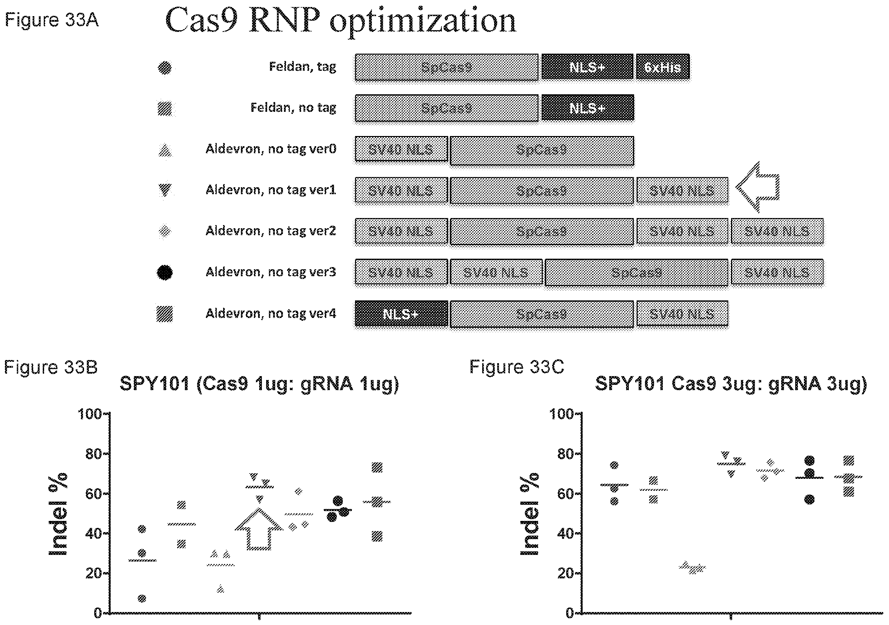

Figures 33A-C show several Cas9 RNP constructs used for Cas9 RNP optimization and the Indel% associated with each of the Cas9 RNP constructs.
Figure 33A shows several Cas9 RNP constructs.
Figure 33B shows the Indel% for each of the Cas9 RNP constructs using 1µg Cas9: 1µg SPY101 gRNA.
Figure 33C shows the Indel% for each of the Cas9 RNP constructs using 3µg Cas9: 3µg SPY101 gRNA.

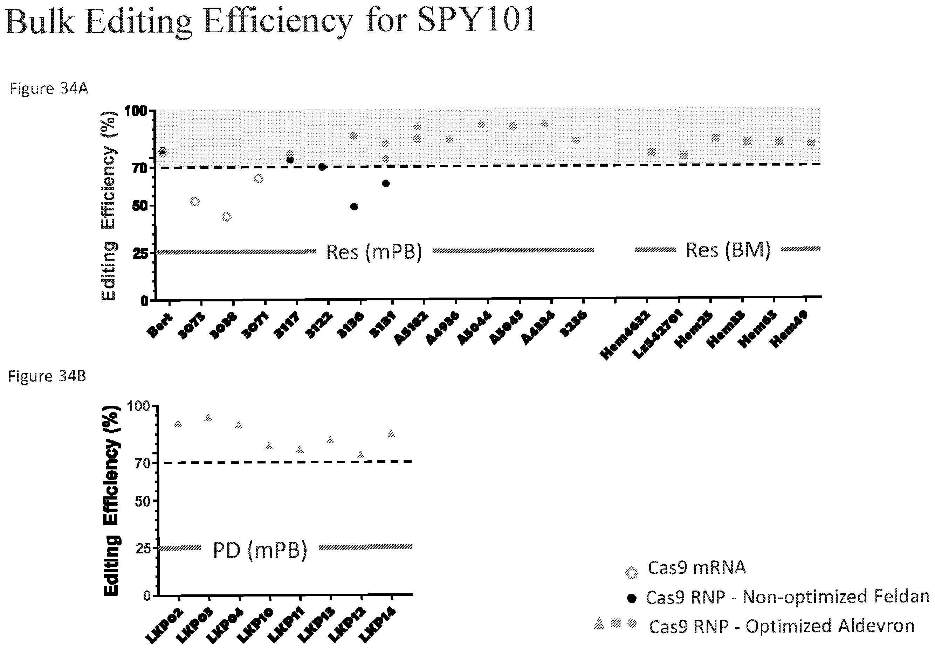

Figures 34A-B show the gene editing efficiency (%) for human mPB CD34+ HSPCs treated with either Cas9 mRNA or Cas9 protein (Feldan or Aldevron) at non-clinical and clinical scale.
Figure 34A shows the gene editing efficiency (%) for human mPB or bone marrow (BM) derived CD34+ HSPCs treated with either Cas9 mRNA or Cas9 protein (Feldan or Aldevron) at non-clinical scale.
Figure 34B shows the gene editing efficiency (%) for human mPB CD34+ HSPCs treated with Cas9 protein (Aldevron) at clinical scale.

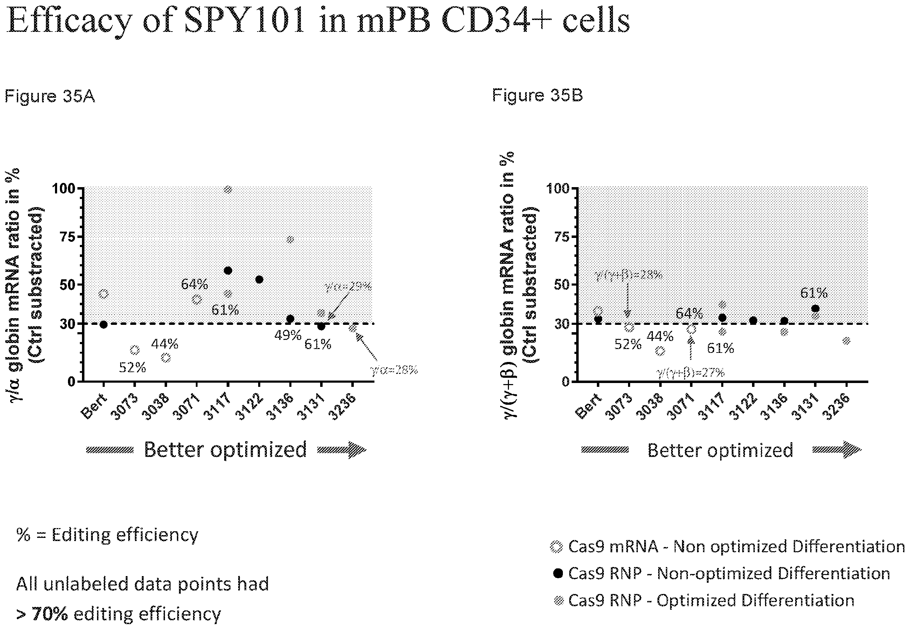

Figures 35A-B show the efficacy of SPY101 in human mPB CD34+ HSPCs by presenting the γ/α globin mRNA ratio in % and γ/(γ+β) globin mRNA ratio in % for cells treated with either Cas9 mRNA and SPY101 gRNA or Cas9 protein (Feldan or Aldevron) complexed with SPY101 gRNA.
Figure 35A shows the γ/α globin mRNA ratio in % for human mPB CD34+ HSPCs treated with either Cas9 mRNA and SPY101 gRNA or Cas9 protein (Feldan or Aldevron) complexed with SPY101 gRNA.
Figure 35B shows the γ/(γ+β) globin mRNA ratio in % for human mPB CD34+ HSPCs treated with either Cas9 mRNA and SPY101 gRNA or Cas9 protein (Feldan or Aldevron) complexed with SPY101 gRNA.

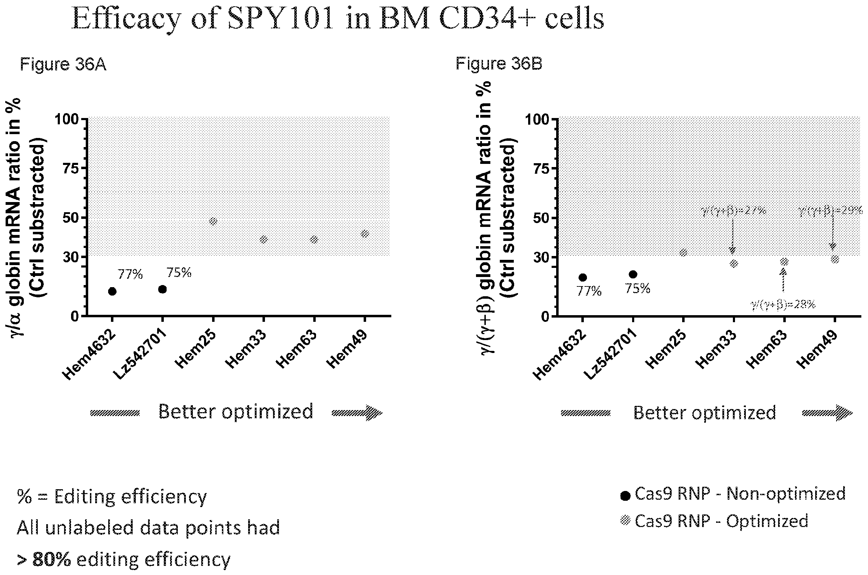

Figures 36A-B show the efficacy of SPY101 in bone marrow derived CD34+ HSPCs by presenting the γ/α globin mRNA ratio in % and γ/(γ+β) globin mRNA ratio in % for cells treated with Cas9 protein (Aldevron, technically optimized vs. non-optimized) complexed with SPY101 gRNA.
Figure 36A shows the γ/α globin mRNA ratio in % for bone marrow derived CD34+ HSPCs treated with Cas9 protein complexed with SPY101 gRNA.
Figure 36B shows the γ/(γ+β) globin mRNA ratio in % for bone marrow derived CD34+ HSPCs treated with Cas9 protein complexed with SPY101 gRNA.

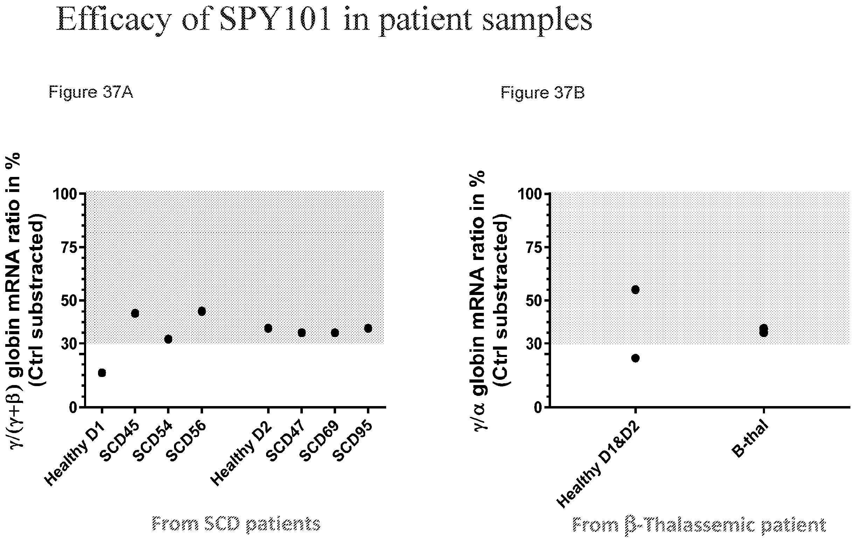

Figures 37A-B show the efficacy of SPY101 in SCD and β-Thalassemic patient samples.
Figure 37A shows the average γ/(γ+β) globin mRNA ratio in % for erythroid differentiated cells from six SCD patients and two healthy donors that were treated with SPY101 gRNA and Cas9 protein. All values were subtracted from their respective control samples treated with GFP gRNA and Cas9 protein.
Figure 37B shows the γ/α globin mRNA ratio in % for erythroid differentiated cells from one β-Thaiassemic patient and two healthy donors that were treated with SPY101 gRNA and Cas9 protein. All values were subtracted from their respective control samples treated with GFP gRNA and Cas9 protein.

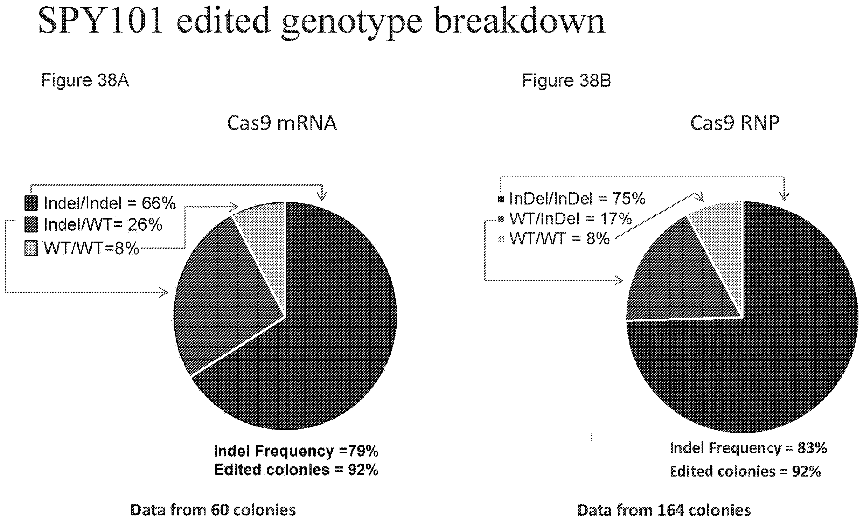

Figures 38A-B show the Bcl11a Intron (SPY101) rate of DNA editing when using Cas9 mRNA or Cas9 RNP.
Figure 38A shows the BCL11A Intron (SPY101) rate of DNA editing when using Cas9 mRNA.
Figure 38B shows the BCL11A Intron (SPY101) rate of DNA editing when using Cas9 RNP.

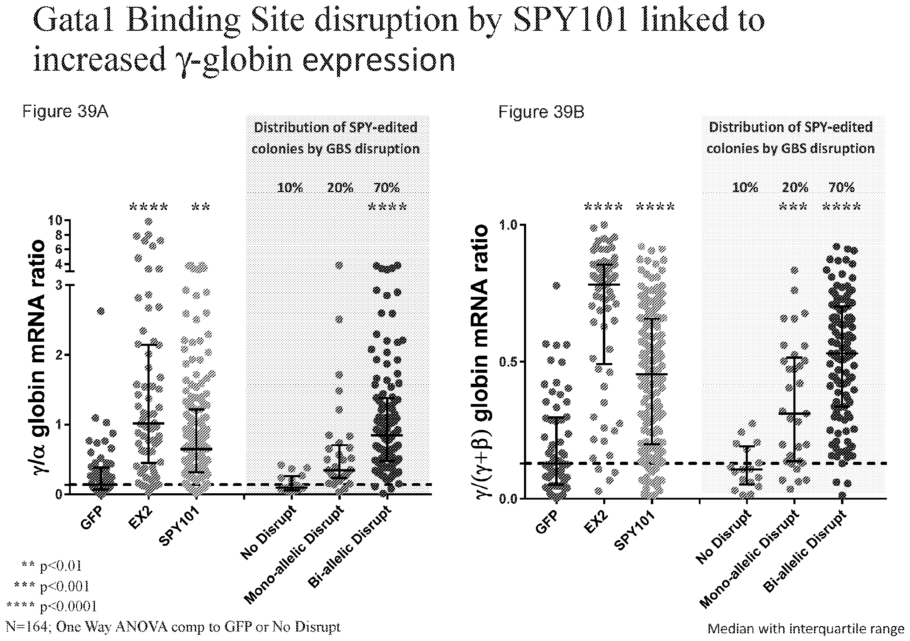

Figures 39A-B show that GATA1 binding site (GBS) disruptions caused by SPY101/Cas9 RNP in single cell colonies derived from erythroid differentiated human mPB CD34+ HSPCs are linked to increased γ-globin expression.
Figure 39A shows the γ/α globin mRNA ratio of SPY101-edited colonies with no GBS disruption, mono-allelic GBS disruptions, or bi-allelic GBS disruptions.
Figure 39B shows the γ/(γ+β) globin mRNA ratio of SPY101-edited colonies with no GBS disruption, mono-allelic GBS disruptions, or a bi-allelic GBS disruptions.

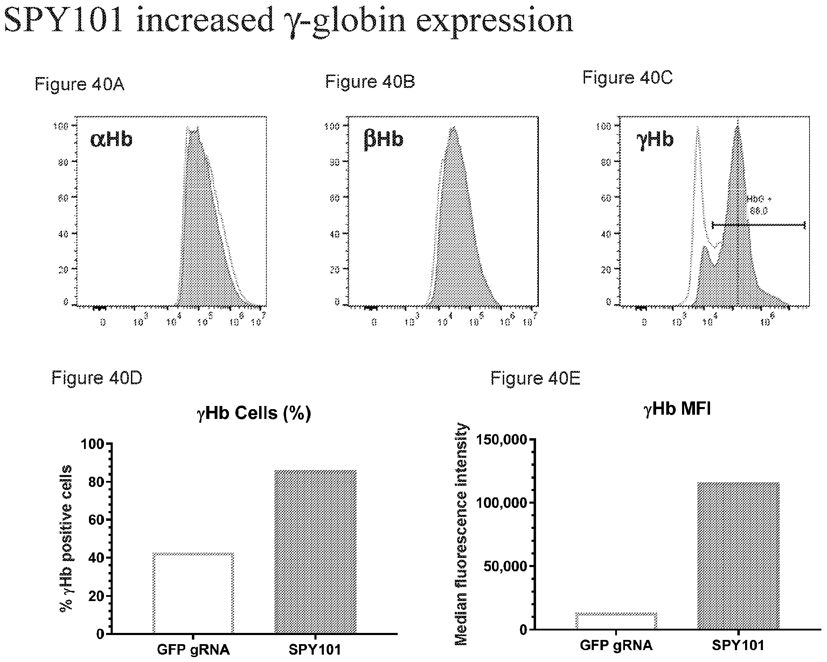

Figures 40A-E show increased γ-globin expression in erythroid differentiated SPY101/Cas9 RNP edited human mPB CD34+ HSPCs by flow cytometry analysis.
Figure 40A is flow cytometry analysis showing α-globin expression in SPY101/Cas9 RNP edited erythroid differentiated human mPB CD34+ HSPCs compared to α-globin expression in GFP gRNA/Cas9 RNP treated erythroid differentiated human mPB CD34+ HSPCs.
Figure 40B is flow cytometry analysis showing β-globin expression in SPY101/Cas9 RNP edited erythroid differentiated human mPB CD34+ HSPCs compared to β-globin expression in GFP gRNA/Cas9 RNP treated erythroid differentiated human mPB CD34+ HSPCs.
Figure 40C is flow cytometry analysis showing γ-globin expression in SPY101/Cas9 RNP edited erythroid differentiated human mPB CD34+ HSPCs compared to γ-globin expression in GFP gRNA/Cas9 RNP treated erythroid differentiated human mPB CD34+ HSPCs.
Figure 40D shows the percentage of γ-globin positive cells in SPY101/Cas9 RNP edited erythroid differentiated human mPB CD34+ HSPCs compared to GFP gRNA/Cas9 RNP treated erythroid differentiated human mPB CD34+ HSPCs.
Figure 40E shows the median fluorescence intensity (MFI) in SPY101/Cas9 RNP edited erythroid differentiated human mPB CD34+ HSPCs compared to GFP gRNA/Cas9 RNP treated erythroid differentiated human mPB CD34+ HSPCs.

### Brief Description of the Sequence Listing

SEQ ID NOs: 1 - 29,482 are 20 bp spacer sequences for targeting within or near a BCL11A gene or other DNA sequence that encodes a regulatory element of the BCL11A gene with a *S*. *pyogenes* Cas9 endonuclease.
SEQ ID NOs: 29,483 -32,387 are 20 bp spacer sequences for targeting within or near a BCL11A gene or other DNA sequence that encodes a regulatory element of the BCL11A gene with a *S*. *aureus* Cas9 endonuclease.
SEQ ID NOs: 32,388 - 33,420 are 20 bp spacer sequences for targeting within or near a BCL11A gene or other DNA sequence that encodes a regulatory element of the BCL11A gene with a *S*. *thermophilus* Cas9 endonuclease.
SEQ ID NOs: 33,421 - 33,851 are 20 bp spacer sequences for targeting within or near a BCL11A gene or other DNA sequence that encodes a regulatory element of the BCL11A gene with a *T. denticola* Cas9 endonuclease.
SEQ ID NOs: 33,852 - 36,731 are 20 bp spacer sequences for targeting within or near a BCL11A gene or other DNA sequence that encodes a regulatory element of the BCL11A gene with a *N. meningitides* Cas9 endonuclease.
SEQ ID NOs: 36,732 - 71,947 are 22 bp spacer sequences for targeting within or near a BCL11A gene or other DNA sequence that encodes a regulatory element of the BCL11A gene with an *Acidominococcus,* a *Lachnospiraceae,* and a *Franciscella Novicida* Cpf1 endonuclease.
SEQ ID NO: 71,948 is a sample guide RNA (gRNA) for a *S*. *pyogenes* Cas9 endonuclease.
SEQ ID NO: 71,949 shows a known family of homing endonuclease, as classified by its structure.
SEQ ID NO: 71,950 is gRNA A (CLO1).
SEQ ID NO: 71,951 is gRNA B (CLO8).
SEQ ID NO: 71,952 is gRNA C (CSO2).
SEQ ID NO: 71,953 is gRNA D (CSO6).
SEQ ID NO: 71,954 is gRNA E (HPFH-15).
SEQ ID NO: 71,955 is gRNA F (HPFH-4).
SEQ ID NO: 71,956 is gRNA G (Kenya02).
SEQ ID NO: 71,957 is gRNA H (Kenya17).
SEQ ID NO: 71,958 is gRNA I (SD2).
SEQ ID NO: 71,959 is gRNA J (SPY101).
SEQ ID NOs: 71,960-71,962 show sample sgRNA sequences.

### Detailed Description

### Fetal hemoglobin

Fetal hemoglobin (HbF, α₂γ₂) is the main oxygen transport protein in a human fetus and includes alpha (α) and gamma (γ) subunits. HbF expression ceases about 6 months after birth. Adult hemoglobin (HbA, α₂β₂) is the main oxygen transport protein in a human after ~34 weeks from birth, and includes alpha (α) and beta (β) subunits. After 34 weeks, a developmental switch results in decreased transcription of the γ-globin genes and increased transcription of β-globin genes. Since many of the forms of hemoglobinopathies are a result of the failure to produce normal β-globin protein in sufficient amounts or failure to produce normal β-globin protein entirely, increased expression of γ-globin (i.e., HbF) will ameliorate β-globin disease severity.

### B-cell lymphoma 11A (BCL11A)

B-cell lymphoma 11A (BCL11A) is a gene located on Chromosome 2 and ranges from 60,451,167 - 60,553,567 bp (GRCh38). BCL11A is a zinc finger transcription factor that represses fetal hemoglobin (HbF) and downregulates HbF expression starting at about 6 weeks after birth. The BCL11A gene contains 4 exons, spanning 102.4 kb of genomic DNA. BCL11A also is under transcription regulation, including a binding domain in intron 2 for the master transcripton factor GATA-1. GATA-1 binding enhances BCL11A expression which, in turn, represses HbF expression. Intron 2 contains multiple DNase hypersensitive sites (DHS), including sites referred to as +55, +58, and +62 based on the distance in kilobases from the transcriptional start site. Various editing strategies are discussed below to delete, modulate, or inactivate the transcriptional control sequences of BCL11A. Naturally occurring SNPs within this region have been associated with decreased BCL11A expression and increased fetal Hb levels (Orkin et al. 2013 GWAS study). These SNPs are organized around 3 DNA Hypersensitivity sites, +55DHS, +58DHS and +62DHS. Of the 3 regions, the +58 DHS region, appears to be the key region associated with increased fetal Hb levels and also harbors a GATA1 transcriptional control region.

### Therapeutic approach

Non-homologous end joining (NHEJ) can be used to delete segments of the transcriptional control sequence of BCL11A, either directly or by altering splice donor or acceptor sites through cleavage by one gRNA targeting several locations, or several gRNAs.

The transcriptional control sequence of the BCL11A gene can also be modulated or inactivated by inserting a wild-type BCL11A gene or cDNA comprising a modified transcriptional control sequence. For example, the donor for modulating or inactivating by homology directed repair (HDR) contains the modified transcriptional control sequence of the BCL11A gene with small or large flanking homology arms to allow for annealing. HDR is essentially an error-free mechanism that uses a supplied homologous DNA sequence as a template during DSB repair. The rate of homology directed repair (HDR) is a function of the distance between the transcriptional control sequence and the cut site so choosing overlapping or nearby target sites is important. Templates can include extra sequences flanked by the homologous regions or can contain a sequence that differs from the genomic sequence, thus allowing sequence editing.

In addition to deleting, modulating, or inactivating the transcriptional control sequence of the BCL11A gene by NHEJ or HDR, a range of other options are possible. If there are small or large deletions, a cDNA can be knocked in that contains a modified transcriptional control sequence of the BCL11A gene. A full length cDNA can be knocked into any "safe harbor"--*i.e*., non-deleterious insertion point that is not the BCL11A gene itself--, with or without suitable regulatory sequences. If this construct is knocked-in near the BCL11A regulatory elements, it should have physiological control, similar to the normal gene. Two or more (*e.g*., a pair) nucleases can be used to delete transcriptional control sequence regions, though a donor would usually have to be provided to modulate or inactivate the function. In this case two gRNA and one donor sequence would be supplied.

Provided herein are cellular, *ex vivo* and *in vivo* methods for using genome engineering tools to create permanent changes to the genome by: 1) modulating or inactivating the transcriptional control sequence of the BCL11A gene, by deletions that arise due to the NHEJ pathway; 2) modulating or inactivating the transcriptional control sequence of the BCL11A gene, by HDR; 3) modulating or inactivating the transcriptional control sequence of the BCL11A gene, by deletions of at least a portion of the transcriptional control sequence and/or knocking-in a wild-type BCL11A gene or cDNA comprising a modified transcriptional control sequence into the gene locus or a safe harbour locus. Such methods use endonucleases, such as CRISPR-associated (Cas9, Cpf1 and the like) nucleases, to permanently delete, insert, or edit the transcriptional control sequence within or near the genomic locus of the BCL11A gene or other DNA sequence that encodes a regulatory element of the BCL11A gene. In this way, examples set forth in the present disclosure can help to delete, modulate, or inactivate the transcriptional control sequence of the BCL11A gene with a single treatment or a limited number of treatments (rather than deliver potential therapies for the lifetime of the patient).

Described herein are methods for treating a patient with a hemoglobinopathy. An aspect of such method is an *ex vivo* cell-based therapy. For example, a patient specific induced pluripotent stem cell (iPSC) can be created. Then, the chromosomal DNA of these iPS cells can be edited using the materials and methods described herein. Next, the genome-edited iPSCs can be differentiated into hematopoietic progenitor cells. Finally, the hematopoietic progenitor cells can be implanted into the patient.

Yet another aspect of such method is an *ex vivo* cell-based therapy. For example, a mesenchymal stem cell can be isolated from the patient, which can be isolated from the patient's bone marrow or peripheral blood. Next, the chromosomal DNA of these mesenchymal stem cells can be edited using the materials and methods described herein. Next, the genome-edited mesenchymal stem cells can be differentiated into hematopoietic progenitor cells. Finally, these hematopoietic progenitor cells can be implanted into the patient.

A further aspect of such method is an *ex vivo* cell-based therapy. For example, a hematopoietic progenitor cell can be isolated from the patient. Next, the chromosomal DNA of these cells can be edited using the materials and methods described herein. Finally, the genome-edited hematopoietic progenitor cells can be implanted into the patient.

One advantage of an *ex vivo* cell therapy approach is the ability to conduct a comprehensive analysis of the therapeutic prior to administration. Nuclease-based therapeutics can have some level of off-target effects. Performing gene correction *ex vivo* allows one to characterize the corrected cell population prior to implantation. The present disclosure includes sequencing the entire genome of the corrected cells to ensure that the off-target effects, if any, can be in genomic locations associated with minimal risk to the patient. Furthermore, populations of specific cells, including clonal populations, can be isolated prior to implantation.

Another advantage of *ex vivo* cell therapy relates to genetic correction in iPSCs compared to other primary cell sources. iPSCs are prolific, making it easy to obtain the large number of cells that will be required for a cell-based therapy. Furthermore, iPSCs are an ideal cell type for performing clonal isolations. This allows screening for the correct genomic correction, without risking a decrease in viability. In contrast, other primary cells are viable for only a few passages and difficult to clonally expand. Thus, manipulation of iPSCs for the treatment of a hemoglobinopathy can be much easier, and can shorten the amount of time needed to make the desired genetic correction.

For *ex vivo* therapy, transplantation requires clearance of bone-marrow niches or the donor HSCs to engraft. Current methods rely on radiation and/or chemotherapy. Due to the limitations these impose, safer conditioning regiments have been and are being developed, such as immunodepletion of bone marrow cells by antibodies or antibody toxin conjugates directed against hematpoietic cell surface markers, for example CD117, c-kit and others. Success of HSC transplantation depends upon efficient homing to bone marrow, subsequent engraftment, and bone marrow repopulation. The level of gene-edited cells engrafted is important, as is the ability of the cells' multilineage engraftment.

Hematopoietic stem cells (HSCs) are an important target for *ex vivo* gene therapy as they provide a prolonged source of the corrected cells. Treated CD34+ cells would be returned to the patient.

Methods can also include an *in vivo* based therapy. Chromosomal DNA of the cells in the patient is edited using the materials and methods described herein. The cells can be bone marrow cells, hematopoietic progenitor cells, or CD34+ cells.

Although blood cells present an attractive target for *ex vivo* treatment and therapy, increased efficacy in delivery may permit direct *in vivo* delivery to the hematopoietic stem cells (HSCs) and/or other B and T cell progenitors, such as CD34+ cells. Ideally the targeting and editing would be directed to the relevant cells. Cleavage in other cells can also be prevented by the use of promoters only active in certain cells and or developmental stages. Additional promoters are inducible, and therefore can be temporally controlled if the nuclease is delivered as a plasmid. The amount of time that delivered RNA and protein remain in the cell can also be adjusted using treatments or domains added to change the half-life. *In vivo* treatment would eliminate a number of treatment steps, but a lower rate of delivery can require higher rates of editing. *In vivo* treatment can eliminate problems and losses from *ex vivo* treatment and engraftment.

An advantage of *in vivo* gene therapy can be the ease of therapeutic production and administration. The same therapeutic approach and therapy will have the potential to be used to treat more than one patient, for example a number of patients who share the same or similar genotype or allele. In contrast, *ex vivo* cell therapy typically requires using a patient's own cells, which are isolated, manipulated and returned to the same patient.

Also described herein is a cellular method for editing the BCL11A gene in a cell by genome editing. For example, a cell can be isolated from a patient or animal. Then, the chromosomal DNA of the cell can be edited using the materials and methods described herein.

The methods provided herein, regardless of whether a cellular or *ex vivo* or *in vivo* method, can involve one or a combination of the following: 1) modulating or inactivating the transcriptional control sequence of the BCL11A gene, by deletions that arise due to the NHEJ pathway, 2) modulating or inactivating the transcriptional control sequence of the BCL11A gene, by HDR, or 3) modulating or inactivating the transcriptional control sequence of the BCL11A gene, by deletion of at least a portion of the transcriptional control sequence and/or knocking-in wild-type BCL11A gene or cDNA comprising a modified transcriptional control sequence into the gene locus or at a heterologous location in the genome (such as a safe harbor site, such as AAVS1). Both the HDR and knock-in strategies utilize a donor DNA template in Homology-Directed Repair (HDR). HDR in either strategy may be accomplished by making one or more single-stranded breaks (SSBs) or double-stranded breaks (DSBs) at specific sites in the genome by using one or more endonucleases.

For example, the NHEJ strategy can involve deleting at least a portion of the transcriptional control sequence of the BCL11A gene by inducing one single stranded break or double stranded break within or near the BCL11A gene or other DNA sequence that encodes a regulatory element of the BCL11A gene with one or more CRISPR endonucleases and a gRNA (*e.g*., crRNA + tracrRNA, or sgRNA), or two or more single stranded breaks or double stranded breaks within or near the BCL11A gene or other DNA sequence that encodes a regulatory element of the BCL11A gene with two or more CRISPR endonucleases and two or more sgRNAs. This approach can require development and optimization of sgRNAs for the transcriptional control sequence of the BCL11A gene.

For example, the HDR strategy can involve modulating or inactivating the transcriptional control sequence of the BCL11A gene by inducing one single stranded break or double stranded break within or near the BCL11A gene or other DNA sequence that encodes a regulatory element of the BCL11A gene with one or more CRISPR endonucleases and a gRNA (*e.g*., crRNA + tracrRNA, or sgRNA), or two or more single stranded breaks or double stranded breaks within or near the BCL11A gene or other DNA sequence that encodes a regulatory element of the BCL11A gene with one or more CRISPR endonucleases and two or more gRNAs, in the presence of a donor DNA template introduced exogenously to direct the cellular DSB response to Homology-Directed Repair (the donor DNA template can be a short single stranded oligonucleotide, a short double stranded oligonucleotide, a long single or double stranded DNA molecule). This approach can require development and optimization of gRNAs and donor DNA molecules comprising a wild-type BCL11A gene comprising a modified transcriptional control sequence.

For example, the knock-in strategy involves knocking-in a wild-type BCL11A gene or cDNA comprising a modified transcriptional control sequence into the locus of the BCL11A gene using a gRNA (*e.g*., crRNA + tracrRNA, or sgRNA) or a pair of gRNAs targeting upstream of or in the transcriptional control sequence of the BCL11A gene, or in a safe harbor site (such as AAVS1). The donor DNA can be single or double stranded DNA and comprises a wild-type BCL11A gene comprising a modified transcriptional control sequence.

The advantages for the above strategies (deletion/modulation/inactivation and knock-in) are similar, including in principle both short and long term beneficial clinical and laboratory effects.

In addition to the editing options listed above, Cas9 or similar proteins can be used to target effector domains to the same target sites that can be identified for editing, or additional target sites within range of the effector domain. A range of chromatin modifying enzymes, methylases or demethlyases can be used to alter expression of the target gene. These types of epigenetic regulation have some advantages, particularly as they are limited in possible off-target effects.

The regulation of transcription and translation implicates a number of different classes of sites that interact with cellular proteins or nucleotides. Often the DNA binding sites of transcription factors or other proteins can be targeted for mutation or deletion to study the role of the site, though they can also be targeted to change gene expression. Sites can be added through non-homologous end joining NHEJ or direct genome editing by homology directed repair (HDR). Increased use of genome sequencing, RNA expression and genome-wide studies of transcription factor binding have increased our ability to identify how the sites lead to developmental or temporal gene regulation. These control systems can be direct or can involve extensive cooperative regulation that can require the integration of activities from multiple enhancers. Transcription factors typically bind 6-12 bp-long degenerate DNA sequences. The low level of specificity provided by individual sites suggests that complex interactions and rules are involved in binding and the functional outcome. Binding sites with less degeneracy can provide simpler means of regulation. Artificial transcription factors can be designed to specify longer sequences that have less similar sequences in the genome and have lower potential for off-target cleavage. Any of these types of binding sites can be mutated, deleted or even created to enable changes in gene regulation or expression (Canver, M.C. et al., Nature (2015)). GATA transcription factors are a family of transcription factors characterized by their ability to bind to the GATA DNA binding sequence. A GATA binding sequence is located in the +58 DNA hypersensitive site (DHS) of the BCL11A gene.

Another class of gene regulatory regions having these features is microRNA (miRNA) binding sites. miRNAs are non-coding RNAs that play key roles in posttranscriptional gene regulation. miRNA can regulate the expression of 30% of all mammalian protein-encoding genes. Specific and potent gene silencing by double stranded RNA (RNAi) was discovered, plus additional small noncoding RNA (Canver, M.C. et al., Nature (2015)). The largest class of noncoding RNAs important for gene silencing are miRNAs. In mammals, miRNAs are first transcribed as a long RNA transcripts, which can be separate transcriptional units, part of protein introns, or other transcripts. The long transcripts are called primary miRNA (pri-miRNA) that include imperfectly base-paired hairpin structures. These pri-miRNA can be cleaved into one or more shorter precursor miRNAs (pre-miRNAs) by Microprocessor, a protein complex in the nucleus, involving Drosha.

Pre-miRNAs are short stem loops ~70 nucleotides in length with a 2-nucleotide 3'-overhang that are exported, into the mature 19-25 nucleotide miRNA:miRNA* duplexes. The miRNA strand with lower base pairing stability (the guide strand) can be loaded onto the RNA-induced silencing complex (RISC). The passenger guide strand (marked with *), can be functional, but is usually degraded. The mature miRNA tethers RISC to partly complementary sequence motifs in target mRNAs predominantly found within the 3' untranslated regions (UTRs) and induces posttranscriptional gene silencing (Bartel, D.P. Cell 136, 215-233 (2009); Saj, A. & Lai, E.C. Curr Opin Genet Dev 21, 504-510 (2011)).

miRNAs can be important in development, differentiation, cell cycle and growth control, and in virtually all biological pathways in mammals and other multicellular organisms. miRNAs can also be involved in cell cycle control, apoptosis and stem cell differentiation, hematopoiesis, hypoxia, muscle development, neurogenesis, insulin secretion, cholesterol metabolism, aging, viral replication and immune responses.

A single miRNA can target hundreds of different mRNA transcripts, while an individual transcript can be targeted by many different miRNAs. More than 28645 microRNAs have been annotated in the latest release of miRBase (v.21). Some miRNAs can be encoded by multiple loci, some of which can be expressed from tandemly co-transcribed clusters. The features allow for complex regulatory networks with multiple pathways and feedback controls. miRNAs can be integral parts of these feedback and regulatory circuits and can help regulate gene expression by keeping protein production within limits (Herranz, H. & Cohen, S.M. Genes Dev 24, 1339-1344 (2010); Posadas, D.M. & Carthew, R.W. Curr Opin Genet Dev 27, 1-6 (2014)).

miRNA can also be important in a large number of human diseases that are associated with abnormal miRNA expression. This association underscores the importance of the miRNA regulatory pathway. Recent miRNA deletion studies have linked miRNA with regulation of the immune responses (Stern-Ginossar, N. et al., Science 317, 376-381 (2007)).

miRNA also have a strong link to cancer and can play a role in different types of cancer. miRNAs have been found to be downregulated in a number of tumors. miRNA can be important in the regulation of key cancer-related pathways, such as cell cycle control and the DNA damage response, and can therefore be used in diagnosis and can be targeted clinically. MicroRNAs can delicately regulate the balance of angiogenesis, such that experiments depleting all microRNAs suppresses tumor angiogenesis (Chen, S. et al., Genes Dev 28, 1054-1067 (2014)).

As has been shown for protein coding genes, miRNA genes can also be subject to epigenetic changes occurring with cancer. Many miRNA loci can be associated with CpG islands increasing their opportunity for regulation by DNA methylation (Weber, B., Stresemann, C., Brueckner, B. & Lyko, F. Cell Cycle 6, 1001-1005 (2007)). The majority of studies have used treatment with chromatin remodeling drugs to reveal epigenetically silenced miRNAs.

In addition to their role in RNA silencing, miRNA can also activate translation (Posadas, D.M. & Carthew, R.W. Curr Opin Genet Dev 27, 1-6 (2014)). Knocking out these sites may lead to decreased expression of the targeted gene, while introducing these sites may increase expression.

Individual miRNA can be knocked out most effectively by mutating the seed sequence (bases 2-8 of the microRNA), which can be important for binding specificity. Cleavage in this region, followed by mis-repair by NHEJ can effectively abolish miRNA function by blocking binding to target sites. miRNA could also be inhibited by specific targeting of the special loop region adjacent to the palindromic sequence. Catalytically inactive Cas9 can also be used to inhibit shRNA expression (Zhao, Y. et al., Sci Rep 4, 3943 (2014)). In addition to targeting the miRNA, the binding sites can also be targeted and mutated to prevent the silencing by miRNA.

### Human Cells

For ameliorating hemoglobinopathies, as described and illustrated herein, the principal targets for gene editing are human cells. For example, in the *ex vivo* methods, the human cells can be somatic cells, which after being modified using the techniques as described, can give rise to progenitor cells. For example, in the *in vivo* methods, the human cells can be a bone marrow cell, a hematopoietic progenitor cell, or a CD34+ cell.

By performing gene editing in autologous cells that are derived from and therefore already completely matched with the patient in need, it is possible to generate cells that can be safely re-introduced into the patient, and effectively give rise to a population of cells that can be effective in ameliorating one or more clinical conditions associated with the patient's disease.

Progenitor cells (also referred to as stem cells herein) are capable of both proliferation and giving rise to more progenitor cells, these in turn having the ability to generate a large number of mother cells that can in turn give rise to differentiated or differentiable daughter cells. The daughter cells themselves can be induced to proliferate and produce progeny that subsequently differentiate into one or more mature cell types, while also retaining one or more cells with parental developmental potential. The term "stem cell" refers then, to a cell with the capacity or potential, under particular circumstances, to differentiate to a more specialized or differentiated phenotype, and which retains the capacity, under certain circumstances, to proliferate without substantially differentiating. In one aspect, the term progenitor or stem cell refers to a generalized mother cell whose descendants (progeny) specialize, often in different directions, by differentiation, *e.g*., by acquiring completely individual characters, as occurs in progressive diversification of embryonic cells and tissues. Cellular differentiation is a complex process typically occurring through many cell divisions. A differentiated cell may derive from a multipotent cell that itself is derived from a multipotent cell, and so on. While each of these multipotent cells may be considered stem cells, the range of cell types that each can give rise to may vary considerably. Some differentiated cells also have the capacity to give rise to cells of greater developmental potential. Such capacity may be natural or may be induced artificially upon treatment with various factors. In many biological instances, stem cells can also be "multipotent" because they can produce progeny of more than one distinct cell type, but this is not required for "stem-ness."

Self-renewal can be another important aspect of the stem cell. In theory, self-renewal can occur by either of two major mechanisms. Stem cells can divide asymmetrically, with one daughter retaining the stem state and the other daughter expressing some distinct other specific function and phenotype. Alternatively, some of the stem cells in a population can divide symmetrically into two stems, thus maintaining some stem cells in the population as a whole, while other cells in the population give rise to differentiated progeny only. Generally, "progenitor cells" have a cellular phenotype that is more primitive (*i.e.*, is at an earlier step along a developmental pathway or progression than is a fully differentiated cell). Often, progenitor cells also have significant or very high proliferative potential. Progenitor cells can give rise to multiple distinct differentiated cell types or to a single differentiated cell type, depending on the developmental pathway and on the environment in which the cells develop and differentiate.

In the context of cell ontogeny, the adjective "differentiated," or "differentiating" is a relative term. A "differentiated cell" is a cell that has progressed further down the developmental pathway than the cell to which it is being compared. Thus, stem cells can differentiate into lineage-restricted precursor cells (such as a hematopoietic progenitor cell), which in turn can differentiate into other types of precursor cells further down the pathway (such as a hematopoietic precursor), and then to an end-stage differentiated cell, such as a erythrocyte, which plays a characteristic role in a certain tissue type, and may or may not retain the capacity to proliferate further.

The term "hematopoietic progenitor cell" refers to cells of a stem cell lineage that give rise to all the blood cell types, including erythroid (erythrocytes or red blood cells (RBCs)), myeloid (monocytes and macrophages, neutrophils, basophils, eosinophils, megakaryocytes / platelets, and dendritic cells), and lymphoid (T-cells, B-cells, NK-cells).

A "cell of the erythroid lineage" indicates that the cell being contacted is a cell that undergoes erythropoiesis, such that upon final differentiation it forms an erythrocyte or red blood cell. Such cells originate from bone marrow hematopoietic progenitor cells. Upon exposure to specific growth factors and other components of the hematopoietic microenvironment, hematopoietic progenitor cells can mature through a series of intermediate differentiation cellular types, all intermediates of the erythroid lineage, into RBCs. Thus, cells of the "erythroid lineage" comprise hematopoietic progenitor cells, rubriblasts, prorubricytes, erythroblasts, metarubricytes, reticulocytes, and erythrocytes.

The hematopoietic progenitor cell can express at least one of the following cell surface markers characteristic of hematopoietic progenitor cells: CD34+, CD59+, Thyl/CD90+, CD381o/-, and C-kit/CDI 17+. In some examples provided herein, the hematopoietic progenitors can be CD34+.

The hematopoietic progenitor cell can be a peripheral blood stem cell obtained from the patient after the patient has been treated with one or more factors such as granulocyte colony stimulating factor (optionally in combination with Plerixaflor). CD34+ cells can be enriched using CliniMACS^{®} Cell Selection System (Miltenyi Biotec). CD34+ cells can be stimulated in serum-free medium (e.g., CellGrow SCGM media, CellGenix) with cytokines (e.g., SCF, rhTPO, rhFLT3) before genome editing. Addition of SR1 and dmPGE2 and/or other factors is contemplated to improve long-term engraftment.

The hematopoietic progenitor cells of the erythroid lineage can have a cell surface marker characteristic of the erythroid lineage: such as CD71 and Terl 19.

Hematopoietic stem cells (HSCs) can be an important target for gene therapy as they provide a prolonged source of the corrected cells. HSCs give rise to both the myeloid and lymphoid lineages of blood cells. Mature blood cells have a finite life-span and must be continuously replaced throughout life. Blood cells are continually produced by the proliferation and differentiation of a population of pluripotent HSCs that can be replenished by self-renewal. Bone marrow (BM) is the major site of hematopoiesis in humans and a good source for hematopoietic stem and progenitor cells (HSPCs). HSPCs can be found in small numbers in the peripheral blood (PB). In some indications or treatments their numbers increase. The progeny of HSCs mature through stages, generating multi-potential and lineage-committed progenitor cells including the lymphoid progenitor cells giving rise to the cells expressing BCL 11A. B and T cell progenitors are the two cell populations requiring the activity of BCL11A, so they could be edited at the stages prior to re-arrangement, though correcting progenitors has the advantage of continuing to be a source of corrected cells. Treated cells, such as CD34+ cells, would be returned to the patient. The level of engraftment can be important, as is the ability of the cells' multilineage engraftment of gene-edited cells following CD34+ infusion *in vivo.*

### Induced Pluripotent Stem Cells

The genetically engineered human cells described herein can be induced pluripotent stem cells (iPSCs). An advantage of using iPSCs is that the cells can be derived from the same subject to which the progenitor cells are to be administered. That is, a somatic cell can be obtained from a subject, reprogrammed to an induced pluripotent stem cell, and then re-differentiated into a progenitor cell to be administered to the subject (*e.g*., autologous cells). Because the progenitors are essentially derived from an autologous source, the risk of engraftment rejection or allergic response can be reduced compared to the use of cells from another subject or group of subjects. In addition, the use of iPSCs negates the need for cells obtained from an embryonic source. Thus, in one aspect, the stem cells used in the disclosed methods are not embryonic stem cells.

Although differentiation is generally irreversible under physiological contexts, several methods have been recently developed to reprogram somatic cells to iPSCs. Exemplary methods are known to those of skill in the art and are described briefly herein below.

The term "reprogramming" refers to a process that alters or reverses the differentiation state of a differentiated cell (*e.g*., a somatic cell). Stated another way, reprogramming refers to a process of driving the differentiation of a cell backwards to a more undifferentiated or more primitive type of cell. It should be noted that placing many primary cells in culture can lead to some loss of fully differentiated characteristics. Thus, simply culturing such cells included in the term differentiated cells does not render these cells non-differentiated cells (*e.g*., undifferentiated cells) or pluripotent cells. The transition of a differentiated cell to pluripotency requires a reprogramming stimulus beyond the stimuli that lead to partial loss of differentiated character in culture. Reprogrammed cells also have the characteristic of the capacity of extended passaging without loss of growth potential, relative to primary cell parents, which generally have capacity for only a limited number of divisions in culture.

The cell to be reprogrammed can be either partially or terminally differentiated prior to reprogramming. Reprogramming can encompass complete reversion of the differentiation state of a differentiated cell (*e.g*., a somatic cell) to a pluripotent state or a multipotent state. Reprogramming can encompass complete or partial reversion of the differentiation state of a differentiated cell (*e.g*., a somatic cell) to an undifferentiated cell (*e.g.*, an embryonic-like cell). Reprogramming can result in expression of particular genes by the cells, the expression of which further contributes to reprogramming. In certain examples described herein, reprogramming of a differentiated cell (*e.g*., a somatic cell) can cause the differentiated cell to assume an undifferentiated state (*e.g*., is an undifferentiated cell). The resulting cells are referred to as "reprogrammed cells," or "induced pluripotent stem cells (iPSCs or iPS cells)."

Reprogramming can involve alteration, *e.g*., reversal, of at least some of the heritable patterns of nucleic acid modification (*e.g*., methylation), chromatin condensation, epigenetic changes, genomic imprinting, etc., that occur during cellular differentiation. Reprogramming is distinct from simply maintaining the existing undifferentiated state of a cell that is already pluripotent or maintaining the existing less than fully differentiated state of a cell that is already a multipotent cell (*e.g*., a hematopoietic stem cell). Reprogramming is also distinct from promoting the self-renewal or proliferation of cells that are already pluripotent or multipotent, although the compositions and methods described herein can also be of use for such purposes, in some examples.

Many methods are known in the art that can be used to generate pluripotent stem cells from somatic cells. Any such method that reprograms a somatic cell to the pluripotent phenotype would be appropriate for use in the methods described herein.

Reprogramming methodologies for generating pluripotent cells using defined combinations of transcription factors have been described. Mouse somatic cells can be converted to ES cell-like cells with expanded developmental potential by the direct transduction of Oct4, Sox2, Klf4, and c-Myc; see, *e.g*., Takahashi and Yamanaka, Cell 126(4): 663-76 (2006). iPSCs resemble ES cells, as they restore the pluripotency-associated transcriptional circuitry and much of the epigenetic landscape. In addition, mouse iPSCs satisfy all the standard assays for pluripotency: specifically, *in vitro* differentiation into cell types of the three germ layers, teratoma formation, contribution to chimeras, germline transmission [see, *e.g.*, Maherali and Hochedlinger, Cell Stem Cell. 3(6):595-605 (2008)], and tetraploid complementation.

Human iPSCs can be obtained using similar transduction methods, and the transcription factor trio, OCT4, SOX2, and NANOG, has been established as the core set of transcription factors that govern pluripotency; see, *e.g*., Budniatzky and Gepstein, Stem Cells Transl Med. 3(4):448-57 (2014); Barrett et al., Stem Cells Trans Med 3:1-6 sctm.2014-0121 (2014); Focosi et al., Blood Cancer Journal 4: e211 (2014); and references cited therein. The production of iPSCs can be achieved by the introduction of nucleic acid sequences encoding stem cell-associated genes into an adult, somatic cell, historically using viral vectors.

iPSCs can be generated or derived from terminally differentiated somatic cells, as well as from adult stem cells, or somatic stem cells. That is, a non-pluripotent progenitor cell can be rendered pluripotent or multipotent by reprogramming. In such instances, it may not be necessary to include as many reprogramming factors as required to reprogram a terminally differentiated cell. Further, reprogramming can be induced by the non-viral introduction of reprogramming factors, *e.g*., by introducing the proteins themselves, or by introducing nucleic acids that encode the reprogramming factors, or by introducing messenger RNAs that upon translation produce the reprogramming factors (see *e.g*., Warren et al., Cell Stem Cell, 7(5):618-30 (2010). Reprogramming can be achieved by introducing a combination of nucleic acids encoding stem cell-associated genes, including, for example, Oct-4 (also known as Oct-3/4 or Pouf51), SoxI, Sox2, Sox3, Sox 15, Sox 18, NANOG, Klfl, Klf2, Klf4, Klf5, NR5A2, c-Myc, 1-Myc, n-Myc, Rem2, Tert, and LIN28. Reprogramming using the methods and compositions described herein can further comprise introducing one or more of Oct-3/4, a member of the Sox family, a member of the Klf family, and a member of the Myc family to a somatic cell. The methods and compositions described herein can further comprise introducing one or more of each of Oct-4, Sox2, Nanog, c-MYC and Klf4 for reprogramming. As noted above, the exact method used for reprogramming is not necessarily critical to the methods and compositions described herein. However, where cells differentiated from the reprogrammed cells are to be used in, *e.g*., human therapy, in one aspect the reprogramming is not effected by a method that alters the genome. Thus, in such examples, reprogramming can be achieved, e.g., without the use of viral or plasmid vectors.

The efficiency of reprogramming (*i.e*., the number of reprogrammed cells) derived from a population of starting cells can be enhanced by the addition of various agents, *e.g*., small molecules, as shown by Shi et al., Cell-Stem Cell 2:525-528 (2008); Huangfu et al., Nature Biotechnology 26(7):795-797 (2008) and Marson et al., Cell-Stem Cell 3: 132-135 (2008). Thus, an agent or combination of agents that enhance the efficiency or rate of induced pluripotent stem cell production can be used in the production of patient-specific or disease-specific iPSCs. Some non-limiting examples of agents that enhance reprogramming efficiency include soluble Wnt, Wnt conditioned media, BIX-01294 (a G9a histone methyltransferase), PD0325901 (a MEK inhibitor), DNA methyltransferase inhibitors, histone deacetylase (HDAC) inhibitors, valproic acid, 5'-azacytidine, dexamethasone, suberoylanilide, hydroxamic acid (SAHA), vitamin C, and trichostatin (TSA), among others.

Other non-limiting examples of reprogramming enhancing agents include: Suberoylanilide Hydroxamic Acid (SAHA (*e.g*., MK0683, vorinostat) and other hydroxamic acids), BML-210, Depudecin (*e.g.*, (-)-Depudecin), HC Toxin, Nullscript (4-(I,3-Dioxo-IH,3H-benzo[de]isoquinolin-2-yl)-N-hydroxybutanamide), Phenylbutyrate (*e.g*., sodium phenylbutyrate) and Valproic Acid ((VP A) and other short chain fatty acids), Scriptaid, Suramin Sodium, Trichostatin A (TSA), APHA Compound 8, Apicidin, Sodium Butyrate, pivaloyloxymethyl butyrate (Pivanex, AN-9), Trapoxin B, Chlamydocin, Depsipeptide (also known as FR901228 or FK228), benzamides (*e.g*., CI-994 (*e.g*., N-acetyl dinaline) and MS-27-275), MGCD0103, NVP-LAQ-824, CBHA (m-carboxycinnaminic acid bishydroxamic acid), JNJ16241199, Tubacin, A-161906, proxamide, oxamflatin, 3-CI-UCHA (*e.g.*, 6-(3-chlorophenylureido)caproic hydroxamic acid), AOE (2-amino-8-oxo-9, 10-epoxydecanoic acid), CHAP31 and CHAP 50. Other reprogramming enhancing agents include, for example, dominant negative forms of the HDACs (*e.g*., catalytically inactive forms), siRNA inhibitors of the HDACs, and antibodies that specifically bind to the HDACs. Such inhibitors are available, *e.g*., from BIOMOL International, Fukasawa, Merck Biosciences, Novartis, Gloucester Pharmaceuticals, Titan Pharmaceuticals, MethylGene, and Sigma Aldrich.

To confirm the induction of pluripotent stem cells for use with the methods described herein, isolated clones can be tested for the expression of a stem cell marker. Such expression in a cell derived from a somatic cell identifies the cells as induced pluripotent stem cells. Stem cell markers can be selected from the non-limiting group including SSEA3, SSEA4, CD9, Nanog, Fbxl5, Ecatl, Esgl, Eras, Gdf3, Fgf4, Cripto, Daxl, Zpf296, Slc2a3, Rexl, Utfl, and Natl. In one case, for example, a cell that expresses Oct4 or Nanog is identified as pluripotent. Methods for detecting the expression of such markers can include, for example, RT-PCR and immunological methods that detect the presence of the encoded polypeptides, such as Western blots or flow cytometric analyses. Detection can involve not only RT-PCR, but can also include detection of protein markers. Intracellular markers may be best identified via RT-PCR, or protein detection methods such as immunocytochemistry, while cell surface markers are readily identified, *e.g*., by immunocytochemistry.

The pluripotent stem cell character of isolated cells can be confirmed by tests evaluating the ability of the iPSCs to differentiate into cells of each of the three germ layers. As one example, teratoma formation in nude mice can be used to evaluate the pluripotent character of the isolated clones. The cells can be introduced into nude mice and histology and/or immunohistochemistry can be performed on a tumor arising from the cells. The growth of a tumor comprising cells from all three germ layers, for example, further indicates that the cells are pluripotent stem cells.

### Creating patient specific iPSCs

One step of the *ex vivo* methods of the present disclosure can involve creating a patient specific iPS cell, patient specific iPS cells, or a patient specific iPS cell line. There are many established methods in the art for creating patient specific iPS cells, as described in Takahashi and Yamanaka 2006; Takahashi, Tanabe *et al.* 2007. For example, the creating step can comprise: a) isolating a somatic cell, such as a skin cell or fibroblast, from the patient; and b) introducing a set of pluripotency-associated genes into the somatic cell in order to induce the cell to become a pluripotent stem cell. The set of pluripotency-associated genes can be one or more of the genes selected from the group consisting of OCT4, SOX2, KLF4, Lin28, NANOG, and cMYC.

### Performing a biopsy or aspirate of the patient's bone marrow

A biopsy or aspirate is a sample of tissue or fluid taken from the body. There are many different kinds of biopsies or aspirates. Nearly all of them involve using a sharp tool to remove a small amount of tissue. If the biopsy will be on the skin or other sensitive area, numbing medicine can be applied first. A biopsy or aspirate can be performed according to any of the known methods in the art. For example, in a bone marrow aspirate, a large needle is used to enter the pelvis bone to collect bone marrow.

### Isolating a mesenchymal stem cell

Mesenchymal stem cells can be isolated according to any method known in the art, such as from a patient's bone marrow or peripheral blood. For example, marrow aspirate can be collected into a syringe with heparin. Cells can be washed and centrifuged on a Percoll^{™} density gradient. Cells, such as blood cells, liver cells, interstitial cells, macrophages, mast cells, and thymocytes, can be separated using Percoll^{™}. The cells can be cultured in Dulbecco's modified Eagle's medium (DMEM) (low glucose) containing 10% fetal bovine serum (FBS) (Pittinger MF, Mackay AM, Beck SC et al., Science 1999; 284:143-147).

### Treating a patient with GCSF

A patient may optionally be treated with granulocyte colony stimulating factor (GCSF) in accordance with any method known in the art. The GCSF can be administered in combination with Plerixaflor.

### Isolating a hematopoietic progenitor cell from a patient

A hematopoietic progenitor cell can be isolated from a patient by any method known in the art. CD34+ cells can be enriched using CliniMACS^{®} Cell Selection System (Miltenyi Biotec). CD34+ cells can be weakly stimulated in serum-free medium (e.g., CellGrow SCGM media, CellGenix) with cytokines (e.g., SCF, rhTPO, rhFLT3) before genome editing.

### Genome Editing

Genome editing generally refers to the process of modifying the nucleotide sequence of a genome, preferably in a precise or pre-determined manner. Examples of methods of genome editing described herein include methods of using site-directed nucleases to cut deoxyribonucleic acid (DNA) at precise target locations in the genome, thereby creating single-strand or double-strand DNA breaks at particular locations within the genome. Such breaks can be and regularly are repaired by natural, endogenous cellular processes, such as homology-directed repair (HDR) and NHEJ, as recently reviewed in Cox et al., Nature Medicine 21(2), 121-31 (2015). These two main DNA repair processes consist of a family of alternative pathways. NHEJ directly joins the DNA ends resulting from a double-strand break, sometimes with the loss or addition of nucleotide sequence, which may disrupt or enhance gene expression. HDR utilizes a homologous sequence, or donor sequence, as a template for inserting a defined DNA sequence at the break point. The homologous sequence can be in the endogenous genome, such as a sister chromatid. Alternatively, the donor can be an exogenous nucleic acid, such as a plasmid, a single-strand oligonucleotide, a double-stranded oligonucleotide, a duplex oligonucleotide or a virus, that has regions of high homology with the nuclease-cleaved locus, but which can also contain additional sequence or sequence changes including deletions that can be incorporated into the cleaved target locus. A third repair mechanism can be microhomology-mediated end joining (MMEJ), also referred to as "Alternative NHEJ", in which the genetic outcome is similar to NHEJ in that small deletions and insertions can occur at the cleavage site. MMEJ can make use of homologous sequences of a few basepairs flanking the DNA break site to drive a more favored DNA end joining repair outcome, and recent reports have further elucidated the molecular mechanism of this process; see, *e.g*., Cho and Greenberg, Nature 518, 174-76 (2015); Kent et al., Nature Structural and Molecular Biology, Adv. Online doi:10.1038/nsmb.2961 (2015); Mateos-Gomez et al., Nature 518, 254-57 (2015); Ceccaldi et al., Nature 528, 258-62 (2015). In some instances it may be possible to predict likely repair outcomes based on analysis of potential microhomologies at the site of the DNA break.

Each of these genome editing mechanisms can be used to create desired genomic alterations. A step in the genome editing process can be to create one or two DNA breaks, the latter as double-strand breaks or as two single-stranded breaks, in the target locus as near the site of intended mutation. This can be achieved via the use of site-directed polypeptides, as described and illustrated herein.

Site-directed polypeptides, such as a DNA endonuclease, can introduce double-strand breaks or single-strand breaks in nucleic acids, *e.g*., genomic DNA. The double-strand break can stimulate a cell's endogenous DNA-repair pathways (*e.g*., homology-dependent repair or non-homologous end joining or alternative non-homologous end joining (A-NHEJ) or microhomology-mediated end joining). NHEJ can repair cleaved target nucleic acid without the need for a homologous template. This can sometimes result in small deletions or insertions (indels) in the target nucleic acid at the site of cleavage, and can lead to disruption or alteration of gene expression. HDR can occur when a homologous repair template, or donor, is available. The homologous donor template can comprise sequences that can be homologous to sequences flanking the target nucleic acid cleavage site. The sister chromatid can be used by the cell as the repair template. However, for the purposes of genome editing, the repair template can be supplied as an exogenous nucleic acid, such as a plasmid, duplex oligonucleotide, single-strand oligonucleotide, double-stranded oligonucleotide, or viral nucleic acid. With exogenous donor templates, an additional nucleic acid sequence (such as a transgene) or modification (such as a single or multiple base change or a deletion) can be introduced between the flanking regions of homology so that the additional or altered nucleic acid sequence also becomes incorporated into the target locus. MMEJ can result in a genetic outcome that is similar to NHEJ in that small deletions and insertions can occur at the cleavage site. MMEJ can make use of homologous sequences of a few basepairs flanking the cleavage site to drive a favored end-joining DNA repair outcome. In some instances it may be possible to predict likely repair outcomes based on analysis of potential microhomologies in the nuclease target regions.

Thus, in some cases, homologous recombination can be used to insert an exogenous polynucleotide sequence into the target nucleic acid cleavage site. An exogenous polynucleotide sequence is termed a donor polynucleotide (or donor or donor sequence or polynucleotide donor template) herein. The donor polynucleotide, a portion of the donor polynucleotide, a copy of the donor polynucleotide, or a portion of a copy of the donor polynucleotide can be inserted into the target nucleic acid cleavage site. The donor polynucleotide can be an exogenous polynucleotide sequence, i.e., a sequence that does not naturally occur at the target nucleic acid cleavage site.

The modifications of the target DNA due to NHEJ and/or HDR can lead to, for example, mutations, deletions, alterations, integrations, gene correction, gene replacement, gene tagging, transgene insertion, nucleotide deletion, gene disruption, translocations and/or gene mutation. The processes of deleting genomic DNA and integrating non-native nucleic acid into genomic DNA are examples of genome editing.

### CRISPR Endonuclease System

A CRISPR (Clustered Regularly Interspaced Short Palindromic Repeats) genomic locus can be found in the genomes of many prokaryotes (*e.g*., bacteria and archaea). In prokaryotes, the CRISPR locus encodes products that function as a type of immune system to help defend the prokaryotes against foreign invaders, such as virus and phage. There are three stages of CRISPR locus function: integration of new sequences into the CRISPR locus, expression of CRISPR RNA (crRNA), and silencing of foreign invader nucleic acid. Five types of CRISPR systems (*e.g*., Type I, Type II, Type III, Type U, and Type V) have been identified.

A CRISPR locus includes a number of short repeating sequences referred to as "repeats." When expressed, the repeats can form secondary structures (e.g., hairpins) and/or comprise unstructured single-stranded sequences. The repeats usually occur in clusters and frequently diverge between species. The repeats are regularly interspaced with unique intervening sequences referred to as "spacers," resulting in a repeat-spacer-repeat locus architecture. The spacers are identical to or have high homology with known foreign invader sequences. A spacer-repeat unit encodes a crisprRNA (crRNA), which is processed into a mature form of the spacer-repeat unit. A crRNA comprises a "seed" or spacer sequence that is involved in targeting a target nucleic acid (in the naturally occurring form in prokaryotes, the spacer sequence targets the foreign invader nucleic acid). A spacer sequence is located at the 5' or 3' end of the crRNA.

A CRISPR locus also comprises polynucleotide sequences encoding CRISPR Associated (Cas) genes. Cas genes encode endonucleases involved in the biogenesis and the interference stages of crRNA function in prokaryotes. Some Cas genes comprise homologous secondary and/or tertiary structures.

### Type II CRISPR Systems

crRNA biogenesis in a Type II CRISPR system in nature requires a trans-activating CRISPR RNA (tracrRNA). The tracrRNA can be modified by endogenous RNaselll, and then hybridizes to a crRNA repeat in the pre-crRNA array. Endogenous RNaselll can be recruited to cleave the pre-crRNA. Cleaved crRNAs can be subjected to exoribonuclease trimming to produce the mature crRNA form (*e.g*., 5' trimming). The tracrRNA can remain hybridized to the crRNA, and the tracrRNA and the crRNA associate with a site-directed polypeptide (*e.g*., Cas9). The crRNA of the crRNA-tracrRNA-Cas9 complex can guide the complex to a target nucleic acid to which the crRNA can hybridize. Hybridization of the crRNA to the target nucleic acid can activate Cas9 for targeted nucleic acid cleavage. The target nucleic acid in a Type II CRISPR system is referred to as a protospacer adjacent motif (PAM). In nature, the PAM is essential to facilitate binding of a site-directed polypeptide (*e.g.*, Cas9) to the target nucleic acid. Type II systems (also referred to as Nmeni or CASS4) are further subdivided into Type II-A (CASS4) and II-B (CASS4a). Jinek et al., Science, 337(6096):816-821 (2012) showed that the CRISPR/Cas9 system is useful for RNA-programmable genome editing, and international patent application publication number WO2013/176772 provides numerous examples and applications of the CRISPR/Cas endonuclease system for site-specific gene editing.

### Type V CRISPR Systems

Type V CRISPR systems have several important differences from Type II systems. For example, Cpf1 is a single RNA-guided endonuclease that, in contrast to Type II systems, lacks tracrRNA. In fact, Cpf1-associated CRISPR arrays can be processed into mature crRNAs without the requirement of an additional trans-activating tracrRNA. The Type V CRISPR array can be processed into short mature crRNAs of 42-44 nucleotides in length, with each mature crRNA beginning with 19 nucleotides of direct repeat followed by 23-25 nucleotides of spacer sequence. In contrast, mature crRNAs in Type II systems can start with 20-24 nucleotides of spacer sequence followed by about 22 nucleotides of direct repeat. Also, Cpf1 can utilize a T-rich protospacer-adjacent motif such that Cpf1-crRNA complexes efficiently cleave target DNA proceeded by a short T-rich PAM, which is in contrast to the G-rich PAM following the target DNA for Type II systems. Thus, Type V systems cleave at a point that is distant from the PAM, while Type II systems cleave at a point that is adjacent to the PAM. In addition, in contrast to Type II systems, Cpf1 cleaves DNA via a staggered DNA double-stranded break with a 4 or 5 nucleotide 5' overhang. Type II systems cleave via a blunt double-stranded break. Similar to Type II systems, Cpf1 contains a predicted RuvC-like endonuclease domain, but lacks a second HNH endonuclease domain, which is in contrast to Type II systems.

### Cas Genes/Polypeptides and Protospacer Adjacent Motifs

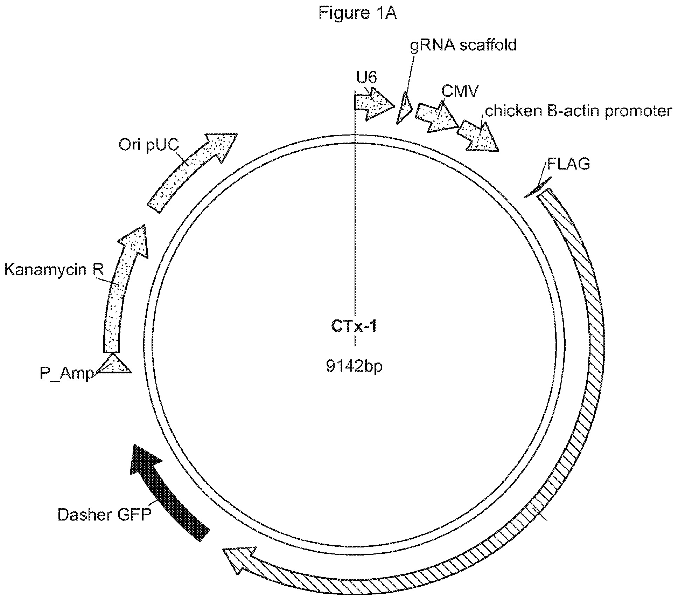

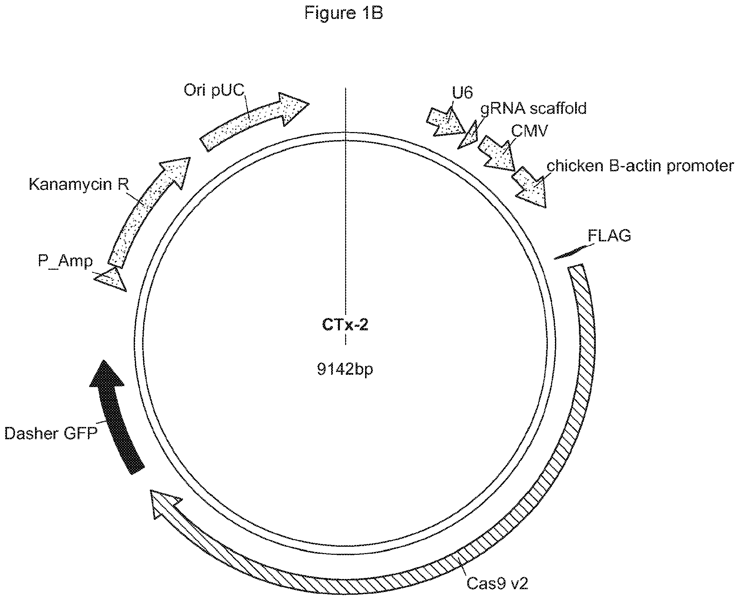

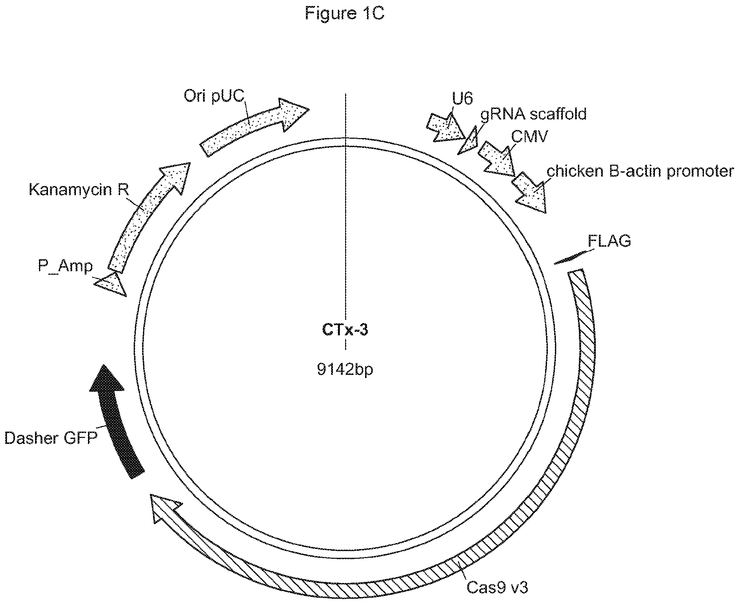

Exemplary CRISPR/Cas polypeptides include the Cas9 polypeptides in Fig. 1 of Fonfara et al., Nucleic Acids Research, 42: 2577-2590 (2014). The CRISPR/Cas gene naming system has undergone extensive rewriting since the Cas genes were discovered. Fig. 5 of Fonfara, *supra,* provides PAM sequences for the Cas9 polypeptides from various species.

### Site-Directed Polypeptides

A site-directed polypeptide is a nuclease used in genome editing to cleave DNA. The site-directed nuclease or polypeptide can be administered to a cell or a patient as either: one or more polypeptides, or one or more mRNAs encoding the polypeptide.

In the context of a CRISPR/Cas or CRISPR/Cpf1 system, the site-directed polypeptide can bind to a guide RNA that, in turn, specifies the site in the target DNA to which the polypeptide is directed. In the CRISPR/Cas or CRISPR/Cpf1 systems disclosed herein, the site-directed polypeptide can be an endonuclease, such as a DNA endonuclease.

A site-directed polypeptide can comprises a plurality of nucleic acid-cleaving (i.e., nuclease) domains. Two or more nucleic acid-cleaving domains can be linked together via a linker. For example, the linker can comprise a flexible linker. Linkers can comprise 1, 2, 3, 4, 5, 6, 7, 8, 9, 10, 11, 12, 13, 14, 15, 16, 17, 18, 19, 20, 21, 22, 23, 24, 25, 30, 35, 40 or more amino acids in length.

Naturally-occurring wild-type Cas9 enzymes comprise two nuclease domains, a HNH nuclease domain and a RuvC domain. Herein, the "Cas9" refers to both naturally-occurring and recombinant Cas9s. Cas9 enzymes contemplated herein can comprise a HNH or HNH-like nuclease domain, and/or a RuvC or RuvC-like nuclease domain.

HNH or HNH-like domains comprise a McrA-like fold. HNH or HNH-like domains comprises two antiparallel β-strands and an α-helix. HNH or HNH-like domains comprises a metal binding site (*e.g*., a divalent cation binding site). HNH or HNH-like domains can cleave one strand of a target nucleic acid (*e.g.*, the complementary strand of the crRNA targeted strand).

RuvC or RuvC-like domains comprise an RNaseH or RnaseH-like fold. RuvC/RnaseH domains are involved in a diverse set of nucleic acid-based functions including acting on both RNA and DNA. The RnaseH domain comprises 5 β-strands surrounded by a plurality of α-helices. RuvC/RnaseH or RuvC/RnaseH-like domains comprise a metal binding site (*e.g*., a divalent cation binding site). RuvC/RnaseH or RuvC/RnaseH-like domains can cleave one strand of a target nucleic acid (*e.g*., the non-complementary strand of a double-stranded target DNA).

Site-directed polypeptides can introduce double-strand breaks or single-strand breaks in nucleic acids, *e.g*., genomic DNA. The double-strand break can stimulate a cell's endogenous DNA-repair pathways (*e.g*., homology-dependent repair (HDR) or NHEJ or alternative non-homologous end joining (A-NHEJ) or microhomology-mediated end joining (MMEJ)). NHEJ can repair cleaved target nucleic acid without the need for a homologous template. This can sometimes result in small deletions or insertions (indels) in the target nucleic acid at the site of cleavage, and can lead to disruption or alteration of gene expression. HDR can occur when a homologous repair template, or donor, is available. The homologous donor template can comprise sequences that are homologous to sequences flanking the target nucleic acid cleavage site. The sister chromatid can be used by the cell as the repair template. However, for the purposes of genome editing, the repair template can be supplied as an exogenous nucleic acid, such as a plasmid, duplex oligonucleotide, single-strand oligonucleotide or viral nucleic acid. With exogenous donor templates, an additional nucleic acid sequence (such as a transgene) or modification (such as a single or multiple base change or a deletion) can be introduced between the flanking regions of homology so that the additional or altered nucleic acid sequence also becomes incorporated into the target locus. MMEJ can result in a genetic outcome that is similar to NHEJ in that small deletions and insertions can occur at the cleavage site. MMEJ can make use of homologous sequences of a few basepairs flanking the cleavage site to drive a favored end-joining DNA repair outcome. In some instances it may be possible to predict likely repair outcomes based on analysis of potential microhomologies in the nuclease target regions.

Thus, in some cases, homologous recombination can be used to insert an exogenous polynucleotide sequence into the target nucleic acid cleavage site. An exogenous polynucleotide sequence is termed a donor polynucleotide (or donor or donor sequence) herein. The donor polynucleotide, a portion of the donor polynucleotide, a copy of the donor polynucleotide, or a portion of a copy of the donor polynucleotide can be inserted into the target nucleic acid cleavage site. The donor polynucleotide can be an exogenous polynucleotide sequence, *i.e*., a sequence that does not naturally occur at the target nucleic acid cleavage site.

The modifications of the target DNA due to NHEJ and/or HDR can lead to, for example, mutations, deletions, alterations, integrations, gene correction, gene replacement, gene tagging, transgene insertion, nucleotide deletion, gene disruption, translocations and/or gene mutation. The processes of deleting genomic DNA and integrating non-native nucleic acid into genomic DNA are examples of genome editing.

The site-directed polypeptide can comprise an amino acid sequence having at least 10%, at least 15%, at least 20%, at least 30%, at least 40%, at least 50%, at least 60%, at least 70%, at least 75%, at least 80%, at least 85%, at least 90%, at least 95%, at least 99%, or 100% amino acid sequence identity to a wild-type exemplary site-directed polypeptide [*e.g*., Cas9 from *S*. *pyogenes,* US2014/0068797 Sequence ID No. 8 or Sapranauskas et al., Nucleic Acids Res, 39(21): 9275-9282 (2011)], and various other site-directed polypeptides. The site-directed polypeptide can comprise at least 70, 75, 80, 85, 90, 95, 97, 99, or 100% identity to a wild-type site-directed polypeptide (*e.g*., Cas9 from *S*. *pyogenes, supra*) over 10 contiguous amino acids. The site-directed polypeptide can comprise at most: 70, 75, 80, 85, 90, 95, 97, 99, or 100% identity to a wild-type site-directed polypeptide (*e.g*., Cas9 from *S*. *pyogenes, supra*) over 10 contiguous amino acids. The site-directed polypeptide can comprise at least: 70, 75, 80, 85, 90, 95, 97, 99, or 100% identity to a wild-type site-directed polypeptide (*e.g*., Cas9 from *S*. *pyogenes, supra*) over 10 contiguous amino acids in a HNH nuclease domain of the site-directed polypeptide. The site-directed polypeptide can comprise at most: 70, 75, 80, 85, 90, 95, 97, 99, or 100% identity to a wild-type site-directed polypeptide (*e.g*., Cas9 from *S*. *pyogenes, supra)* over 10 contiguous amino acids in a HNH nuclease domain of the site-directed polypeptide. The site-directed polypeptide can comprise at least: 70, 75, 80, 85, 90, 95, 97, 99, or 100% identity to a wild-type site-directed polypeptide (*e.g*., Cas9 from *S*. *pyogenes, supra)* over 10 contiguous amino acids in a RuvC nuclease domain of the site-directed polypeptide. The site-directed polypeptide can comprise at most: 70, 75, 80, 85, 90, 95, 97, 99, or 100% identity to a wild-type site-directed polypeptide (*e.g*., Cas9 from *S*. *pyogenes, supra*) over 10 contiguous amino acids in a RuvC nuclease domain of the site-directed polypeptide.

The site-directed polypeptide can comprise a modified form of a wild-type exemplary site-directed polypeptide. The modified form of the wild- type exemplary site-directed polypeptide can comprise a mutation that reduces the nucleic acid-cleaving activity of the site-directed polypeptide. The modified form of the wild-type exemplary site-directed polypeptide can have less than 90%, less than 80%, less than 70%, less than 60%, less than 50%, less than 40%, less than 30%, less than 20%, less than 10%, less than 5%, or less than 1% of the nucleic acid-cleaving activity of the wild-type exemplary site-directed polypeptide (*e.g*., Cas9 from *S*. *pyogenes, supra*). The modified form of the site-directed polypeptide can have no substantial nucleic acid-cleaving activity. When a site-directed polypeptide is a modified form that has no substantial nucleic acid-cleaving activity, it is referred to herein as "enzymatically inactive."

The modified form of the site-directed polypeptide can comprise a mutation such that it can induce a single-strand break (SSB) on a target nucleic acid (*e.g*., by cutting only one of the sugar-phosphate backbones of a double-strand target nucleic acid). The mutation can result in less than 90%, less than 80%, less than 70%, less than 60%, less than 50%, less than 40%, less than 30%, less than 20%, less than 10%, less than 5%, or less than 1% of the nucleic acid-cleaving activity in one or more of the plurality of nucleic acid-cleaving domains of the wild-type site directed polypeptide (*e.g*., Cas9 from *S*. *pyogenes, supra*). The mutation can result in one or more of the plurality of nucleic acid-cleaving domains retaining the ability to cleave the complementary strand of the target nucleic acid, but reducing its ability to cleave the non-complementary strand of the target nucleic acid. The mutation can result in one or more of the plurality of nucleic acid-cleaving domains retaining the ability to cleave the non-complementary strand of the target nucleic acid, but reducing its ability to cleave the complementary strand of the target nucleic acid. For example, residues in the wild-type exemplary *S*. *pyogenes* Cas9 polypeptide, such as Asp10, His840, Asn854 and Asn856, are mutated to inactivate one or more of the plurality of nucleic acid-cleaving domains (*e.g*., nuclease domains). The residues to be mutated can correspond to residues Asp10, His840, Asn854 and Asn856 in the wild-type exemplary *S*. *pyogenes* Cas9 polypeptide (*e.g*., as determined by sequence and/or structural alignment). Non-limiting examples of mutations include D10A, H840A, N854A or N856A. One skilled in the art will recognize that mutations other than alanine substitutions can be suitable.

A D10A mutation can be combined with one or more of H840A, N854A, or N856A mutations to produce a site-directed polypeptide substantially lacking DNA cleavage activity. A H840A mutation can be combined with one or more of D10A, N854A, or N856A mutations to produce a site-directed polypeptide substantially lacking DNA cleavage activity. A N854A mutation can be combined with one or more of H840A, D10A, or N856A mutations to produce a site-directed polypeptide substantially lacking DNA cleavage activity. A N856A mutation can be combined with one or more of H840A, N854A, or D10A mutations to produce a site-directed polypeptide substantially lacking DNA cleavage activity. Site-directed polypeptides that comprise one substantially inactive nuclease domain are referred to as "nickases".

Nickase variants of RNA-guided endonucleases, for example Cas9, can be used to increase the specificity of CRISPR-mediated genome editing. Wild type Cas9 is typically guided by a single guide RNA designed to hybridize with a specified ~20 nucleotide sequence in the target sequence (such as an endogenous genomic locus). However, several mismatches can be tolerated between the guide RNA and the target locus, effectively reducing the length of required homology in the target site to, for example, as little as 13 nt of homology, and thereby resulting in elevated potential for binding and double-strand nucleic acid cleavage by the CRISPR/Cas9 complex elsewhere in the target genome - also known as off-target cleavage. Because nickase variants of Cas9 each only cut one strand, in order to create a double-strand break it is necessary for a pair of nickases to bind in close proximity and on opposite strands of the target nucleic acid, thereby creating a pair of nicks, which is the equivalent of a double-strand break. This requires that two separate guide RNAs - one for each nickase - must bind in close proximity and on opposite strands of the target nucleic acid. This requirement essentially doubles the minimum length of homology needed for the double-strand break to occur, thereby reducing the likelihood that a double-strand cleavage event will occur elsewhere in the genome, where the two guide RNA sites - if they exist - are unlikely to be sufficiently close to each other to enable the double-strand break to form. As described in the art, nickases can also be used to promote HDR versus NHEJ. HDR can be used to introduce selected changes into target sites in the genome through the use of specific donor sequences that effectively mediate the desired changes.

Mutations contemplated can include substitutions, additions, and deletions, or any combination thereof. The mutation converts the mutated amino acid to alanine. The mutation converts the mutated amino acid to another amino acid (*e.g*., glycine, serine, threonine, cysteine, valine, leucine, isoleucine, methionine, proline, phenylalanine, tyrosine, tryptophan, aspartic acid, glutamic acid, asparagines, glutamine, histidine, lysine, or arginine). The mutation converts the mutated amino acid to a non-natural amino acid (*e.g*., selenomethionine). The mutation converts the mutated amino acid to amino acid mimics (*e.g*., phosphomimics). The mutation can be a conservative mutation. For example, the mutation converts the mutated amino acid to amino acids that resemble the size, shape, charge, polarity, conformation, and/or rotamers of the mutated amino acids *(e.g*., cysteine/serine mutation, lysine/asparagine mutation, histidine/phenylalanine mutation). The mutation can cause a shift in reading frame and/or the creation of a premature stop codon. Mutations can cause changes to regulatory regions of genes or loci that affect expression of one or more genes.

The site-directed polypeptide (*e.g*., variant, mutated, enzymatically inactive and/or conditionally enzymatically inactive site-directed polypeptide) can target nucleic acid. The site-directed polypeptide (*e.g*., variant, mutated, enzymatically inactive and/or conditionally enzymatically inactive endoribonuclease) can target DNA. The site-directed polypeptide (*e.g.*, variant, mutated, enzymatically inactive and/or conditionally enzymatically inactive endoribonuclease) can target RNA.

The site-directed polypeptide can comprise one or more non-native sequences (*e.g.*, the site-directed polypeptide is a fusion protein).

The site-directed polypeptide can comprise an amino acid sequence comprising at least 15% amino acid identity to a Cas9 from a bacterium (*e.g.*, *S*. *pyogenes*), a nucleic acid binding domain, and two nucleic acid cleaving domains (*i.e*., a HNH domain and a RuvC domain).

The site-directed polypeptide can comprise an amino acid sequence comprising at least 15% amino acid identity to a Cas9 from a bacterium (*e.g.*, *S*. *pyogenes*), and two nucleic acid cleaving domains (*i.e*., a HNH domain and a RuvC domain).

The site-directed polypeptide can comprise an amino acid sequence comprising at least 15% amino acid identity to a Cas9 from a bacterium (*e.g.*, *S*. *pyogenes*), and two nucleic acid cleaving domains, wherein one or both of the nucleic acid cleaving domains comprise at least 50% amino acid identity to a nuclease domain from Cas9 from a bacterium (*e.g.*, *S. pyogenes*).

The site-directed polypeptide can comprise an amino acid sequence comprising at least 15% amino acid identity to a Cas9 from a bacterium (*e.g.*, *S*. *pyogenes*), two nucleic acid cleaving domains (*i.e*., a HNH domain and a RuvC domain), and non-native sequence (for example, a nuclear localization signal) or a linker linking the site-directed polypeptide to a non-native sequence.

The site-directed polypeptide can comprise an amino acid sequence comprising at least 15% amino acid identity to a Cas9 from a bacterium (*e.g.*, *S*. *pyogenes*), two nucleic acid cleaving domains (*i.e*., a HNH domain and a RuvC domain), wherein the site-directed polypeptide comprises a mutation in one or both of the nucleic acid cleaving domains that reduces the cleaving activity of the nuclease domains by at least 50%.

The site-directed polypeptide can comprise an amino acid sequence comprising at least 15% amino acid identity to a Cas9 from a bacterium (*e.g*., *S*. *pyogenes*), and two nucleic acid cleaving domains (*i.e*., a HNH domain and a RuvC domain), wherein one of the nuclease domains comprises mutation of aspartic acid 10, and/or wherein one of the nuclease domains can comprise a mutation of histidine 840, and wherein the mutation reduces the cleaving activity of the nuclease domain(s) by at least 50%.

The one or more site-directed polypeptides, e.g. DNA endonucleases, can comprise two nickases that together effect one double-strand break at a specific locus in the genome, or four nickases that together effect or cause two double-strand breaks at specific loci in the genome. Alternatively, one site-directed polypeptide, e.g. DNA endonuclease, can effect or cause one double-strand break at a specific locus in the genome.

The site-directed polypeptide can be flanked at the N-terminus, the C-terminus, or both the N-terminus and C-terminus by one or more nuclear localization signals (NLSs). For example, a Cas9 endonuclease can be flanked by two NLSs, one NLS located at the N-terminus and the second NLS located at the C-terminus. The NLS can be any NLS known in the art, such as a SV40 NLS.

### Genome-targeting Nucleic Acid

The present disclosure provides a genome-targeting nucleic acid that can direct the activities of an associated polypeptide (*e.g*., a site-directed polypeptide) to a specific target sequence within a target nucleic acid. The genome-targeting nucleic acid can be an RNA. A genome-targeting RNA is referred to as a "guide RNA" or "gRNA" herein. A guide RNA can comprise at least a spacer sequence that hybridizes to a target nucleic acid sequence of interest, and a CRISPR repeat sequence. In Type II systems, the gRNA also comprises a second RNA called the tracrRNA sequence. In the Type II guide RNA (gRNA), the CRISPR repeat sequence and tracrRNA sequence hybridize to each other to form a duplex. In the Type V guide RNA (gRNA), the crRNA forms a duplex. In both systems, the duplex can bind a site-directed polypeptide, such that the guide RNA and site-direct polypeptide form a complex. The genome-targeting nucleic acid can provide target specificity to the complex by virtue of its association with the site-directed polypeptide. The genome-targeting nucleic acid thus can direct the activity of the site-directed polypeptide.

Exemplary guide RNAs include the spacer sequences in SEQ ID NOs: 1 - 71,947 and the sgRNA sequences in SEQ ID NOs: 71,950-71,959 of the Sequence Listing. As is understood by the person of ordinary skill in the art, each guide RNA can be designed to include a spacer sequence complementary to its genomic target sequence. For example, each of the spacer sequences in SEQ ID NOs: 1 - 71,947 of the Sequence Listing can be put into a single RNA chimera or a crRNA (along with a corresponding tracrRNA). See Jinek et al., Science, 337, 816-821 (2012) and Deltcheva et al., Nature, 471, 602-607 (2011) or Table 1.

The genome-targeting nucleic acid can be a double-molecule guide RNA. The genome-targeting nucleic acid can be a single-molecule guide RNA.

A double-molecule guide RNA can comprise two strands of RNA. The first strand comprises in the 5' to 3' direction, an optional spacer extension sequence, a spacer sequence and a minimum CRISPR repeat sequence. The second strand can comprise a minimum tracrRNA sequence (complementary to the minimum CRISPR repeat sequence), a 3' tracrRNA sequence and an optional tracrRNA extension sequence.

A single-molecule guide RNA (sgRNA) in a Type II system can comprise, in the 5' to 3' direction, an optional spacer extension sequence, a spacer sequence, a minimum CRISPR repeat sequence, a single-molecule guide linker, a minimum tracrRNA sequence, a 3' tracrRNA sequence and an optional tracrRNA extension sequence. The optional tracrRNA extension can comprise elements that contribute additional functionality (*e.g*., stability) to the guide RNA. The single-molecule guide linker can link the minimum CRISPR repeat and the minimum tracrRNA sequence to form a hairpin structure. The optional tracrRNA extension can comprise one or more hairpins.

A single-molecule guide RNA (sgRNA) in a Type V system can comprise, in the 5' to 3' direction, a minimum CRISPR repeat sequence and a spacer sequence.

The sgRNA can comprise a 20 nucleotide spacer sequence at the 5' end of the sgRNA sequence. The sgRNA can comprise a less than a 20 nucleotide spacer sequence at the 5' end of the sgRNA sequence. The sgRNA can comprise a more than 20 nucleotide spacer sequence at the 5' end of the sgRNA sequence. The sgRNA can comprise a variable length spacer sequence with 17-30 nucleotides at the 5' end of the sgRNA sequence (see Table 1).

The sgRNA can comprise no uracil at the 3'end of the sgRNA sequence, such as in SEQ ID NO: 71,961 of Table 1. The sgRNA can comprise one or more uracil at the 3'end of the sgRNA sequence, such as in SEQ ID NO: 71,962 in Table 1. For example, the sgRNA can comprise 1 uracil (U) at the 3' end of the sgRNA sequence. The sgRNA can comprise 2 uracil (UU) at the 3' end of the sgRNA sequence. The sgRNA can comprise 3 uracil (UUU) at the 3' end of the sgRNA sequence. The sgRNA can comprise 4 uracil (UUUU) at the 3' end of the sgRNA sequence. The sgRNA can comprise 5 uracil (UUUUU) at the 3' end of the sgRNA sequence. The sgRNA can comprise 6 uracil (UUUUUU) at the 3' end of the sgRNA sequence. The sgRNA can comprise 7 uracil (UUUUUUU) at the 3' end of the sgRNA sequence. The sgRNA can comprise 8 uracil (UUUUUUUU) at the 3' end of the sgRNA sequence.

The sgRNA can be unmodified or modified. For example, modified sgRNAs can comprise one or more 2'-O-methyl phosphorothioate nucleotides.

**Table 1**

| **SEQ ID NO.** | **sgRNA sequence** |
|---|---|
| 71,960 | nnnnnnnnnnnnnnnnnnnguuuuagagcuagaaauagcaaguuaaaauaaggcuaguccguua ucaacuugaaaaaguggcaccgagucggugcuuuu |
| 71,961 | nnnnnnnnnnnnnnnnnnnnguuuuagagcuagaaauagcaaguuaaaauaaggcuaguccguua ucaacuugaaaaaguggcaccgagucggugc |
| 71,962 | n(17-30)guuuuagagcuagaaauagcaaguuaaaauaaggcuaguccguuaucaacuugaaaaagu ggcaccgagucggugcu(1-8) |

By way of illustration, guide RNAs used in the CRISPR/Cas/Cpf1 system, or other smaller RNAs can be readily synthesized by chemical means, as illustrated below and described in the art. While chemical synthetic procedures are continually expanding, purifications of such RNAs by procedures such as high performance liquid chromatography (HPLC, which avoids the use of gels such as PAGE) tends to become more challenging as polynucleotide lengths increase significantly beyond a hundred or so nucleotides. One approach used for generating RNAs of greater length is to produce two or more molecules that are ligated together. Much longer RNAs, such as those encoding a Cas9 or Cpf1 endonuclease, are more readily generated enzymatically. Various types of RNA modifications can be introduced during or after chemical synthesis and/or enzymatic generation of RNAs, e.g., modifications that enhance stability, reduce the likelihood or degree of innate immune response, and/or enhance other attributes, as described in the art.

### Spacer Extension Sequence

In some examples of genome-targeting nucleic acids, a spacer extension sequence can modify activity, provide stability and/or provide a location for modifications of a genome-targeting nucleic acid. A spacer extension sequence can modify on- or off-target activity or specificity. In some examples, a spacer extension sequence can be provided. The spacer extension sequence can have a length of more than 1, 5, 10, 15, 20, 25, 30, 35, 40, 45, 50, 60, 70, 80, 90, 100, 120, 140, 160, 180, 200, 220, 240, 260, 280, 300, 320, 340, 360, 380, 400, 1000, 2000, 3000, 4000, 5000, 6000, or 7000 or more nucleotides. The spacer extension sequence can have a length of less than 1, 5, 10, 15, 20, 25, 30, 35, 40, 45, 50, 60, 70, 80, 90, 100, 120, 140, 160, 180, 200, 220, 240, 260, 280, 300, 320, 340, 360, 380, 400, 1000, 2000, 3000, 4000, 5000, 6000, 7000 or more nucleotides. The spacer extension sequence can be less than 10 nucleotides in length. The spacer extension sequence can be between 10-30 nucleotides in length. The spacer extension sequence can be between 30-70 nucleotides in length.

The spacer extension sequence can comprise another moiety (*e.g.,* a stability control sequence, an endoribonuclease binding sequence, a ribozyme). The moiety can decrease or increase the stability of a nucleic acid targeting nucleic acid. The moiety can be a transcriptional terminator segment (*i.e.,* a transcription termination sequence). The moiety can function in a eukaryotic cell. The moiety can function in a prokaryotic cell. The moiety can function in both eukaryotic and prokaryotic cells. Non-limiting examples of suitable moieties include: a 5' cap (*e.g.,* a 7-methylguanylate cap (m7 G)), a riboswitch sequence (*e.g.,* to allow for regulated stability and/or regulated accessibility by proteins and protein complexes), a sequence that forms a dsRNA duplex (*i.e.,* a hairpin), a sequence that targets the RNA to a subcellular location (*e.g.,* nucleus, mitochondria, chloroplasts, and the like), a modification or sequence that provides for tracking (*e.g.,* direct conjugation to a fluorescent molecule, conjugation to a moiety that facilitates fluorescent detection, a sequence that allows for fluorescent detection, etc.), and/or a modification or sequence that provides a binding site for proteins (*e.g.,* proteins that act on DNA, including transcriptional activators, transcriptional controls, DNA methyltransferases, DNA demethylases, histone acetyltransferases, histone deacetylases, and the like).

### Spacer Sequence

The spacer sequence hybridizes to a sequence in a target nucleic acid of interest. The spacer of a genome-targeting nucleic acid can interact with a target nucleic acid in a sequence-specific manner via hybridization (*i.e.,* base pairing). The nucleotide sequence of the spacer can vary depending on the sequence of the target nucleic acid of interest.

In a CRISPR/Cas system herein, the spacer sequence can be designed to hybridize to a target nucleic acid that is located 5' of a PAM of the Cas9 enzyme used in the system. The spacer may perfectly match the target sequence or may have mismatches. Each Cas9 enzyme has a particular PAM sequence that it recognizes in a target DNA. For example, *S. pyogenes* recognizes in a target nucleic acid a PAM that comprises the sequence 5'-NRG-3', where R comprises either A or G, where N is any nucleotide and N is immediately 3' of the target nucleic acid sequence targeted by the spacer sequence.

The target nucleic acid sequence can comprise 20 nucleotides. The target nucleic acid can comprise less than 20 nucleotides. The target nucleic acid can comprise more than 20 nucleotides. The target nucleic acid can comprise at least: 5, 10, 15, 16, 17, 18, 19, 20, 21, 22, 23, 24, 25, 30 or more nucleotides. The target nucleic acid can comprise at most: 5, 10, 15, 16, 17, 18, 19, 20, 21, 22, 23, 24, 25, 30 or more nucleotides. The target nucleic acid sequence can comprise 20 bases immediately 5' of the first nucleotide of the PAM. For example, in a sequence comprising 5'-NNNNNNNNNNNNNNNNNNNNNRG-3' (SEQ ID NO: 71,948), the target nucleic acid can comprise the sequence that corresponds to the Ns, wherein N is any nucleotide, and the underlined NRG sequence is the *S. pyogenes* PAM.

The spacer sequence that hybridizes to the target nucleic acid can have a length of at least about 6 nucleotides (nt). The spacer sequence can be at least about 6 nt, at least about 10 nt, at least about 15 nt, at least about 18 nt, at least about 19 nt, at least about 20 nt, at least about 25 nt, at least about 30 nt, at least about 35 nt or at least about 40 nt, from about 6 nt to about 80 nt, from about 6 nt to about 50 nt, from about 6 nt to about 45 nt, from about 6 nt to about 40 nt, from about 6 nt to about 35 nt, from about 6 nt to about 30 nt, from about 6 nt to about 25 nt, from about 6 nt to about 20 nt, from about 6 nt to about 19 nt, from about 10 nt to about 50 nt, from about 10 nt to about 45 nt, from about 10 nt to about 40 nt, from about 10 nt to about 35 nt, from about 10 nt to about 30 nt, from about 10 nt to about 25 nt, from about 10 nt to about 20 nt, from about 10 nt to about 19 nt, from about 19 nt to about 25 nt, from about 19 nt to about 30 nt, from about 19 nt to about 35 nt, from about 19 nt to about 40 nt, from about 19 nt to about 45 nt, from about 19 nt to about 50 nt, from about 19 nt to about 60 nt, from about 20 nt to about 25 nt, from about 20 nt to about 30 nt, from about 20 nt to about 35 nt, from about 20 nt to about 40 nt, from about 20 nt to about 45 nt, from about 20 nt to about 50 nt, or from about 20 nt to about 60 nt. In some examples, the spacer sequence can comprise 20 nucleotides. In some examples, the spacer can comprise 19 nucleotides.

In some examples, the percent complementarity between the spacer sequence and the target nucleic acid is at least about 30%, at least about 40%, at least about 50%, at least about 60%, at least about 65%, at least about 70%, at least about 75%, at least about 80%, at least about 85%, at least about 90%, at least about 95%, at least about 97%, at least about 98%, at least about 99%, or 100%. In some examples, the percent complementarity between the spacer sequence and the target nucleic acid is at most about 30%, at most about 40%, at most about 50%, at most about 60%, at most about 65%, at most about 70%, at most about 75%, at most about 80%, at most about 85%, at most about 90%, at most about 95%, at most about 97%, at most about 98%, at most about 99%, or 100%. In some examples, the percent complementarity between the spacer sequence and the target nucleic acid is 100% over the six contiguous 5'-most nucleotides of the target sequence of the complementary strand of the target nucleic acid. The percent complementarity between the spacer sequence and the target nucleic acid can be at least 60% over about 20 contiguous nucleotides. The length of the spacer sequence and the target nucleic acid can differ by 1 to 6 nucleotides, which may be thought of as a bulge or bulges.

The spacer sequence can be designed or chosen using a computer program. The computer program can use variables, such as predicted melting temperature, secondary structure formation, predicted annealing temperature, sequence identity, genomic context, chromatin accessibility, % GC, frequency of genomic occurrence (*e.g.,* of sequences that are identical or are similar but vary in one or more spots as a result of mismatch, insertion or deletion), methylation status, presence of SNPs, and the like.

### Minimum CRISPR Repeat Sequence

A minimum CRISPR repeat sequence can be a sequence with at least about 30%, about 40%, about 50%, about 60%, about 65%, about 70%, about 75%, about 80%, about 85%, about 90%, about 95%, or 100% sequence identity to a reference CRISPR repeat sequence (*e.g.,* crRNA from *S. pyogenes).*

A minimum CRISPR repeat sequence can comprise nucleotides that can hybridize to a minimum tracrRNA sequence in a cell. The minimum CRISPR repeat sequence and a minimum tracrRNA sequence can form a duplex, *i.e.* a base-paired double-stranded structure. Together, the minimum CRISPR repeat sequence and the minimum tracrRNA sequence can bind to the site-directed polypeptide. At least a part of the minimum CRISPR repeat sequence can hybridize to the minimum tracrRNA sequence. At least a part of the minimum CRISPR repeat sequence can comprise at least about 30%, about 40%, about 50%, about 60%, about 65%, about 70%, about 75%, about 80%, about 85%, about 90%, about 95%, or 100% complementary to the minimum tracrRNA sequence. At least a part of the minimum CRISPR repeat sequence can comprise at most about 30%, about 40%, about 50%, about 60%, about 65%, about 70%, about 75%, about 80%, about 85%, about 90%, about 95%, or 100% complementary to the minimum tracrRNA sequence.

The minimum CRISPR repeat sequence can have a length from about 7 nucleotides to about 100 nucleotides. For example, the length of the minimum CRISPR repeat sequence is from about 7 nucleotides (nt) to about 50 nt, from about 7 nt to about 40 nt, from about 7 nt to about 30 nt, from about 7 nt to about 25 nt, from about 7 nt to about 20 nt, from about 7 nt to about 15 nt, from about 8 nt to about 40 nt, from about 8 nt to about 30 nt, from about 8 nt to about 25 nt, from about 8 nt to about 20 nt, from about 8 nt to about 15 nt, from about 15 nt to about 100 nt, from about 15 nt to about 80 nt, from about 15 nt to about 50 nt, from about 15 nt to about 40 nt, from about 15 nt to about 30 nt, or from about 15 nt to about 25 nt. In some examples, the minimum CRISPR repeat sequence can be approximately 9 nucleotides in length. The minimum CRISPR repeat sequence can be approximately 12 nucleotides in length.

The minimum CRISPR repeat sequence can be at least about 60% identical to a reference minimum CRISPR repeat sequence (*e.g.,* wild-type crRNA from *S. pyogenes*) over a stretch of at least 6, 7, or 8 contiguous nucleotides. For example, the minimum CRISPR repeat sequence can be at least about 65% identical, at least about 70% identical, at least about 75% identical, at least about 80% identical, at least about 85% identical, at least about 90% identical, at least about 95% identical, at least about 98% identical, at least about 99% identical or 100% identical to a reference minimum CRISPR repeat sequence over a stretch of at least 6, 7, or 8 contiguous nucleotides.

### Minimum tracrRNA Sequence

A minimum tracrRNA sequence can be a sequence with at least about 30%, about 40%, about 50%, about 60%, about 65%, about 70%, about 75%, about 80%, about 85%, about 90%, about 95%, or 100% sequence identity to a reference tracrRNA sequence (*e.g.,* wild type tracrRNA from *S. pyogenes).*

A minimum tracrRNA sequence can comprise nucleotides that hybridize to a minimum CRISPR repeat sequence in a cell. A minimum tracrRNA sequence and a minimum CRISPR repeat sequence form a duplex, *i.e.* a base-paired double-stranded structure. Together, the minimum tracrRNA sequence and the minimum CRISPR repeat can bind to a site-directed polypeptide. At least a part of the minimum tracrRNA sequence can hybridize to the minimum CRISPR repeat sequence. The minimum tracrRNA sequence can be at least about 30%, about 40%, about 50%, about 60%, about 65%, about 70%, about 75%, about 80%, about 85%, about 90%, about 95%, or 100% complementary to the minimum CRISPR repeat sequence.

The minimum tracrRNA sequence can have a length from about 7 nucleotides to about 100 nucleotides. For example, the minimum tracrRNA sequence can be from about 7 nucleotides (nt) to about 50 nt, from about 7 nt to about 40 nt, from about 7 nt to about 30 nt, from about 7 nt to about 25 nt, from about 7 nt to about 20 nt, from about 7 nt to about 15 nt, from about 8 nt to about 40 nt, from about 8 nt to about 30 nt, from about 8 nt to about 25 nt, from about 8 nt to about 20 nt, from about 8 nt to about 15 nt, from about 15 nt to about 100 nt, from about 15 nt to about 80 nt, from about 15 nt to about 50 nt, from about 15 nt to about 40 nt, from about 15 nt to about 30 nt or from about 15 nt to about 25 nt long. The minimum tracrRNA sequence can be approximately 9 nucleotides in length. The minimum tracrRNA sequence can be approximately 12 nucleotides. The minimum tracrRNA can consist of tracrRNA nt 23-48 described in Jinek *et al., supra.*

The minimum tracrRNA sequence can be at least about 60% identical to a reference minimum tracrRNA (e.g., wild type, tracrRNA from *S. pyogenes*) sequence over a stretch of at least 6, 7, or 8 contiguous nucleotides. For example, the minimum tracrRNA sequence can be at least about 65% identical, about 70% identical, about 75% identical, about 80% identical, about 85% identical, about 90% identical, about 95% identical, about 98% identical, about 99% identical or 100% identical to a reference minimum tracrRNA sequence over a stretch of at least 6, 7, or 8 contiguous nucleotides.

The duplex between the minimum CRISPR RNA and the minimum tracrRNA can comprise a double helix. The duplex between the minimum CRISPR RNA and the minimum tracrRNA can comprise at least about 1, 2, 3, 4, 5, 6, 7, 8, 9, or 10 or more nucleotides. The duplex between the minimum CRISPR RNA and the minimum tracrRNA can comprise at most about 1, 2, 3, 4, 5, 6, 7, 8, 9, or 10 or more nucleotides.

The duplex can comprise a mismatch (*i.e.,* the two strands of the duplex are not 100% complementary). The duplex can comprise at least about 1, 2, 3, 4, or 5 or mismatches. The duplex can comprise at most about 1, 2, 3, 4, or 5 or mismatches. The duplex can comprise no more than 2 mismatches.

### Bulges

In some cases, there can be a "bulge" in the duplex between the minimum CRISPR RNA and the minimum tracrRNA. A bulge is an unpaired region of nucleotides within the duplex. A bulge can contribute to the binding of the duplex to the site-directed polypeptide. The bulge can comprise, on one side of the duplex, an unpaired 5'-XXXY-3' where X is any purine and Y comprises a nucleotide that can form a wobble pair with a nucleotide on the opposite strand, and an unpaired nucleotide region on the other side of the duplex. The number of unpaired nucleotides on the two sides of the duplex can be different.

In one example, the bulge can comprise an unpaired purine (*e.g.,* adenine) on the minimum CRISPR repeat strand of the bulge. In some examples, the bulge can comprise an unpaired 5'-AAGY-3' of the minimum tracrRNA sequence strand of the bulge, where Y comprises a nucleotide that can form a wobble pairing with a nucleotide on the minimum CRISPR repeat strand.

A bulge on the minimum CRISPR repeat side of the duplex can comprise at least 1, 2, 3, 4, or 5 or more unpaired nucleotides. A bulge on the minimum CRISPR repeat side of the duplex can comprise at most 1, 2, 3, 4, or 5 or more unpaired nucleotides. A bulge on the minimum CRISPR repeat side of the duplex can comprise 1 unpaired nucleotide.

A bulge on the minimum tracrRNA sequence side of the duplex can comprise at least 1, 2, 3, 4, 5, 6, 7, 8, 9, or 10 or more unpaired nucleotides. A bulge on the minimum tracrRNA sequence side of the duplex can comprise at most 1, 2, 3, 4, 5, 6, 7, 8, 9, or 10 or more unpaired nucleotides. A bulge on a second side of the duplex (*e.g.,* the minimum tracrRNA sequence side of the duplex) can comprise 4 unpaired nucleotides.

A bulge can comprise at least one wobble pairing. In some examples, a bulge can comprise at most one wobble pairing. A bulge can comprise at least one purine nucleotide. A bulge can comprise at least 3 purine nucleotides. A bulge sequence can comprises at least 5 purine nucleotides. A bulge sequence can comprise at least one guanine nucleotide. In some examples, a bulge sequence can comprise at least one adenine nucleotide.

### Hairpins

In various examples, one or more hairpins can be located 3' to the minimum tracrRNA in the 3' tracrRNA sequence.

The hairpin can start at least about 1, 2, 3, 4, 5, 6, 7, 8, 9, 10, 15, or 20 or more nucleotides 3' from the last paired nucleotide in the minimum CRISPR repeat and minimum tracrRNA sequence duplex. The hairpin can start at most about 1, 2, 3, 4, 5, 6, 7, 8, 9 or 10 or more nucleotides 3' of the last paired nucleotide in the minimum CRISPR repeat and minimum tracrRNA sequence duplex.

The hairpin can comprise at least about 1, 2, 3, 4, 5, 6, 7, 8, 9, 10, 15, or 20 or more consecutive nucleotides. The hairpin can comprise at most about 1, 2, 3, 4, 5, 6, 7, 8, 9, 10, 15, or more consecutive nucleotides.

The hairpin can comprise a CC dinucleotide (i.e., two consecutive cytosine nucleotides).

The hairpin can comprise duplexed nucleotides (*e.g.,* nucleotides in a hairpin, hybridized together). For example, a hairpin can comprise a CC dinucleotide that is hybridized to a GG dinucleotide in a hairpin duplex of the 3' tracrRNA sequence.

One or more of the hairpins can interact with guide RNA-interacting regions of a site-directed polypeptide.

In some examples, there are two or more hairpins, and in other examples there are three or more hairpins.

### 3' tracrRNA sequence

A 3' tracrRNA sequence can comprise a sequence with at least about 30%, about 40%, about 50%, about 60%, about 65%, about 70%, about 75%, about 80%, about 85%, about 90%, about 95%, or 100% sequence identity to a reference tracrRNA sequence (*e.g.,* a tracrRNA from *S. pyogenes).*

The 3' tracrRNA sequence can have a length from about 6 nucleotides to about 100 nucleotides. For example, the 3' tracrRNA sequence can have a length from about 6 nucleotides (nt) to about 50 nt, from about 6 nt to about 40 nt, from about 6 nt to about 30 nt, from about 6 nt to about 25 nt, from about 6 nt to about 20 nt, from about 6 nt to about 15 nt, from about 8 nt to about 40 nt, from about 8 nt to about 30 nt, from about 8 nt to about 25 nt, from about 8 nt to about 20 nt, from about 8 nt to about 15 nt, from about 15 nt to about 100 nt, from about 15 nt to about 80 nt, from about 15 nt to about 50 nt, from about 15 nt to about 40 nt, from about 15 nt to about 30 nt, or from about 15 nt to about 25 nt. The 3' tracrRNA sequence can have a length of approximately 14 nucleotides.

The 3' tracrRNA sequence can be at least about 60% identical to a reference 3' tracrRNA sequence (*e.g.,* wild type 3' tracrRNA sequence from *S. pyogenes)* over a stretch of at least 6, 7, or 8 contiguous nucleotides. For example, the 3' tracrRNA sequence can be at least about 60% identical, about 65% identical, about 70% identical, about 75% identical, about 80% identical, about 85% identical, about 90% identical, about 95% identical, about 98% identical, about 99% identical, or 100% identical, to a reference 3' tracrRNA sequence (*e.g.,* wild type 3' tracrRNA sequence from S. *pyogenes*) over a stretch of at least 6, 7, or 8 contiguous nucleotides.

The 3' tracrRNA sequence can comprise more than one duplexed region (e.g., hairpin, hybridized region). The 3' tracrRNA sequence can comprise two duplexed regions.

The 3' tracrRNA sequence can comprise a stem loop structure. The stem loop structure in the 3' tracrRNA can comprise at least 1, 2, 3, 4, 5, 6, 7, 8, 9, 10, 15 or 20 or more nucleotides. The stem loop structure in the 3' tracrRNA can comprise at most 1, 2, 3, 4, 5, 6, 7, 8, 9 or 10 or more nucleotides. The stem loop structure can comprise a functional moiety. For example, the stem loop structure can comprise an aptamer, a ribozyme, a protein-interacting hairpin, a CRISPR array, an intron, or an exon. The stem loop structure can comprise at least about 1, 2, 3, 4, or 5 or more functional moieties. The stem loop structure can comprise at most about 1, 2, 3, 4, or 5 or more functional moieties.

The hairpin in the 3' tracrRNA sequence can comprise a P-domain. In some examples, the P-domain can comprise a double-stranded region in the hairpin.

### tracrRNA Extension Sequence

A tracrRNA extension sequence may be provided whether the tracrRNA is in the context of single-molecule guides or double-molecule guides. The tracrRNA extension sequence can have a length from about 1 nucleotide to about 400 nucleotides. The tracrRNA extension sequence can have a length of more than 1, 5, 10, 15, 20, 25, 30, 35, 40, 45, 50, 60, 70, 80, 90, 100, 120, 140, 160, 180, 200, 220, 240, 260, 280, 300, 320, 340, 360, 380, or 400 nucleotides. The tracrRNA extension sequence can have a length from about 20 to about 5000 or more nucleotides. The tracrRNA extension sequence can have a length of more than 1000 nucleotides. The tracrRNA extension sequence can have a length of less than 1, 5, 10, 15, 20, 25, 30, 35, 40, 45, 50, 60, 70, 80, 90, 100, 120, 140, 160, 180, 200, 220, 240, 260, 280, 300, 320, 340, 360, 380, 400 or more nucleotides. The tracrRNA extension sequence can have a length of less than 1000 nucleotides. The tracrRNA extension sequence can comprise less than 10 nucleotides in length. The tracrRNA extension sequence can be 10-30 nucleotides in length. The tracrRNA extension sequence can be 30-70 nucleotides in length.

The tracrRNA extension sequence can comprise a functional moiety (e.g., a stability control sequence, ribozyme, endoribonuclease binding sequence). The functional moiety can comprise a transcriptional terminator segment (*i.e.,* a transcription termination sequence). The functional moiety can have a total length from about 10 nucleotides (nt) to about 100 nucleotides, from about 10 nt to about 20 nt, from about 20 nt to about 30 nt, from about 30 nt to about 40 nt, from about 40 nt to about 50 nt, from about 50 nt to about 60 nt, from about 60 nt to about 70 nt, from about 70 nt to about 80 nt, from about 80 nt to about 90 nt, or from about 90 nt to about 100 nt, from about 15 nt to about 80 nt, from about 15 nt to about 50 nt, from about 15 nt to about 40 nt, from about 15 nt to about 30 nt, or from about 15 nt to about 25 nt. The functional moiety can function in a eukaryotic cell. The functional moiety can function in a prokaryotic cell. The functional moiety can function in both eukaryotic and prokaryotic cells.

Non-limiting examples of suitable tracrRNA extension functional moieties include a 3' poly-adenylated tail, a riboswitch sequence (*e.g.,* to allow for regulated stability and/or regulated accessibility by proteins and protein complexes), a sequence that forms a dsRNA duplex (*i.e.,* a hairpin), a sequence that targets the RNA to a subcellular location (*e.g.,* nucleus, mitochondria, chloroplasts, and the like), a modification or sequence that provides for tracking (*e.g.,* direct conjugation to a fluorescent molecule, conjugation to a moiety that facilitates fluorescent detection, a sequence that allows for fluorescent detection, etc.), and/or a modification or sequence that provides a binding site for proteins (*e.g.,* proteins that act on DNA, including transcriptional activators, transcriptional controls, DNA methyltransferases, DNA demethylases, histone acetyltransferases, histone deacetylases, and the like). The tracrRNA extension sequence can comprise a primer binding site or a molecular index (*e.g.,* barcode sequence). The tracrRNA extension sequence can comprise one or more affinity tags.

### Single-Molecule Guide Linker Sequence

The linker sequence of a single-molecule guide nucleic acid can have a length from about 3 nucleotides to about 100 nucleotides. In Jinek *et al., supra,* for example, a simple 4 nucleotide "tetraloop" (-GAAA-) was used, Science, 337(6096):816-821 (2012). An illustrative linker has a length from about 3 nucleotides (nt) to about 90 nt, from about 3 nt to about 80 nt, from about 3 nt to about 70 nt, from about 3 nt to about 60 nt, from about 3 nt to about 50 nt, from about 3 nt to about 40 nt, from about 3 nt to about 30 nt, from about 3 nt to about 20 nt, from about 3 nt to about 10 nt. For example, the linker can have a length from about 3 nt to about 5 nt, from about 5 nt to about 10 nt, from about 10 nt to about 15 nt, from about 15 nt to about 20 nt, from about 20 nt to about 25 nt, from about 25 nt to about 30 nt, from about 30 nt to about 35 nt, from about 35 nt to about 40 nt, from about 40 nt to about 50 nt, from about 50 nt to about 60 nt, from about 60 nt to about 70 nt, from about 70 nt to about 80 nt, from about 80 nt to about 90 nt, or from about 90 nt to about 100 nt. The linker of a single-molecule guide nucleic acid can be between 4 and 40 nucleotides. The linker can be at least about 100, 500, 1000, 1500, 2000, 2500, 3000, 3500, 4000, 4500, 5000, 5500, 6000, 6500, or 7000 or more nucleotides. The linker can be at most about 100, 500, 1000, 1500, 2000, 2500, 3000, 3500, 4000, 4500, 5000, 5500, 6000, 6500, or 7000 or more nucleotides.

Linkers can comprise any of a variety of sequences, although in some examples the linker will not comprise sequences that have extensive regions of homology with other portions of the guide RNA, which might cause intramolecular binding that could interfere with other functional regions of the guide. In Jinek *et al., supra,* a simple 4 nucleotide sequence -GAAA- was used, Science, 337(6096):816-821 (2012), but numerous other sequences, including longer sequences can likewise be used.

The linker sequence can comprise a functional moiety. For example, the linker sequence can comprise one or more features, including an aptamer, a ribozyme, a protein-interacting hairpin, a protein binding site, a CRISPR array, an intron, or an exon. The linker sequence can comprise at least about 1, 2, 3, 4, or 5 or more functional moieties. In some examples, the linker sequence can comprise at most about 1, 2, 3, 4, or 5 or more functional moieties.

A step of the *ex vivo* methods of the present disclosure can comprise editing the patient specific iPSC cells using genome engineering. Alternatively, a step of the ex *vivo* methods of the present disclosure can comprise editing mesenchymal stem cell, or hematopoietic progenitor cell. Likewise, a step of the *in vivo* methods of the present disclosure can comprise editing the cells in a patient having hemoglobinopathy using genome engineering. Similarly, a step in the cellular methods of the present disclosure can comprise editing within or near a BCL11A gene or other DNA sequence that encodes a regulatory element of the BCL11A gene in a human cell by genome engineering.

Different patients with hemoglobinopathy will generally require different deletion, modulation, or inactivation strategies. Any CRISPR endonuclease may be used in the methods of the present disclosure, each CRISPR endonuclease having its own associated PAM, which may or may not be disease specific. For example, gRNA spacer sequences for targeting within or near a BCL11A gene or other DNA sequence that encodes a regulatory element of the BCL11A gene with a CRISPR/Cas9 endonuclease from *S. pyogenes* have been identified in SEQ ID NOs: 1- 29,482 of the Sequence Listing. gRNA spacer sequences for targeting within or near a BCL1 1A gene or other DNA sequence that encodes a regulatory element of the BCL11A gene with a CRISPR/Cas9 endonuclease from *S*. *aureus* have been identified in SEQ ID NOs: 29,483-32,387 of the Sequence Listing. gRNA spacer sequences for targeting within or near a BCL11A gene or other DNA sequence that encodes a regulatory element of the BCL1 1 A gene with a CRISPR/Cas9 endonuclease from *S*. *thermophilus* have been identified in SEQ ID NOs. 32,388-33,420 of the Sequence Listing. gRNA spacer sequences for targeting within or near a BCL1 1 A gene or other DNA sequence that encodes a regulatory element of the BCL11A gene with a CRISPR/Cas9 endonuclease from *T. denticola* have been identified in SEQ ID NOs. 33,421-33,851 of the Sequence Listing. gRNA spacer sequences for targeting within or near a BCL11A gene or other DNA sequence that encodes a regulatory element of the BCL11A gene with a CRISPR/Cas9 endonuclease from *N. meningitides* have been identified in SEQ ID NOs. 33,852-36,731. gRNA spacer sequences for targeting within or near a BCL11A gene or other DNA sequence that encodes a regulatory element of the BCL11A gene with a CRISPR/Cpf1 endonuclease from *Acidominococcus, Lachnospiraceae, and Franciscella Novicida* have been identified in SEQ ID NOs. 36,732-71,947.

For example, the transcriptional control sequence of the BCL11A gene can be modulated or inactivated by deletions that arise due to the NHEJ pathway. NHEJ can be used to delete segments of the transcriptional control sequence of the BCL11A gene, either directly or by altering splice donor or acceptor sites through cleavage by one gRNA targeting several locations, or several gRNAs.

The transcriptional control sequence of the BCL11A gene can also be modulated or inactivated by inserting a wild-type BCL11A gene or cDNA comprising a modified transcriptional control sequence. For example, the donor for modulating or activating by HDR contains the modified transcriptional control sequence of the BCL11A gene with small or large flanking homology arms to allow for annealing. HDR is essentially an error-free mechanism that uses a supplied homologous DNA sequence as a template during DSB repair. The rate of homology directed repair (HDR) is a function of the distance between the transcriptional control sequence and the cut site so choosing overlapping or nearest target sites is important. Templates can include extra sequences flanked by the homologous regions or can contain a sequence that differs from the genomic sequence, thus allowing sequence editing.

In addition to modulating or inactivating the transcriptional control sequence of the BCL11A gene by NHEJ or HDR, a range of other options are possible. If there are small or large deletions, a cDNA can be knocked in that contains a modified transcriptional control sequence. A full length cDNA can be knocked into any "safe harbor", but must use a supplied or other promoter. If this construct is knocked into the correct location, it will have physiological control, similar to the normal gene. Pairs of nucleases can be used to delete gene regions, though a donor would usually have to be provided to modulate or inactivate the function. In this case two gRNA would be supplied and one donor sequence.

Some genome engineering strategies involve modulating or inactivating a transcriptional control sequence of the BCL11A gene by deleting at least a portion of the transcriptional control sequence of the BCL11A gene and/or knocking-in a wild-type BCL11A gene or cDNA comprising a modified transcriptional control sequence into the locus of the corresponding gene or a safe harbour locus by homology directed repair (HDR), which is also known as homologous recombination (HR). This strategy can modulate or inactivate the transcriptional control sequence of the BCL11A gene and reverse, treat, and/or mitigate the diseased state. Donor nucleotides for modulating/inactivating transcriptional control sequences often are small (< 300 bp). This is advantageous, as HDR efficiencies may be inversely related to the size of the donor molecule. Also, it is expected that the donor templates can fit into size constrained adeno-associated virus (AAV) molecules, which have been shown to be an effective means of donor template delivery.

Homology direct repair is a cellular mechanism for repairing double-stranded breaks (DSBs). The most common form is homologous recombination. There are additional pathways for HDR, including single-strand annealing and alternative-HDR. Genome engineering tools allow researchers to manipulate the cellular homologous recombination pathways to create site-specific modifications to the genome. It has been found that cells can repair a double-stranded break using a synthetic donor molecule provided in trans. Therefore, by introducing a double-stranded break near a specific mutation and providing a suitable donor, targeted changes can be made in the genome. Specific cleavage increases the rate of HDR more than 1,000 fold above the rate of 1 in 10⁶ cells receiving a homologous donor alone. The rate of homology directed repair (HDR) at a particular nucleotide is a function of the distance to the cut site, so choosing overlapping or nearest target sites is important. Gene editing offers the advantage over gene addition, as correcting in situ leaves the rest of the genome unperturbed.

Supplied donors for editing by HDR vary markedly but can contain the intended sequence with small or large flanking homology arms to allow annealing to the genomic DNA. The homology regions flanking the introduced genetic changes can be 30 bp or smaller, or as large as a multi-kilobase cassette that can contain promoters, cDNAs, etc. Both single-stranded and double-stranded oligonucleotide donors have been used. These oligonucleotides range in size from less than 100 nt to over many kb, though longer ssDNA can also be generated and used. Double-stranded donors can be used, including PCR amplicons, plasmids, and mini-circles. In general, it has been found that an AAV vector can be a very effective means of delivery of a donor template, though the packaging limits for individual donors is <5kb. Active transcription of the donor increased HDR three-fold, indicating the inclusion of promoter may increase conversion. Conversely, CpG methylation of the donor decreased gene expression and HDR.

In addition to wildtype endonucleases, such as Cas9, nickase variants exist that have one or the other nuclease domain inactivated resulting in cutting of only one DNA strand. HDR can be directed from individual Cas nickases or using pairs of nickases that flank the target area. Donors can be single-stranded, nicked, or dsDNA.

The donor DNA can be supplied with the nuclease or independently by a variety of different methods, for example by transfection, nano-particle, micro-injection, or viral transduction. A range of tethering options have been proposed to increase the availability of the donors for HDR. Examples include attaching the donor to the nuclease, attaching to DNA binding proteins that bind nearby, or attaching to proteins that are involved in DNA end binding or repair.

The repair pathway choice can be guided by a number of culture conditions, such as those that influence cell cycling, or by targeting of DNA repair and associated proteins. For example, to increase HDR, key NHEJ molecules can be suppressed, such as KU70, KU80 or DNA ligase IV.

Without a donor present, the ends from a DNA break or ends from different breaks can be joined using the several nonhomologous repair pathways in which the DNA ends are joined with little or no base-pairing at the junction. In addition to canonical NHEJ, there are similar repair mechanisms, such as alt-NHEJ. If there are two breaks, the intervening segment can be deleted or inverted. NHEJ repair pathways can lead to insertions, deletions or mutations at the joints.

NHEJ was used to insert a 15-kb inducible gene expression cassette into a defined locus in human cell lines after nuclease cleavage. Maresca, M., Lin, V.G., Guo, N. & Yang, Y., Genome Res 23, 539-546 (2013).

In addition to genome editing by NHEJ or HDR, site-specific gene insertions have been conducted that use both the NHEJ pathway and HR. A combination approach may be applicable in certain settings, possibly including intron/exon borders. NHEJ may prove effective for ligation in the intron, while the error-free HDR may be better suited in the coding region.

As a further alternative, wild-type BCL11A gene or cDNA comprising a modified transcriptional control sequence can be knocked-in to the locus of the corresponding gene or knocked-in to a safe harbor site, such as AAVS1. In some examples, the methods can provide one gRNA or a pair of gRNAs that can be used to facilitate incorporation of a new sequence from a polynucleotide donor template to knock-in a part of or the entire wild-type BCL11A gene or cDNA comprising a modified transcriptional control sequence.

The methods can provide gRNA pairs that make a deletion by cutting the gene twice, one gRNA cutting at the 5' end of one or more mutations and the other gRNA cutting at the 3' end of one or more mutations that facilitates insertion of a new sequence from a polynucleotide donor template to replace the transcriptional control sequence of the BCL11A gene. The cutting can be accomplished by a pair of DNA endonucleases that each makes a DSB in the genome, or by multiple nickases that together make a DSB in the genome.

Alternatively, the methods can provide one gRNA to make one double-strand cut around a transcriptional control sequence of the BCL11A gene that facilitates insertion of a new sequence from a polynucleotide donor template to replace the transcriptional control sequence of the BCL11A gene with a wild-type BCL11A gene or cDNA comprising a modified transcriptional control sequence. The double-strand cut can be made by a single DNA endonuclease or multiple nickases that together make a DSB in the genome.

Illustrative modifications within or near the BCL11A gene or other DNA sequence that encodes a regulatory element of the BCL11A gene include replacements within or near (proximal) the transcriptional control sequence of the BCL11A gene referred to above, such as within the region of less than 3 kb, less than 2kb, less than 1 kb, less than 0.5 kb upstream or downstream of the transcriptional control sequence.

Such variants can include replacements that are larger in the 5' and/or 3' direction than the specific replacement in question, or smaller in either direction. Accordingly, by "near" or "proximal" with respect to specific replacements, it is intended that the SSB or DSB locus associated with a desired replacement boundary (also referred to herein as an endpoint) can be within a region that is less than about 3 kb from the reference locus noted. The SSB or DSB locus can be more proximal and within 2 kb, within 1 kb, within 0.5 kb, or within 0.1 kb. In the case of small replacement, the desired endpoint can be at or "adjacent to" the reference locus, by which it is intended that the endpoint can be within 100 bp, within 50 bp, within 25 bp, or less than about 10 bp to 5 bp from the reference locus.

Examples comprising larger or smaller replacements can be expected to provide the same benefit, as long as the transcriptional control activity is modulated or inactivated. It is thus expected that many variations of the replacements described and illustrated herein can be effective for ameliorating hemoglobinopathies.

Another genome engineering strategy involves exon or intron deletion. Targeted deletion of specific exons or introns can be an attractive strategy for treating a large subset of patients with a single therapeutic cocktail. Deletions can either be single exon or intron deletions or multi-exon or intron deletions. While multi-exon deletions can reach a larger number of patients, for larger deletions the efficiency of deletion greatly decreases with increased size. Therefore, deletions range can be from 40 to 10,000 base pairs (bp) in size. For example, deletions can range from 40-100; 100-300; 300-500; 500-1,000; 1,000-2,000; 2,000-3,000; 3,000-5,000; or 5,000-10,000 base pairs in size. It may be desirable to delete an intron if the intron contains a regulatory element, such as a transcriptional control element (e.g., a transcription factor binding site).

In order to ensure that the pre-mRNA is properly processed following deletion, the surrounding splicing signals can be deleted. Splicing donor and acceptors are generally within 100 base pairs of the neighboring intron. Therefore, in some examples, methods can provide all gRNAs that cut approximately +/- 100-3100 bp with respect to each exon/intron junction of interest.

For any of the genome editing strategies, gene editing can be confirmed by sequencing or PCR analysis.

### Target Sequence Selection

Shifts in the location of the 5' boundary and/or the 3' boundary relative to particular reference loci can be used to facilitate or enhance particular applications of gene editing, which depend in part on the endonuclease system selected for the editing, as further described and illustrated herein.

In a first nonlimiting example of such target sequence selection, many endonuclease systems have rules or criteria that can guide the initial selection of potential target sites for cleavage, such as the requirement of a PAM sequence motif in a particular position adjacent to the DNA cleavage sites in the case of CRISPR Type II or Type V endonucleases.

In another nonlimiting example of target sequence selection or optimization, the frequency of off-target activity for a particular combination of target sequence and gene editing endonuclease (*i.e.* the frequency of DSBs occurring at sites other than the selected target sequence) can be assessed relative to the frequency of on-target activity. In some cases, cells that have been correctly edited at the desired locus can have a selective advantage relative to other cells. Illustrative, but nonlimiting, examples of a selective advantage include the acquisition of attributes such as enhanced rates of replication, persistence, resistance to certain conditions, enhanced rates of successful engraftment or persistence *in vivo* following introduction into a patient, and other attributes associated with the maintenance or increased numbers or viability of such cells. In other cases, cells that have been correctly edited at the desired locus can be positively selected for by one or more screening methods used to identify, sort or otherwise select for cells that have been correctly edited. Both selective advantage and directed selection methods can take advantage of the phenotype associated with the correction. In some cases, cells can be edited two or more times in order to create a second modification that creates a new phenotype that is used to select or purify the intended population of cells. Such a second modification could be created by adding a second gRNA for a selectable or screenable marker. In some cases, cells can be correctly edited at the desired locus using a DNA fragment that contains the cDNA and also a selectable marker.

Whether any selective advantage is applicable or any directed selection is to be applied in a particular case, target sequence selection can also be guided by consideration of off-target frequencies in order to enhance the effectiveness of the application and/or reduce the potential for undesired alterations at sites other than the desired target. As described further and illustrated herein and in the art, the occurrence of off-target activity can be influenced by a number of factors including similarities and dissimilarities between the target site and various off-target sites, as well as the particular endonuclease used. Bioinformatics tools are available that assist in the prediction of off-target activity, and frequently such tools can also be used to identify the most likely sites of off-target activity, which can then be assessed in experimental settings to evaluate relative frequencies of off-target to on-target activity, thereby allowing the selection of sequences that have higher relative on-target activities. Illustrative examples of such techniques are provided herein, and others are known in the art.

Another aspect of target sequence selection relates to homologous recombination events. Sequences sharing regions of homology can serve as focal points for homologous recombination events that result in deletion of intervening sequences. Such recombination events occur during the normal course of replication of chromosomes and other DNA sequences, and also at other times when DNA sequences are being synthesized, such as in the case of repairs of double-strand breaks (DSBs), which occur on a regular basis during the normal cell replication cycle but can also be enhanced by the occurrence of various events (such as UV light and other inducers of DNA breakage) or the presence of certain agents (such as various chemical inducers). Many such inducers cause DSBs to occur indiscriminately in the genome, and DSBs can be regularly induced and repaired in normal cells. During repair, the original sequence can be reconstructed with complete fidelity, however, in some cases, small insertions or deletions (referred to as "indels") are introduced at the DSB site.

DSBs can also be specifically induced at particular locations, as in the case of the endonucleases systems described herein, which can be used to cause directed or preferential gene modification events at selected chromosomal locations. The tendency for homologous sequences to be subject to recombination in the context of DNA repair (as well as replication) can be taken advantage of in a number of circumstances, and is the basis for one application of gene editing systems, such as CRISPR, in which homology directed repair is used to insert a sequence of interest, provided through use of a "donor" polynucleotide, into a desired chromosomal location.

Regions of homology between particular sequences, which can be small regions of "microhomology" that can comprise as few as ten basepairs or less, can also be used to bring about desired deletions. For example, a single DSB can be introduced at a site that exhibits microhomology with a nearby sequence. During the normal course of repair of such DSB, a result that occurs with high frequency is the deletion of the intervening sequence as a result of recombination being facilitated by the DSB and concomitant cellular repair process.

In some circumstances, however, selecting target sequences within regions of homology can also give rise to much larger deletions, including gene fusions (when the deletions are in coding regions), which may or may not be desired given the particular circumstances.

The examples provided herein further illustrate the selection of various target regions for the creation of DSBs designed to induce replacements that result in the modulation or inactivation of transcriptional control protein activity, as well as the selection of specific target sequences within such regions that are designed to minimize off-target events relative to on-target events.

### Nucleic acid modifications

In some cases, polynucleotides introduced into cells can comprise one or more modifications that can be used individually or in combination, for example, to enhance activity, stability or specificity, alter delivery, reduce innate immune responses in host cells, or for other enhancements, as further described herein and known in the art.

In certain examples, modified polynucleotides can be used in the CRISPR/Cas9/Cpf1 system, in which case the guide RNAs (either single-molecule guides or double-molecule guides) and/or a DNA or an RNA encoding a Cas or Cpf1 endonuclease introduced into a cell can be modified, as described and illustrated below. Such modified polynucleotides can be used in the CRISPR/Cas9/Cpf1 system to edit any one or more genomic loci.

Using the CRISPR/Cas9/Cpf1 system for purposes of nonlimiting illustrations of such uses, modifications of guide RNAs can be used to enhance the formation or stability of the CRISPR/Cas9/Cpf1 genome editing complex comprising guide RNAs, which can be single-molecule guides or double-molecule, and a Cas or Cpf1 endonuclease. Modifications of guide RNAs can also or alternatively be used to enhance the initiation, stability or kinetics of interactions between the genome editing complex with the target sequence in the genome, which can be used, for example, to enhance on-target activity. Modifications of guide RNAs can also or alternatively be used to enhance specificity, *e.g.,* the relative rates of genome editing at the on-target site as compared to effects at other (off-target) sites.

Modifications can also or alternatively be used to increase the stability of a guide RNA, *e.g.,* by increasing its resistance to degradation by ribonucleases (RNases) present in a cell, thereby causing its half-life in the cell to be increased. Modifications enhancing guide RNA half-life can be particularly useful in aspects in which a Cas or Cpf1 endonuclease is introduced into the cell to be edited via an RNA that needs to be translated in order to generate endonuclease, because increasing the half-life of guide RNAs introduced at the same time as the RNA encoding the endonuclease can be used to increase the time that the guide RNAs and the encoded Cas or Cpf1 endonuclease co-exist in the cell.

Modifications can also or alternatively be used to decrease the likelihood or degree to which RNAs introduced into cells elicit innate immune responses. Such responses, which have been well characterized in the context of RNA interference (RNAi), including small-interfering RNAs (siRNAs), as described below and in the art, tend to be associated with reduced half-life of the RNA and/or the elicitation of cytokines or other factors associated with immune responses.

One or more types of modifications can also be made to RNAs encoding an endonuclease that are introduced into a cell, including, without limitation, modifications that enhance the stability of the RNA (such as by increasing its degradation by RNAses present in the cell), modifications that enhance translation of the resulting product (*i.e.* the endonuclease), and/or modifications that decrease the likelihood or degree to which the RNAs introduced into cells elicit innate immune responses.

Combinations of modifications, such as the foregoing and others, can likewise be used. In the case of CRISPR/Cas9/Cpf1, for example, one or more types of modifications can be made to guide RNAs (including those exemplified above), and/or one or more types of modifications can be made to RNAs encoding Cas endonuclease (including those exemplified above).

By way of illustration, guide RNAs used in the CRISPR/Cas9/Cpf1 system, or other smaller RNAs can be readily synthesized by chemical means, enabling a number of modifications to be readily incorporated, as illustrated below and described in the art. While chemical synthetic procedures are continually expanding, purifications of such RNAs by procedures such as high performance liquid chromatography (HPLC, which avoids the use of gels such as PAGE) tends to become more challenging as polynucleotide lengths increase significantly beyond a hundred or so nucleotides. One approach that can be used for generating chemically-modifed RNAs of greater length is to produce two or more molecules that are ligated together. Much longer RNAs, such as those encoding a Cas9 endonuclease, are more readily generated enzymatically. While fewer types of modifications are available for use in enzymatically produced RNAs, there are still modifications that can be used to, *e.g.,* enhance stability, reduce the likelihood or degree of innate immune response, and/or enhance other attributes, as described further below and in the art; and new types of modifications are regularly being developed.

By way of illustration of various types of modifications, especially those used frequently with smaller chemically synthesized RNAs, modifications can comprise one or more nucleotides modified at the 2' position of the sugar, in some aspects a 2'-O-alkyl, 2'-O-alkyl-O-alkyl, or 2'-fluoro-modified nucleotide. In some examples, RNA modifications can comprise 2'-fluoro, 2'-amino or 2' O-methyl modifications on the ribose of pyrimidines, abasic residues, or an inverted base at the 3' end of the RNA. Such modifications can be routinely incorporated into oligonucleotides and these oligonucleotides have been shown to have a higher Tm (*i.e.,* higher target binding affinity) than 2'-deoxyoligonucleotides against a given target.

A number of nucleotide and nucleoside modifications have been shown to make the oligonucleotide into which they are incorporated more resistant to nuclease digestion than the native oligonucleotide; these modified oligos survive intact for a longer time than unmodified oligonucleotides. Specific examples of modified oligonucleotides include those comprising modified backbones, for example, phosphorothioates, phosphotriesters, methyl phosphonates, short chain alkyl or cycloalkyl intersugar linkages or short chain heteroatomic or heterocyclic intersugar linkages. Some oligonucleotides are oligonucleotides with phosphorothioate backbones and those with heteroatom backbones, particularly CH₂ -NH-O-CH₂, CH,~N(CH₃)~O~CH₂ (known as a methylene(methylimino) or MMI backbone), CH₂ --ON (CH₃)-CH₂, CH₂ -N (CH₃)-N (CH₃)-CH₂ and O-N (CH₃)- CH₂ -CH₂ backbones, wherein the native phosphodiester backbone is represented as O- P-- O- CH,); amide backbones [see De Mesmaeker et al., Ace. Chem. Res., 28:366-374 (1995)]; morpholino backbone structures (see Summerton and Weller, U.S. Pat. No. 5,034,506); peptide nucleic acid (PNA) backbone (wherein the phosphodiester backbone of the oligonucleotide is replaced with a polyamide backbone, the nucleotides being bound directly or indirectly to the aza nitrogen atoms of the polyamide backbone, see Nielsen et al., Science 1991, 254, 1497). Phosphorus-containing linkages include, but are not limited to, phosphorothioates, chiral phosphorothioates, phosphorodithioates, phosphotriesters, aminoalkylphosphotriesters, methyl and other alkyl phosphonates comprising 3'alkylene phosphonates and chiral phosphonates, phosphinates, phosphoramidates comprising 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'; see US Patent Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050.

Morpholino-based oligomeric compounds are described in Braasch and David Corey, Biochemistry, 41(14): 4503-4510 (2002); Genesis, Volume 30, Issue 3, (2001); Heasman, Dev. Biol., 243: 209-214 (2002); Nasevicius et al., Nat. Genet., 26:216-220 (2000); Lacerra et al., Proc. Natl. Acad. Sci., 97: 9591-9596 (2000); and U.S. Pat. No. 5,034,506, issued Jul. 23, 1991.

Cyclohexenyl nucleic acid oligonucleotide mimetics are described in Wang et al., J. Am. Chem. Soc., 122: 8595-8602 (2000).

Modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These comprise those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S, and CH₂ component parts; see US Patent Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439.

One or more substituted sugar moieties can also be included, e.g., one of the following at the 2' position: OH, SH, SCH₃, F, OCN, OCH₃ OCH₃, OCH₃ O(CH₂)n CH₃, O(CH₂)n NH₂, or O(CH₂)n CH₃, where n is from 1 to about 10; C1 to C10 lower alkyl, alkoxyalkoxy, substituted lower alkyl, alkaryl or aralkyl; Cl; Br; CN; CF₃; OCF₃; O-, S-, or N-alkyl; O-, S-, or N-alkenyl; SOCH₃; SO₂ CH₃; ONO₂; NO₂; N₃; NH₂; heterocycloalkyl; heterocycloalkaryl; aminoalkylamino; polyalkylamino; substituted silyl; an RNA cleaving group; a reporter group; an intercalator; a group for improving the pharmacokinetic properties of an oligonucleotide; or a group for improving the pharmacodynamic properties of an oligonucleotide and other substituents having similar properties. In some aspects, a modification includes 2'-methoxyethoxy (2'-O-CH₂CH₂OCH₃, also known as 2'-O-(2-methoxyethyl)) (Martin et al, Helv. Chim. Acta, 1995, 78, 486). Other modifications include 2'-methoxy (2'-O-CH₃), 2'-propoxy (2'-OCH₂ CH₂CH₃) and 2'-fluoro (2'-F). Similar modifications can also be made at other positions on the oligonucleotide, particularly the 3' position of the sugar on the 3' terminal nucleotide and the 5' position of 5' terminal nucleotide. Oligonucleotides can also have sugar mimetics, such as cyclobutyls in place of the pentofuranosyl group.

In some examples, both a sugar and an internucleoside linkage, i.e., the backbone, of the nucleotide units can be replaced with novel groups. The base units can be maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide can be replaced with an amide containing backbone, for example, an aminoethylglycine backbone. The nucleobases can be retained and bound directly or indirectly to aza nitrogen atoms of the amide portion of the backbone. Representative United States patents that teach the preparation of PNA compounds comprise, but are not limited to, US Patent Nos. 5,539,082; 5,714,331; and 5,719,262. Further teaching of PNA compounds can be found in Nielsen et al, Science, 254: 1497-1500 (1991).

Guide RNAs can also include, additionally or alternatively, nucleobase (often referred to in the art simply as "base") modifications or substitutions. As used herein, "unmodified" or "natural" nucleobases include adenine (A), guanine (G), thymine (T), cytosine (C), and uracil (U). Modified nucleobases include nucleobases found only infrequently or transiently in natural nucleic acids, *e.g.,* hypoxanthine, 6-methyladenine, 5-Me pyrimidines, particularly 5-methylcytosine (also referred to as 5-methyl-2' deoxycytosine and often referred to in the art as 5-Me-C), 5-hydroxymethylcytosine (HMC), glycosyl HMC and gentobiosyl HMC, as well as synthetic nucleobases, e.g., 2-aminoadenine, 2-(methylamino)adenine, 2-(imidazolylalkyl)adenine, 2-(aminoalklyamino)adenine or other heterosubstituted alkyladenines, 2-thiouracil, 2-thiothymine, 5-bromouracil, 5-hydroxymethyluracil, 8-azaguanine, 7-deazaguanine, N6 (6-aminohexyl)adenine, and 2,6-diaminopurine. Kornberg, A., DNA Replication, W. H. Freeman & Co., San Francisco, pp75-77 (1980); Gebeyehu et al., Nucl. Acids Res. 15:4513 (1997). A "universal" base known in the art, *e.g.,* inosine, can also be included. 5-Me-C substitutions have been shown to increase nucleic acid duplex stability by 0.6-1.2 °C. (Sanghvi, Y. S., in Crooke, S. T. and Lebleu, B., eds., Antisense Research and Applications, CRC Press, Boca Raton, 1993, pp. 276-278) and are aspects of base substitutions.

Modified nucleobases can comprise other synthetic and natural nucleobases, such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudo-uracil), 4-thiouracil, 8-halo, 8-amino, 8-thiol, 8-thioalkyl, 8-hydroxyl and other 8-substituted adenines and guanines, 5-halo particularly 5- bromo, 5-trifluoromethyl and other 5-substituted uracils and cytosines, 7-methylquanine and 7-methyladenine, 8-azaguanine and 8-azaadenine, 7-deazaguanine and 7-deazaadenine, and 3-deazaguanine and 3-deazaadenine.

Further, nucleobases can comprise those disclosed in United States Patent No. 3,687,808, those disclosed in 'The Concise Encyclopedia of Polymer Science And Engineering', pages 858-859, Kroschwitz, J.I., ed. John Wiley & Sons, 1990, those disclosed by Englisch et al., Angewandle Chemie, International Edition', 1991, 30, page 613, and those disclosed by Sanghvi, Y. S., Chapter 15, Antisense Research and Applications', pages 289- 302, Crooke, S.T. and Lebleu, B. ea., CRC Press, 1993. Certain of these nucleobases are particularly useful for increasing the binding affinity of the oligomeric compounds of the invention. These include 5-substituted pyrimidines, 6-azapyrimidines and N-2, N-6 and 0-6 substituted purines, comprising 2-aminopropyladenine, 5-propynyluracil and 5-propynylcytosine. 5-methylcytosine substitutions have been shown to increase nucleic acid duplex stability by 0.6-1.2°C (Sanghvi, Y.S., Crooke, S.T. and Lebleu, B., eds, 'Antisense Research and Applications', CRC Press, Boca Raton, 1993, pp. 276-278) and are aspects of base substitutions, even more particularly when combined with 2'-O-methoxyethyl sugar modifications. Modified nucleobases are described in US Patent Nos. 3,687,808, as well as 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,596,091; 5,614,617; 5,681,941; 5,750,692; 5,763,588; 5,830,653; 6,005,096; and US Patent Application Publication 2003/0158403.

Thus, the term "modified" refers to a non-natural sugar, phosphate, or base that is incorporated into a guide RNA, an endonuclease, or transcriptional control sequence of BCL11A or both a guide RNA and an endonuclease. It is not necessary for all positions in a given oligonucleotide to be uniformly modified, and in fact more than one of the aforementioned modifications can be incorporated in a single oligonucleotide, or even in a single nucleoside within an oligonucleotide.

The guide RNAs and/or mRNA (or DNA) encoding an endonuclease can be chemically linked to one or more moieties or conjugates that enhance the activity, cellular distribution, or cellular uptake of the oligonucleotide. Such moieties comprise, but are not limited to, lipid moieties such as a cholesterol moiety [Letsinger et al., Proc. Natl. Acad. Sci. USA, 86: 6553-6556 (1989)]; cholic acid [Manoharan et al., Bioorg. Med. Chem. Let., 4: 1053-1060 (1994)]; a thioether, *e.g.,* hexyl-S- tritylthiol [Manoharan et al, Ann. N. Y. Acad. Sci., 660: 306-309 (1992) and Manoharan et al., Bioorg. Med. Chem. Let., 3: 2765-2770 (1993)]; a thiocholesterol [Oberhauser et al., Nucl. Acids Res., 20: 533-538 (1992)]; an aliphatic chain, *e.g.,* dodecandiol or undecyl residues [Kabanov et al., FEBS Lett., 259: 327-330 (1990) and Svinarchuk et al., Biochimie, 75: 49- 54 (1993)]; a phospholipid, *e.g.,* di-hexadecyl-rac-glycerol or triethylammonium 1 ,2-di-O-hexadecyl- rac-glycero-3-H-phosphonate [Manoharan et al., Tetrahedron Lett., 36: 3651-3654 (1995) and Shea et al., Nucl. Acids Res., 18: 3777-3783 (1990)]; a polyamine or a polyethylene glycol chain [Mancharan et al., Nucleosides & Nucleotides, 14: 969-973 (1995)]; adamantane acetic acid [Manoharan et al., Tetrahedron Lett., 36: 3651-3654 (1995)]; a palmityl moiety [(Mishra et al., Biochim. Biophys. Acta, 1264: 229-237 (1995)]; or an octadecylamine or hexylamino-carbonyl-t oxycholesterol moiety [Crooke et al., J. Pharmacol. Exp. Ther., 277: 923-937 (1996)]. See also US Patent Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599, 928 and 5,688,941.

Sugars and other moieties can be used to target proteins and complexes comprising nucleotides, such as cationic polysomes and liposomes, to particular sites. For example, hepatic cell directed transfer can be mediated via asialoglycoprotein receptors (ASGPRs); see, *e.g.,* Hu, et al., Protein Pept Lett. 21(10):1025-30 (2014). Other systems known in the art and regularly developed can be used to target biomolecules of use in the present case and/or complexes thereof to particular target cells of interest.

These targeting moieties or conjugates can include conjugate groups covalently bound to functional groups, such as primary or secondary hydroxyl groups. Conjugate groups of the disclosure include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugate groups include cholesterols, lipids, phospholipids, biotin, phenazine, folate, phenanthridine, anthraquinone, acridine, fluoresceins, rhodamines, coumarins, and dyes. Groups that enhance the pharmacodynamic properties, in the context of this disclosure, include groups that improve uptake, enhance resistance to degradation, and/or strengthen sequence-specific hybridization with the target nucleic acid. Groups that enhance the pharmacokinetic properties, in the context of this disclosure, include groups that improve uptake, distribution, metabolism or excretion of the compounds of the present disclosure. Representative conjugate groups are disclosed in U.S. Pat. No. 6,287,860. Conjugate moieties include, but are not limited to, lipid moieties such as a cholesterol moiety, cholic acid, a thioether, *e.g.,* hexyl-5-tritylthiol, a thiocholesterol, an aliphatic chain, *e.g.,* dodecandiol or undecyl residues, a phospholipid, *e.g.,* di-hexadecyl-rac-glycerol or triethylammonium l,2-di-O-hexadecyl-rac-glycero-3-H-phosphonate, a polyamine or a polyethylene glycol chain, or adamantane acetic acid, a palmityl moiety, or an octadecylamine or hexylamino-carbonyl-oxy cholesterol moiety. See, *e.g.,* U.S. Pat. Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

Longer polynucleotides that are less amenable to chemical synthesis and are typically produced by enzymatic synthesis can also be modified by various means. Such modifications can include, for example, the introduction of certain nucleotide analogs, the incorporation of particular sequences or other moieties at the 5' or 3' ends of molecules, and other modifications. By way of illustration, the mRNA encoding Cas9 is approximately 4 kb in length and can be synthesized by *in vitro* transcription. Modifications to the mRNA can be applied to, *e.g.,* increase its translation or stability (such as by increasing its resistance to degradation with a cell), or to reduce the tendency of the RNA to elicit an innate immune response that is often observed in cells following introduction of exogenous RNAs, particularly longer RNAs such as that encoding Cas9.

Numerous such modifications have been described in the art, such as polyA tails, 5' cap analogs (*e.g.,* Anti Reverse Cap Analog (ARCA) or m7G(5')ppp(5')G (mCAP)), modified 5' or 3' untranslated regions (UTRs), use of modified bases (such as Pseudo-UTP, 2-Thio-UTP, 5-Methylcytidine-5'-Triphosphate (5-Methyl-CTP) or N6-Methyl-ATP), or treatment with phosphatase to remove 5' terminal phosphates. These and other modifications are known in the art, and new modifications of RNAs are regularly being developed.

There are numerous commercial suppliers of modified RNAs, including for example, TriLink Biotech, AxoLabs, Bio-Synthesis Inc., Dharmacon and many others. As described by TriLink, for example, 5-Methyl-CTP can be used to impart desirable characteristics, such as increased nuclease stability, increased translation or reduced interaction of innate immune receptors with *in vitro* transcribed RNA. 5-Methylcytidine-5'-Triphosphate (5-Methyl-CTP), N6-Methyl-ATP, as well as Pseudo-UTP and 2-Thio-UTP, have also been shown to reduce innate immune stimulation in culture and *in vivo* while enhancing translation, as illustrated in publications by Kormann *et al.* and Warren *et al.* referred to below.

It has been shown that chemically modified mRNA delivered *in vivo* can be used to achieve improved therapeutic effects; see, e.g., Kormann et al., Nature Biotechnology 29, 154-157 (2011). Such modifications can be used, for example, to increase the stability of the RNA molecule and/or reduce its immunogenicity. Using chemical modifications such as Pseudo-U, N6-Methyl-A, 2-Thio-U and 5-Methyl-C, it was found that substituting just one quarter of the uridine and cytidine residues with 2-Thio-U and 5-Methyl-C respectively resulted in a significant decrease in toll-like receptor (TLR) mediated recognition of the mRNA in mice. By reducing the activation of the innate immune system, these modifications can be used to effectively increase the stability and longevity of the mRNA *in vivo;* see, *e.g.,* Kormann *et al., supra.*

It has also been shown that repeated administration of synthetic messenger RNAs incorporating modifications designed to bypass innate anti-viral responses can reprogram differentiated human cells to pluripotency. See, *e.g.,* Warren, et al., Cell Stem Cell, 7(5):618-30 (2010). Such modified mRNAs that act as primary reprogramming proteins can be an efficient means of reprogramming multiple human cell types. Such cells are referred to as induced pluripotency stem cells (iPSCs), and it was found that enzymatically synthesized RNA incorporating 5-Methyl-CTP, Pseudo-UTP and an Anti Reverse Cap Analog (ARCA) could be used to effectively evade the cell's antiviral response; see, *e.g.,* Warren *et al., supra.*

Other modifications of polynucleotides described in the art include, for example, the use of polyA tails, the addition of 5' cap analogs (such as m7G(5')ppp(5')G (mCAP)), modifications of 5' or 3' untranslated regions (UTRs), or treatment with phosphatase to remove 5' terminal phosphates - and new approaches are regularly being developed.

A number of compositions and techniques applicable to the generation of modified RNAs for use herein have been developed in connection with the modification of RNA interference (RNAi), including small-interfering RNAs (siRNAs). siRNAs present particular challenges *in vivo* because their effects on gene silencing via mRNA interference are generally transient, which can require repeat administration. In addition, siRNAs are double-stranded RNAs (dsRNA) and mammalian cells have immune responses that have evolved to detect and neutralize dsRNA, which is often a by-product of viral infection. Thus, there are mammalian enzymes such as PKR (dsRNA-responsive kinase), and potentially retinoic acid-inducible gene I (RIG-I), that can mediate cellular responses to dsRNA, as well as Toll-like receptors (such as TLR3, TLR7 and TLR8) that can trigger the induction of cytokines in response to such molecules; see, *e.g.,* the reviews by Angart et al., Pharmaceuticals (Basel) 6(4): 440-468 (2013); Kanasty et al., Molecular Therapy 20(3): 513-524 (2012); Burnett et al., Biotechnol J. 6(9):1130-46 (2011); Judge and MacLachlan, Hum Gene Ther 19(2):111-24 (2008); and references cited therein.

A large variety of modifications have been developed and applied to enhance RNA stability, reduce innate immune responses, and/or achieve other benefits that can be useful in connection with the introduction of polynucleotides into human cells, as described herein; see, *e.g.,* the reviews by Whitehead KA et al., Annual Review of Chemical and Biomolecular Engineering, 2: 77-96 (2011); Gaglione and Messere, Mini Rev Med Chem, 10(7):578-95 (2010); Chernolovskaya et al, Curr Opin Mol Ther., 12(2):158-67 (2010); Deleavey et al., Curr Protoc Nucleic Acid Chem Chapter 16:Unit 16.3 (2009); Behlke, Oligonucleotides 18(4):305-19 (2008); Fucini et al., Nucleic Acid Ther 22(3): 205-210 (2012); Bremsen et al., Front Genet 3:154 (2012).

As noted above, there are a number of commercial suppliers of modified RNAs, many of which have specialized in modifications designed to improve the effectiveness of siRNAs. A variety of approaches are offered based on various findings reported in the literature. For example, Dharmacon notes that replacement of a non-bridging oxygen with sulfur (phosphorothioate, PS) has been extensively used to improve nuclease resistance of siRNAs, as reported by Kole, Nature Reviews Drug Discovery 11:125-140 (2012). Modifications of the 2'-position of the ribose have been reported to improve nuclease resistance of the internucleotide phosphate bond while increasing duplex stability (Tm), which has also been shown to provide protection from immune activation. A combination of moderate PS backbone modifications with small, well-tolerated 2'-substitutions (2'-O-Methyl, 2'-Fluoro, 2'-Hydro) have been associated with highly stable siRNAs for applications *in vivo,* as reported by Soutschek et al. Nature 432:173-178 (2004); and 2'-O-Methyl modifications have been reported to be effective in improving stability as reported by Volkov, Oligonucleotides 19:191-202 (2009). With respect to decreasing the induction of innate immune responses, modifying specific sequences with 2'-O-Methyl, 2'-Fluoro, 2'-Hydro have been reported to reduce TLR7/TLR8 interaction while generally preserving silencing activity; see, e.g., Judge et al., Mol. Ther. 13:494-505 (2006); and Cekaite et al., J. Mol. Biol. 365:90-108 (2007). Additional modifications, such as 2-thiouracil, pseudouracil, 5-methylcytosine, 5-methyluracil, and N6-methyladenosine have also been shown to minimize the immune effects mediated by TLR3, TLR7, and TLR8; see, *e.g.,* Kariko, K. et al., Immunity 23:165-175 (2005).

As is also known in the art, and commercially available, a number of conjugates can be applied to polynucleotides, such as RNAs, for use herein that can enhance their delivery and/or uptake by cells, including for example, cholesterol, tocopherol and folic acid, lipids, peptides, polymers, linkers and aptamers; see, *e.g.,* the review by Winkler, Ther. Deliv. 4:791-809 (2013), and references cited therein.

### Codon-Optimization

A polynucleotide encoding a site-directed polypeptide can be codon-optimized according to methods standard in the art for expression in the cell containing the target DNA of interest. For example, if the intended target nucleic acid is in a human cell, a human codon-optimized polynucleotide encoding Cas9 is contemplated for use for producing the Cas9 polypeptide.

### Complexes of a Genome-targeting Nucleic Acid and a Site-Directed Polypeptide

A genome-targeting nucleic acid interacts with a site-directed polypeptide (e.g., a nucleic acid-guided nuclease such as Cas9), thereby forming a complex. The genome-targeting nucleic acid guides the site-directed polypeptide to a target nucleic acid.

### RNPs

The site-directed polypeptide and genome-targeting nucleic acid can each be administered separately to a cell or a patient. On the other hand, the site-directed polypeptide can be pre-complexed with one or more genome-targeting nucleic acids (guide RNA, sgRNA, or crRNA together with a tracrRNA). The pre-complexed material can then be administered to a cell or a patient. Such pre-complexed material is known as a ribonucleoprotein particle (RNP). The site-directed polypeptide in the RNP can be, for example, a Cas9 endonuclease or a Cpf1 endonuclease. The site-directed polypeptide can be flanked at the N-terminus, the C-terminus, or both the N-terminus and C-terminus by one or more nuclear localization signals (NLSs). For example, a Cas9 endonuclease can be flanked by two NLSs, one NLS located at the N-terminus and the second NLS located at the C-terminus. The NLS can be any NLS known in the art, such as a SV40 NLS. The weight ratio of genome-targeting nucleic acid to site-directed polypeptide in the RNP can be 1:1. For example, the weight ratio of sgRNA to Cas9 endonuclease in the RNP can be 1:1. For example, the sgRNA can comprise the nucleic acid sequence of SEQ ID NO: 71,959, the Cas9 endonuclease can be a *S. pyogenes* Cas9 comprising a N-terminus SV40 NLS and a C-terminus SV40 NLS, and the weight ratio of sgRNA to Cas9 endonuclease can be 1:1.

### Nucleic Acids Encoding System Components

The present disclosure discloses a nucleic acid comprising a nucleotide sequence encoding a genome-targeting nucleic acid of the disclosure, a site-directed polypeptide of the disclosure, and/or any nucleic acid or proteinaceous molecule necessary to carry out the aspects of the methods of the disclosure.

The nucleic acid encoding a genome-targeting nucleic acid of the disclosure, a site-directed polypeptide of the disclosure, and/or any nucleic acid or proteinaceous molecule necessary to carry out the aspects of the methods of the disclosure can comprise a vector (*e.g.,* a recombinant expression vector).

The term "vector" refers to a nucleic acid molecule capable of transporting another nucleic acid to which it has been linked. One type of vector is a "plasmid", which refers to a circular double-stranded DNA loop into which additional nucleic acid segments can be ligated. Another type of vector is a viral vector, wherein additional nucleic acid segments can be ligated into the viral genome. Certain vectors are capable of autonomous replication in a host cell into which they are introduced (*e.g.,* bacterial vectors having a bacterial origin of replication and episomal mammalian vectors). Other vectors (*e.g.,* non-episomal mammalian vectors) are integrated into the genome of a host cell upon introduction into the host cell, and thereby are replicated along with the host genome.

In some examples, vectors can be capable of directing the expression of nucleic acids to which they are operatively linked. Such vectors are referred to herein as "recombinant expression vectors", or more simply "expression vectors", which serve equivalent functions.

The term "operably linked" means that the nucleotide sequence of interest is linked to regulatory sequence(s) in a manner that allows for expression of the nucleotide sequence. The term "regulatory sequence" is intended to include, for example, promoters, enhancers and other expression control elements (*e.g.,* polyadenylation signals). Such regulatory sequences are well known in the art and are described, for example, in Goeddel; Gene Expression Technology: Methods in Enzymology 185, Academic Press, San Diego, CA (1990). Regulatory sequences include those that direct constitutive expression of a nucleotide sequence in many types of host cells, and those that direct expression of the nucleotide sequence only in certain host cells (*e.g.,* tissue-specific regulatory sequences). It will be appreciated by those skilled in the art that the design of the expression vector can depend on such factors as the choice of the target cell, the level of expression desired, and the like.

Expression vectors contemplated include, but are not limited to, viral vectors based on vaccinia virus, poliovirus, adenovirus, adeno-associated virus, SV40, herpes simplex virus, human immunodeficiency virus, retrovirus (*e.g.,* Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, and mammary tumor virus) and other recombinant vectors. Other vectors contemplated for eukaryotic target cells include, but are not limited to, the vectors pXT1, pSG5, pSVK3, pBPV, pMSG, and pSVLSV40 (Pharmacia). Additional vectors contemplated for eukaryotic target cells include, but are not limited to, the vectors pCTx-1, pCTx-2, and pCTx-3, which are described in Figures 1A to 1C. Other vectors can be used so long as they are compatible with the host cell.

In some examples, a vector can comprise one or more transcription and/or translation control elements. Depending on the host/vector system utilized, any of a number of suitable transcription and translation control elements, including constitutive and inducible promoters, transcription enhancer elements, transcription terminators, etc. can be used in the expression vector. The vector can be a self-inactivating vector that either inactivates the viral sequences or the components of the CRISPR machinery or other elements.

Non-limiting examples of suitable eukaryotic promoters (*i.e.,* promoters functional in a eukaryotic cell) include those from cytomegalovirus (CMV) immediate early, herpes simplex virus (HSV) thymidine kinase, early and late SV40, long terminal repeats (LTRs) from retrovirus, human elongation factor-1 promoter (EF1), a hybrid construct comprising the cytomegalovirus (CMV) enhancer fused to the chicken beta-actin promoter (CAG), murine stem cell virus promoter (MSCV), phosphoglycerate kinase-1 locus promoter (PGK), and mouse metallothionein-I.

For expressing small RNAs, including guide RNAs used in connection with Cas endonuclease, various promoters such as RNA polymerase III promoters, including for example U6 and H1, can be advantageous. Descriptions of and parameters for enhancing the use of such promoters are known in art, and additional information and approaches are regularly being described; see, e.g., Ma, H. et al., Molecular Therapy - Nucleic Acids 3, e161 (2014) doi:10.1038/mtna.2014.12.

The expression vector can also contain a ribosome binding site for translation initiation and a transcription terminator. The expression vector can also comprise appropriate sequences for amplifying expression. The expression vector can also include nucleotide sequences encoding non-native tags (*e.g.,* histidine tag, hemagglutinin tag, green fluorescent protein, etc.) that are fused to the site-directed polypeptide, thus resulting in a fusion protein.

A promoter can be an inducible promoter (*e.g.,* a heat shock promoter, tetracycline-regulated promoter, steroid-regulated promoter, metal-regulated promoter, estrogen receptor-regulated promoter, etc.). The promoter can be a constitutive promoter (*e.g.,* CMV promoter, UBC promoter). In some cases, the promoter can be a spatially restricted and/or temporally restricted promoter (*e.g.,* a tissue specific promoter, a cell type specific promoter, etc.).

The nucleic acid encoding a genome-targeting nucleic acid of the disclosure and/or a site-directed polypeptide can be packaged into or on the surface of delivery vehicles for delivery to cells. Delivery vehicles contemplated include, but are not limited to, nanospheres, liposomes, quantum dots, nanoparticles, polyethylene glycol particles, hydrogels, and micelles. As described in the art, a variety of targeting moieties can be used to enhance the preferential interaction of such vehicles with desired cell types or locations.

Introduction of the complexes, polypeptides, and nucleic acids of the disclosure into cells can occur by viral or bacteriophage infection, transfection, conjugation, protoplast fusion, lipofection, electroporation, nucleofection, calcium phosphate precipitation, polyethyleneimine (PEI)-mediated transfection, DEAE-dextran mediated transfection, liposome-mediated transfection, particle gun technology, calcium phosphate precipitation, direct micro-injection, nanoparticle-mediated nucleic acid delivery, and the like.

### Delivery

Guide RNA polynucleotides (RNA or DNA) and/or endonuclease polynucleotide(s) (RNA or DNA) can be delivered by viral or non-viral delivery vehicles known in the art, such as electroporation, mechanical force, cell deformation (SQZ Biotech), and cell penetrating peptides. Alternatively, endonuclease polypeptide(s) can be delivered by viral or non-viral delivery vehicles known in the art, such as electroporation or lipid nanoparticles. In further alternative aspects, the DNA endonuclease can be delivered as one or more polypeptides, either alone or pre-complexed with one or more guide RNAs, or one or more crRNA together with a tracrRNA.

Electroporation is a delivery technique in which an electrical field is applied to one or more cells in order to increase the permeability of the cell membrane, which allows substances such as drugs, nucleic acids (genome-targeting nucleic acids), proteins (site-directed polypeptides), or RNPs, to be introduced into the cell. In general, electroporation works by passing thousands of volts across a distance of one to two millimeters of suspended cells in an electroporation cuvette (1.0 - 1.5 kV, 250 - 750V/cm).

Polynucleotides can be delivered by non-viral delivery vehicles including, but not limited to, nanoparticles, liposomes, ribonucleoproteins, positively charged peptides, small molecule RNA-conjugates, aptamer-RNA chimeras, and RNA-fusion protein complexes. Some exemplary non-viral delivery vehicles are described in Peer and Lieberman, Gene Therapy, 18: 1127-1133 (2011) (which focuses on non-viral delivery vehicles for siRNA that are also useful for delivery of other polynucleotides).

Polynucleotides, such as guide RNA, sgRNA, and mRNA encoding an endonuclease, can be delivered to a cell or a patient by a lipid nanoparticle (LNP).

A LNP refers to any particle having a diameter of less than 1000 nm, 500 nm, 250 nm, 200 nm, 150 nm, 100 nm, 75 nm, 50 nm, or 25 nm. Alternatively, a nanoparticle can range in size from 1-1000 nm, 1-500 nm, 1-250 nm, 25-200 nm, 25-100 nm, 35-75 nm, or 25-60 nm.

LNPs can be made from cationic, anionic, or neutral lipids. Neutral lipids, such as the fusogenic phospholipid DOPE or the membrane component cholesterol, can be included in LNPs as 'helper lipids' to enhance transfection activity and nanoparticle stability. Limitations of cationic lipids include low efficacy owing to poor stability and rapid clearance, as well as the generation of inflammatory or antiinflammatory responses.

LNPs can also be comprised of hydrophobic lipids, hydrophilic lipids, or both hydrophobic and hydrophilic lipids.

Any lipid or combination of lipids that are known in the art can be used to produce a LNP. Examples of lipids used to produce LNPs are: DOTMA, DOSPA, DOTAP, DMRIE, DC-cholesterol, DOTAP-cholesterol, GAP-DMORIE-DPyPE, and GL67A-DOPE-DMPE-polyethylene glycol (PEG). Examples of cationic lipids are: 98N12-5, C12-200, DLin-KC2-DMA (KC2), DLin-MC3-DMA (MC3), XTC, MD1, and 7C1. Examples of neutral lipids are: DPSC, DPPC, POPC, DOPE, and SM. Examples of PEG-modified lipids are: PEG-DMG, PEG-CerC14, and PEG-CerC20.

The lipids can be combined in any number of molar ratios to produce a LNP. In addition, the polynucleotide(s) can be combined with lipid(s) in a wide range of molar ratios to produce a LNP.

As stated previously, the site-directed polypeptide and genome-targeting nucleic acid can each be administered separately to a cell or a patient. On the other hand, the site-directed polypeptide can be pre-complexed with one or more guide RNAs, or one or more crRNA together with a tracrRNA. The pre-complexed material can then be administered to a cell or a patient. Such pre-complexed material is known as a ribonucleoprotein particle (RNP).

RNA is capable of forming specific interactions with RNA or DNA. While this property is exploited in many biological processes, it also comes with the risk of promiscuous interactions in a nucleic acid-rich cellular environment. One solution to this problem is the formation of ribonucleoprotein particles (RNPs), in which the RNA is pre-complexed with an endonuclease. Another benefit of the RNP is protection of the RNA from degradation.

The endonuclease in the RNP can be modified or unmodified. Likewise, the gRNA, crRNA, tracrRNA, or sgRNA can be modified or unmodified. Numerous modifications are known in the art and can be used.

The endonuclease and sgRNA can be generally combined in a 1:1 molar ratio. Alternatively, the endonuclease, crRNA and tracrRNA can be generally combined in a 1:1:1 molar ratio. However, a wide range of molar ratios can be used to produce a RNP.

A recombinant adeno-associated virus (AAV) vector can be used for delivery. Techniques to produce rAAV particles, in which an AAV genome to be packaged that includes the polynucleotide to be delivered, rep and cap genes, and helper virus functions are provided to a cell are standard in the art. Production of rAAV typically requires that the following components are present within a single cell (denoted herein as a packaging cell): a rAAV genome, AAV rep and cap genes separate from (*i.e.,* not in) the rAAV genome, and helper virus functions. The AAV rep and cap genes may be from any AAV serotype for which recombinant virus can be derived, and may be from a different AAV serotype than the rAAV genome ITRs, including, but not limited to, AAV serotypes AAV-1, AAV-2, AAV-3, AAV-4, AAV-5, AAV-6, AAV-7, AAV-8, AAV-9, AAV-10, AAV-11, AAV-12, AAV-13 and AAV rh.74. Production of pseudotyped rAAV is disclosed in, for example, international patent application publication number WO 01/83692. See Table 2.

**Table 2**

| **AAV Serotype** | **Genbank Accession No.** |
|---|---|
| AAV-1 | NC_002077.1 |
| AAV-2 | NC_001401.2 |
| AAV-3 | NC_001729.1 |
| AAV-3B | AF028705.1 |
| AAV-4 | NC_001829.1 |
| AAV-5 | NC_006152.1 |
| AAV-6 | AF028704.1 |
| AAV-7 | NC_006260.1 |
| AAV-8 | NC_006261.1 |
| AAV-9 | AX753250.1 |
| AAV-10 | AY631965.1 |
| AAV-11 | AY631966.1 |
| AAV-12 | DQ813647.1 |
| AAV-13 | EU285562.1 |

A method of generating a packaging cell involves creating a cell line that stably expresses all of the necessary components for AAV particle production. For example, a plasmid (or multiple plasmids) comprising a rAAV genome lacking AAV rep and cap genes, AAV rep and cap genes separate from the rAAV genome, and a selectable marker, such as a neomycin resistance gene, are integrated into the genome of a cell. AAV genomes have been introduced into bacterial plasmids by procedures such as GC tailing (Samulski et al., 1982, Proc. Natl. Acad. S6. USA, 79:2077-2081), addition of synthetic linkers containing restriction endonuclease cleavage sites (Laughlin et al., 1983, Gene, 23:65-73) or by direct, blunt-end ligation (Senapathy & Carter, 1984, J. Biol. Chem., 259:4661-4666). The packaging cell line can then be infected with a helper virus, such as adenovirus. The advantages of this method are that the cells are selectable and are suitable for large-scale production of rAAV. Other examples of suitable methods employ adenovirus or baculovirus, rather than plasmids, to introduce rAAV genomes and/or rep and cap genes into packaging cells.

General principles of rAAV production are reviewed in, for example, Carter, 1992, Current Opinions in Biotechnology, 1533-539; and Muzyczka, 1992, Curr. Topics in Microbial. and Immunol., 158:97-129). Various approaches are described in Ratschin et al., Mol. Cell. Biol. 4:2072 (1984); Hermonat et al., Proc. Natl. Acad. Sci. USA, 81:6466 (1984); Tratschin et al., Mo1. Cell. Biol. 5:3251 (1985); McLaughlin et al., J. Virol., 62:1963 (1988); and Lebkowski et al., 1988 Mol. Cell. Biol., 7:349 (1988). Samulski et al. (1989, J. Virol., 63:3822-3828); U.S. Patent No. 5,173,414; WO 95/13365 and corresponding U.S. Patent No. 5,658.776 ; WO 95/13392; WO 96/17947; PCT/US98/18600; WO 97/09441 (PCT/US96/14423); WO 97/08298 (PCT/US96/13872); WO 97/21825 (PCT/US96/20777); WO 97/06243 (PCT/FR96/01064); WO 99/11764; Perrin et al. (1995) Vaccine 13:1244-1250; Paul et al. (1993) Human Gene Therapy 4:609-615; Clark et al. (1996) Gene Therapy 3:1124-1132; U.S. Patent. No. 5,786,211; U.S. Patent No. 5,871,982; and U.S. Patent. No. 6,258,595.

AAV vector serotypes can be matched to target cell types. For example, the following exemplary cell types can be transduced by the indicated AAV serotypes among others. See Table 3.

**Table 3**

| **Tissue/Cell Type** | **Serotype** |
|---|---|
| Liver | AAV8, AA3, AA5, AAV9 |
| Skeletal muscle | AAV1, AAV7, AAV6, AAV8, AAV9 |
| Central nervous system | AAV5, AAV1, AAV4 |
| RPE | AAV5, AAV4 |
| Photoreceptor cells | AAV5 |
| Lung | AAV9 |
| Heart | AAV8 |
| Pancreas | AAV8 |
| Kidney | AAV2, AA8 |

In addition to adeno-associated viral vectors, other viral vectors can be used. Such viral vectors include, but are not limited to, lentivirus, alphavirus, enterovirus, pestivirus, baculovirus, herpesvirus, Epstein Barr virus, papovavirusr, poxvirus, vaccinia virus, and herpes simplex virus.

In some cases, Cas9 mRNA, sgRNA targeting one or two loci within or near the BCL11A gene or other DNA sequence that encodes a regulatory element of the BCL11A gene, and donor DNA can each be separately formulated into lipid nanoparticles, or are all co-formulated into one lipid nanoparticle.

In some cases, Cas9 mRNA can be formulated in a lipid nanoparticle, while sgRNA and donor DNA can be delivered in an AAV vector.

Options are available to deliver the Cas9 nuclease as a DNA plasmid, as mRNA or as a protein. The guide RNA can be expressed from the same DNA, or can also be delivered as an RNA. The RNA can be chemically modified to alter or improve its half-life, or decrease the likelihood or degree of immune response. The endonuclease protein can be complexed with the gRNA prior to delivery. Viral vectors allow efficient delivery; split versions of Cas9 and smaller orthologs of Cas9 can be packaged in AAV, as can donors for HDR. A range of non-viral delivery methods also exist that can deliver each of these components, or non-viral and viral methods can be employed in tandem. For example, nano-particles can be used to deliver the protein and guide RNA, while AAV can be used to deliver a donor DNA.

### Genetically Modified Cells

The term "genetically modified cell" refers to a cell that comprises at least one genetic modification introduced by genome editing (*e.g.,* using the CRISPR/Cas9/Cpf1 system). In some *ex vivo* examples herein, the genetically modified cell can be genetically modified progenitor cell. In some *in vivo* examples herein, the genetically modified cell can be a genetically modified hematopoietic progenitor cell. A genetically modified cell comprising an exogenous genome-targeting nucleic acid and/or an exogenous nucleic acid encoding a genome-targeting nucleic acid is contemplated herein.

The term "control treated population" describes a population of cells that has been treated with identical media, viral induction, nucleic acid sequences, temperature, confluency, flask size, pH, etc., with the exception of the addition of the genome editing components. Any method known in the art can be used to measure the modulation or inactivation of the transcriptional control sequence of the BCL11A gene or protein expression or activity, for example Western Blot analysis of the of the transcriptional control sequence of the BCL11A gene protein or quantifying of the transcriptional control sequence of the BCL11A gene mRNA.

The term "isolated cell" refers to a cell that has been removed from an organism in which it was originally found, or a descendant of such a cell. Optionally, the cell can be cultured *in vitro, e.g.,* under defined conditions or in the presence of other cells. Optionally, the cell can be later introduced into a second organism or reintroduced into the organism from which it (or the cell from which it is descended) was isolated.

The term "isolated population" with respect to an isolated population of cells refers to a population of cells that has been removed and separated from a mixed or heterogeneous population of cells. In some cases, the isolated population can be a substantially pure population of cells, as compared to the heterogeneous population from which the cells were isolated or enriched. In some cases, the isolated population can be an isolated population of human progenitor cells, *e.g.,* a substantially pure population of human progenitor cells, as compared to a heterogeneous population of cells comprising human progenitor cells and cells from which the human progenitor cells were derived.

The term "substantially enhanced," with respect to a particular cell population, refers to a population of cells in which the occurrence of a particular type of cell is increased relative to pre-existing or reference levels, by at least 2-fold, at least 3-, at least 4-, at least 5-, at least 6-, at least 7-, at least 8-, at least 9, at least 10-, at least 20-, at least 50-, at least 100-, at least 400-, at least 1000-, at least 5000-, at least 20000-, at least 100000- or more fold depending, *e.g.,* on the desired levels of such cells for ameliorating hemoglobinopathy.

The term "substantially enriched" with respect to a particular cell population, refers to a population of cells that is at least about 10%, about 20%, about 30%, about 40%, about 50%, about 60%, about 70% or more with respect to the cells making up a total cell population.

The term "substantially pure" with respect to a particular cell population, refers to a population of cells that is at least about 75%, at least about 85%, at least about 90%, or at least about 95% pure, with respect to the cells making up a total cell population. That is, the terms "substantially pure" or "essentially purified," with regard to a population of progenitor cells, refers to a population of cells that contain fewer than about 20%, about 15%, about 10%, about 9%, about 8%, about 7%, about 6%, about 5%, about 4%, about 3%, about 2%, about 1%, or less than 1%, of cells that are not progenitor cells as defined by the terms herein.

### Differentiation of genome-edited iPSCs into hematopoietic progenitor cells

Another step of the *ex vivo* methods of the present disclosure can comprise differentiating the genome-edited iPSCs into hematopoietic progenitor cells. The differentiating step can be performed according to any method known in the art.

### Differentiation of genome-edited mesenchymal stem cells into hematopoietic progenitor cells

Another step of the *ex vivo* methods of the present disclosure can comprise differentiating the genome-edited mesenchymal stem cells into hematopoietic progenitor cells. The differentiating step can be performed according to any method known in the art.

### Implanting cells into patients

Another step of the *ex vivo* methods of the present disclosure can comprise implanting the cells into patients. This implanting step can be accomplished using any method of implantation known in the art. For example, the genetically modified cells can be injected directly in the patient's blood or otherwise administered to the patient. The genetically modified cells may be purified *ex vivo* using a selected marker.

### Pharmaceutically Acceptable Carriers

The *ex vivo* methods of administering progenitor cells to a subject contemplated herein can involve the use of therapeutic compositions comprising progenitor cells.

Therapeutic compositions can contain a physiologically tolerable carrier together with the cell composition, and optionally at least one additional bioactive agent as described herein, dissolved or dispersed therein as an active ingredient. In some cases, the therapeutic composition is not substantially immunogenic when administered to a mammal or human patient for therapeutic purposes, unless so desired.

In general, the progenitor cells described herein can be administered as a suspension with a pharmaceutically acceptable carrier. One of skill in the art will recognize that a pharmaceutically acceptable carrier to be used in a cell composition will not include buffers, compounds, cryopreservation agents, preservatives, or other agents in amounts that substantially interfere with the viability of the cells to be delivered to the subject. A formulation comprising cells can include *e.g.,* osmotic buffers that permit cell membrane integrity to be maintained, and optionally, nutrients to maintain cell viability or enhance engraftment upon administration. Such formulations and suspensions are known to those of skill in the art and/or can be adapted for use with the progenitor cells, as described herein, using routine experimentation.

A cell composition can also be emulsified or presented as a liposome composition, provided that the emulsification procedure does not adversely affect cell viability. The cells and any other active ingredient can be mixed with excipients that are pharmaceutically acceptable and compatible with the active ingredient, and in amounts suitable for use in the therapeutic methods described herein.

Additional agents included in a cell composition can include pharmaceutically acceptable salts of the components therein. Pharmaceutically acceptable salts include the acid addition salts (formed with the free amino groups of the polypeptide) that are formed with inorganic acids, such as, for example, hydrochloric or phosphoric acids, or such organic acids as acetic, tartaric, mandelic and the like. Salts formed with the free carboxyl groups can also be derived from inorganic bases, such as, for example, sodium, potassium, ammonium, calcium or ferric hydroxides, and such organic bases as isopropylamine, trimethylamine, 2-ethylamino ethanol, histidine, procaine and the like.

Physiologically tolerable carriers are well known in the art. Exemplary liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes. Liquid compositions can also contain liquid phases in addition to and to the exclusion of water. Exemplary of such additional liquid phases are glycerine, vegetable oils such as cottonseed oil, and water-oil emulsions. The amount of an active compound used in the cell compositions that is effective in the treatment of a particular disorder or condition can depend on the nature of the disorder or condition, and can be determined by standard clinical techniques.

### Administration & Efficacy

The terms "administering," "introducing" and "transplanting" are used interchangeably in the context of the placement of cells, *e.g.,* progenitor cells, into a subject, by a method or route that results in at least partial localization of the introduced cells at a desired site, such as a site of injury or repair, such that a desired effect(s) is produced. The cells *e.g.,* progenitor cells, or their differentiated progeny can be administered by any appropriate route that results in delivery to a desired location in the subject where at least a portion of the implanted cells or components of the cells remain viable. The period of viability of the cells after administration to a subject can be as short as a few hours, *e.g.,* twenty-four hours, to a few days, to as long as several years, or even the life time of the patient, *i.e.,* long-term engraftment. For example, in some aspects described herein, an effective amount of myogenic progenitor cells is administered via a systemic route of administration, such as an intraperitoneal or intravenous route.

The terms "individual", "subject," "host" and "patient" are used interchangeably herein and refer to any subject for whom diagnosis, treatment or therapy is desired. In some aspects, the subject is a mammal. In some aspects, the subject is a human being.

When provided prophylactically, progenitor cells described herein can be administered to a subject in advance of any symptom of a hemoglobinopathy, *e.g.,* prior to the development of fatigue, shortness of breath, jaundice, slow growth late puberty, joint, bone and chest pain, enlarged spleen and liver. Accordingly, the prophylactic administration of a hematopoietic progenitor cell population serves to prevent a hemoglobinopathy, such as B-thalassemia or Sickle Cell Disease.

When provided therapeutically, hematopoietic progenitor cells are provided at (or after) the onset of a symptom or indication of hemoglobinopathy, *e.g.,* upon the onset of disease.

The hematopoietic progenitor cell population being administered according to the methods described herein can comprise allogeneic hematopoietic progenitor cells obtained from one or more donors. "Allogeneic" refers to a hematopoietic progenitor cell or biological samples comprising hematopoietic progenitor cells obtained from one or more different donors of the same species, where the genes at one or more loci are not identical. For example, a hematopoietic progenitor cell population being administered to a subject can be derived from one more unrelated donor subjects, or from one or more non-identical siblings. In some cases, syngeneic hematopoietic progenitor cell populations can be used, such as those obtained from genetically identical animals, or from identical twins. The hematopoietic progenitor cells can be autologous cells; that is, the hematopoietic progenitor cells are obtained or isolated from a subject and administered to the same subject, *i.e.,* the donor and recipient are the same.

The term "effective amount" refers to the amount of a population of progenitor cells or their progeny needed to prevent or alleviate at least one or more signs or symptoms of hemoglobinopathy, and relates to a sufficient amount of a composition to provide the desired effect, *e.g.,* to treat a subject having hemoglobinopathy. The term "therapeutically effective amount" therefore refers to an amount of progenitor cells or a composition comprising progenitor cells that is sufficient to promote a particular effect when administered to a typical subject, such as one who has or is at risk for hemoglobinopathy. An effective amount would also include an amount sufficient to prevent or delay the development of a symptom of the disease, alter the course of a symptom of the disease (for example but not limited to, slow the progression of a symptom of the disease), or reverse a symptom of the disease. It is understood that for any given case, an appropriate "effective amount" can be determined by one of ordinary skill in the art using routine experimentation.

For use in the various aspects described herein, an effective amount of progenitor cells comprises at least 10² progenitor cells, at least 5 × 10² progenitor cells, at least 10³ progenitor cells, at least 5 × 10³ progenitor cells, at least 10⁴ progenitor cells, at least 5 × 10⁴ progenitor cells, at least 10⁵ progenitor cells, at least 2 × 10⁵ progenitor cells, at least 3 × 10⁵ progenitor cells, at least 4 × 10⁵ progenitor cells, at least 5 × 10⁵ progenitor cells, at least 6 × 10⁵ progenitor cells, at least 7 × 10⁵ progenitor cells, at least 8 × 10⁵ progenitor cells, at least 9 × 10⁵ progenitor cells, at least 1 × 10⁶ progenitor cells, at least 2 × 10⁶ progenitor cells, at least 3 × 10⁶ progenitor cells, at least 4 × 10⁶ progenitor cells, at least 5 × 10⁶ progenitor cells, at least 6 × 10⁶ progenitor cells, at least 7 × 10⁶ progenitor cells, at least 8 × 10⁶ progenitor cells, at least 9 × 10⁶ progenitor cells, or multiples thereof. The progenitor cells can be derived from one or more donors, or can be obtained from an autologous source. In some examples described herein, the progenitor cells can be expanded in culture prior to administration to a subject in need thereof.

"Administered" refers to the delivery of a progenitor cell composition into a subject by a method or route that results in at least partial localization of the cell composition at a desired site. A cell composition can be administered by any appropriate route that results in effective treatment in the subject, *i.e.* administration results in delivery to a desired location in the subject where at least a portion of the composition delivered, *i.e.* at least 1 × 10⁴ cells are delivered to the desired site for a period of time. Modes of administration include injection, infusion, instillation, or ingestion. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. In some examples, the route is intravenous. For the delivery of cells, administration by injection or infusion can be made.

The cells can be administered systemically. The phrases "systemic administration," "administered systemically", "peripheral administration" and "administered peripherally" refer to the administration of a population of progenitor cells other than directly into a target site, tissue, or organ, such that it enters, instead, the subject's circulatory system and, thus, is subject to metabolism and other like processes.

The efficacy of a treatment comprising a composition for the treatment of hemoglobinopathies can be determined by the skilled clinician. However, a treatment is considered "effective treatment," if any one or all of the signs or symptoms of, as but one example, levels of functional BCL11A and functional HbF are altered in a beneficial manner (e.g., decreased by at least 10% for BCL11A and/or increased by at least 10% for HbF), or other clinically accepted symptoms or markers of disease are improved or ameliorated. Efficacy can also be measured by failure of an individual to worsen as assessed by hospitalization or need for medical interventions (*e.g.,* progression of the disease is halted or at least slowed). Methods of measuring these indicators are known to those of skill in the art and/or described herein. Treatment includes any treatment of a disease in an individual or an animal (some non-limiting examples include a human, or a mammal) and includes: (1) inhibiting the disease, *e.g.,* arresting, or slowing the progression of symptoms; or (2) relieving the disease, *e.g.,* causing regression of symptoms; and (3) preventing or reducing the likelihood of the development of symptoms.

The treatment according to the present disclosure can ameliorate one or more symptoms associated with hemoglobinopathies by decreasing the amount of functional BCL11A and/or increasing the amount of functional HbF in the individual. Early signs typically associated with hemoglobinopathies include for example, fatigue, shortness of breath, jaundice, slow growth late puberty, joint, bone and chest pain, enlarged spleen and liver.

### Kits

The present disclosure discloses kits for carrying out the methods described herein. A kit can include one or more of a genome-targeting nucleic acid, a polynucleotide encoding a genome-targeting nucleic acid, a site-directed polypeptide, a polynucleotide encoding a site-directed polypeptide, and/or any nucleic acid or proteinaceous molecule necessary to carry out the aspects of the methods described herein, or any combination thereof.

A kit can comprise: (1) a vector comprising a nucleotide sequence encoding a genome-targeting nucleic acid, (2) the site-directed polypeptide or a vector comprising a nucleotide sequence encoding the site-directed polypeptide, and (3) a reagent for reconstitution and/or dilution of the vector(s) and or polypeptide.

A kit can comprise: (1) a vector comprising (i) a nucleotide sequence encoding a genome-targeting nucleic acid, and (ii) a nucleotide sequence encoding the site-directed polypeptide; and (2) a reagent for reconstitution and/or dilution of the vector.

In any of the above kits, the kit can comprise a single-molecule guide genome-targeting nucleic acid. In any of the above kits, the kit can comprise a double-molecule genome-targeting nucleic acid. In any of the above kits, the kit can comprise two or more double-molecule guides or single-molecule guides. The kits can comprise a vector that encodes the nucleic acid targeting nucleic acid.

In any of the above kits, the kit can further comprise a polynucleotide to be inserted to effect the desired genetic modification.

Components of a kit can be in separate containers, or combined in a single container.

Any kit described above can further comprise one or more additional reagents, where such additional reagents are selected from a buffer, a buffer for introducing a polypeptide or polynucleotide into a cell, a wash buffer, a control reagent, a control vector, a control RNA polynucleotide, a reagent for *in vitro* production of the polypeptide from DNA, adaptors for sequencing and the like. A buffer can be a stabilization buffer, a reconstituting buffer, a diluting buffer, or the like. A kit can also comprise one or more components that can be used to facilitate or enhance the on-target binding or the cleavage of DNA by the endonuclease, or improve the specificity of targeting.

In addition to the above-mentioned components, a kit can further comprise instructions for using the components of the kit to practice the methods. The instructions for practicing the methods can be recorded on a suitable recording medium. For example, the instructions can be printed on a substrate, such as paper or plastic, etc. The instructions can be present in the kits as a package insert, in the labeling of the container of the kit or components thereof (*i.e.,* associated with the packaging or subpackaging), etc. The instructions can be present as an electronic storage data file present on a suitable computer readable storage medium, *e.g.* CD-ROM, diskette, flash drive, etc. In some instances, the actual instructions are not present in the kit, but means for obtaining the instructions from a remote source (*e.g.* via the Internet), can be provided. An example of this case is a kit that comprises a web address where the instructions can be viewed and/or from which the instructions can be downloaded. As with the instructions, this means for obtaining the instructions can be recorded on a suitable substrate.

### Guide RNA Formulation

Guide RNAs of the present disclosure can be formulated with pharmaceutically acceptable excipients such as carriers, solvents, stabilizers, adjuvants, diluents, etc., depending upon the particular mode of administration and dosage form. Guide RNA compositions can be formulated to achieve a physiologically compatible pH, and range from a pH of about 3 to a pH of about 11, about pH 3 to about pH 7, depending on the formulation and route of administration. In some cases, the pH can be adjusted to a range from about pH 5.0 to about pH 8. In some cases, the compositions can comprise a therapeutically effective amount of at least one compound as described herein, together with one or more pharmaceutically acceptable excipients. Optionally, the compositions can comprise a combination of the compounds described herein, or can include a second active ingredient useful in the treatment or prevention of bacterial growth (for example and without limitation, anti-bacterial or anti-microbial agents), or can include a combination of reagents of the present disclosure.

Suitable excipients include, for example, carrier molecules that include large, slowly metabolized macromolecules such as proteins, polysaccharides, polylactic acids, polyglycolic acids, polymeric amino acids, amino acid copolymers, and inactive virus particles. Other exemplary excipients can include antioxidants (for example and without limitation, ascorbic acid), chelating agents (for example and without limitation, EDTA), carbohydrates (for example and without limitation, dextrin, hydroxyalkylcellulose, and hydroxyalkylmethylcellulose), stearic acid, liquids (for example and without limitation, oils, water, saline, glycerol and ethanol), wetting or emulsifying agents, pH buffering substances, and the like.

### Other Possible Therapeutic Approaches

Gene editing can be conducted using nucleases engineered to target specific sequences. To date there are four major types of nucleases: meganucleases and their derivatives, zinc finger nucleases (ZFNs), transcription activator like effector nucleases (TALENs), and CRISPR-Cas9 nuclease systems. The nuclease platforms vary in difficulty of design, targeting density and mode of action, particularly as the specificity of ZFNs and TALENs is through protein-DNA interactions, while RNA-DNA interactions primarily guide Cas9. Cas9 cleavage also requires an adjacent motif, the PAM, which differs between different CRISPR systems. Cas9 from *Streptococcus pyogenes* cleaves using a NGG PAM, CRISPR from *Neisseria meningitidis* can cleave at sites with PAMs including NNNNGATT, NNNNNGTTT and NNNNGCTT. A number of other Cas9 orthologs target protospacer adjacent to alternative PAMs.

CRISPR endonucleases, such as Cas9, can be used in the methods of the present disclosure. However, the teachings described herein, such as therapeutic target sites, could be applied to other forms of endonucleases, such as ZFNs, TALENs, HEs, or MegaTALs, or using combinations of nulceases. However, in order to apply the teachings of the present disclosure to such endonucleases, one would need to, among other things, engineer proteins directed to the specific target sites.

Additional binding domains can be fused to the Cas9 protein to increase specificity. The target sites of these constructs would map to the identified gRNA specified site, but would require additional binding motifs, such as for a zinc finger domain. In the case of Mega-TAL, a meganuclease can be fused to a TALE DNA-binding domain. The meganuclease domain can increase specificity and provide the cleavage. Similarly, inactivated or dead Cas9 (dCas9) can be fused to a cleavage domain and require the sgRNA/Cas9 target site and adjacent binding site for the fused DNA-binding domain. This likely would require some protein engineering of the dCas9, in addition to the catalytic inactivation, to decrease binding without the additional binding site.

### Zinc Finger Nucleases

Zinc finger nucleases (ZFNs) are modular proteins comprised of an engineered zinc finger DNA binding domain linked to the catalytic domain of the type II endonuclease Fokl. Because Fokl functions only as a dimer, a pair of ZFNs must be engineered to bind to cognate target "half-site" sequences on opposite DNA strands and with precise spacing between them to enable the catalytically active Fokl dimer to form. Upon dimerization of the Fokl domain, which itself has no sequence specificity per se, a DNA double-strand break is generated between the ZFN half-sites as the initiating step in genome editing.

The DNA binding domain of each ZFN is typically comprised of 3-6 zinc fingers of the abundant Cys2-His2 architecture, with each finger primarily recognizing a triplet of nucleotides on one strand of the target DNA sequence, although cross-strand interaction with a fourth nucleotide also can be important. Alteration of the amino acids of a finger in positions that make key contacts with the DNA alters the sequence specificity of a given finger. Thus, a four-finger zinc finger protein will selectively recognize a 12 bp target sequence, where the target sequence is a composite of the triplet preferences contributed by each finger, although triplet preference can be influenced to varying degrees by neighboring fingers. An important aspect of ZFNs is that they can be readily re-targeted to almost any genomic address simply by modifying individual fingers, although considerable expertise is required to do this well. In most applications of ZFNs, proteins of 4-6 fingers are used, recognizing 12-18 bp respectively. Hence, a pair of ZFNs will typically recognize a combined target sequence of 24-36 bp, not including the typical 5-7 bp spacer between half-sites. The binding sites can be separated further with larger spacers, including 15-17 bp. A target sequence of this length is likely to be unique in the human genome, assuming repetitive sequences or gene homologs are excluded during the design process. Nevertheless, the ZFN protein-DNA interactions are not absolute in their specificity so off-target binding and cleavage events do occur, either as a heterodimer between the two ZFNs, or as a homodimer of one or the other of the ZFNs. The latter possibility has been effectively eliminated by engineering the dimerization interface of the Fokl domain to create "plus" and "minus" variants, also known as obligate heterodimer variants, which can only dimerize with each other, and not with themselves. Forcing the obligate heterodimer prevents formation of the homodimer. This has greatly enhanced specificity of ZFNs, as well as any other nuclease that adopts these Fokl variants.

A variety of ZFN-based systems have been described in the art, modifications thereof are regularly reported, and numerous references describe rules and parameters that are used to guide the design of ZFNs; see, *e.g.,* Segal et al., Proc Natl Acad Sci USA 96(6):2758-63 (1999); Dreier B et al., J Mol Biol. 303(4):489-502 (2000); Liu Q et al., J Biol Chem. 277(6):3850-6 (2002); Dreier et al., J Biol Chem 280(42):35588-97 (2005); and Dreier et al., J Biol Chem. 276(31):29466-78 (2001).

### Transcription Activator-Like Effector Nucleases (TALENs)

TALENs represent another format of modular nucleases whereby, as with ZFNs, an engineered DNA binding domain is linked to the Fokl nuclease domain, and a pair of TALENs operate in tandem to achieve targeted DNA cleavage. The major difference from ZFNs is the nature of the DNA binding domain and the associated target DNA sequence recognition properties. The TALEN DNA binding domain derives from TALE proteins, which were originally described in the plant bacterial pathogen *Xanthomonas sp.* TALEs are comprised of tandem arrays of 33-35 amino acid repeats, with each repeat recognizing a single basepair in the target DNA sequence that is typically up to 20 bp in length, giving a total target sequence length of up to 40 bp. Nucleotide specificity of each repeat is determined by the repeat variable diresidue (RVD), which includes just two amino acids at positions 12 and 13. The bases guanine, adenine, cytosine and thymine are predominantly recognized by the four RVDs: Asn-Asn, Asn-Ile, His-Asp and Asn-Gly, respectively. This constitutes a much simpler recognition code than for zinc fingers, and thus represents an advantage over the latter for nuclease design. Nevertheless, as with ZFNs, the protein-DNA interactions of TALENs are not absolute in their specificity, and TALENs have also benefitted from the use of obligate heterodimer variants of the Fokl domain to reduce off-target activity.

Additional variants of the Fokl domain have been created that are deactivated in their catalytic function. If one half of either a TALEN or a ZFN pair contains an inactive Fokl domain, then only single-strand DNA cleavage (nicking) will occur at the target site, rather than a DSB. The outcome is comparable to the use of CRISPR/Cas9/Cpf1 "nickase" mutants in which one of the Cas9 cleavage domains has been deactivated. DNA nicks can be used to drive genome editing by HDR, but at lower efficiency than with a DSB. The main benefit is that off-target nicks are quickly and accurately repaired, unlike the DSB, which is prone to NHEJ-mediated mis-repair.

A variety of TALEN-based systems have been described in the art, and modifications thereof are regularly reported; see, *e.g.,* Boch, Science 326(5959):1509-12 (2009); Mak et al., Science 335(6069):716-9 (2012); and Moscou et al., Science 326(5959):1501 (2009). The use of TALENs based on the "Golden Gate" platform, or cloning scheme, has been described by multiple groups; see, *e.g.,* Cermak et al., Nucleic Acids Res. 39(12):e82 (2011); Li et al., Nucleic Acids Res. 39(14):6315-25(2011); Weber et al., PLoS One. 6(2):e16765 (2011); Wang et al., J Genet Genomics 41(6):339-47, Epub 2014 May 17 (2014); and Cermak T et al., Methods MolBiol. 1239:133-59 (2015).

### Homing Endonucleases

Homing endonucleases (HEs) are sequence-specific endonucleases that have long recognition sequences (14-44 base pairs) and cleave DNA with high specificity - often at sites unique in the genome. There are at least six known families of HEs as classified by their structure, including LAGLIDADG (SEQ ID NO. 71,949), GIY-YIG, His-Cis box, H-N-H, PD-(D/E)xK, and Vsr-like that are derived from a broad range of hosts, including eukarya, protists, bacteria, archaea, cyanobacteria and phage. As with ZFNs and TALENs, HEs can be used to create a DSB at a target locus as the initial step in genome editing. In addition, some natural and engineered HEs cut only a single strand of DNA, thereby functioning as site-specific nickases. The large target sequence of HEs and the specificity that they offer have made them attractive candidates to create site-specific DSBs.

A variety of HE-based systems have been described in the art, and modifications thereof are regularly reported; see, e.g., the reviews by Steentoft et al., Glycobiology 24(8):663-80 (2014); Belfort and Bonocora, Methods Mol Biol. 1123:1-26 (2014); Hafez and Hausner, Genome 55(8):553-69 (2012); and references cited therein.

### MegaTAL / Tev-mTALEN / MegaTev

As further examples of hybrid nucleases, the MegaTAL platform and Tev-mTALEN platform use a fusion of TALE DNA binding domains and catalytically active HEs, taking advantage of both the tunable DNA binding and specificity of the TALE, as well as the cleavage sequence specificity of the HE; see, *e.g.,* Boissel et al., NAR 42: 2591-2601 (2014); Kleinstiver et al., G3 4:1155-65 (2014); and Boissel and Scharenberg, Methods Mol. Biol. 1239: 171-96 (2015).

In a further variation, the MegaTev architecture is the fusion of a meganuclease (Mega) with the nuclease domain derived from the GIY-YIG homing endonuclease I-Tevl (Tev). The two active sites are positioned ~30 bp apart on a DNA substrate and generate two DSBs with non-compatible cohesive ends; see, *e.g.,* Wolfs et al., NAR 42, 8816-29 (2014). It is anticipated that other combinations of existing nuclease-based approaches will evolve and be useful in achieving the targeted genome modifications described herein.

### dCas9-Fokl or dCpf1-Fok1 and Other Nucleases

Combining the structural and functional properties of the nuclease platforms described above offers a further approach to genome editing that can potentially overcome some of the inherent deficiencies. As an example, the CRISPR genome editing system typically uses a single Cas9 endonuclease to create a DSB. The specificity of targeting is driven by a 20 or 24 nucleotide sequence in the guide RNA that undergoes Watson-Crick base-pairing with the target DNA (plus an additional 2 bases in the adjacent NAG or NGG PAM sequence in the case of Cas9 from *S. pyogenes*)*.* Such a sequence is long enough to be unique in the human genome, however, the specificity of the RNA/DNA interaction is not absolute, with significant promiscuity sometimes tolerated, particularly in the 5' half of the target sequence, effectively reducing the number of bases that drive specificity. One solution to this has been to completely deactivate the Cas9 or Cpf1 catalytic function - retaining only the RNA-guided DNA binding function - and instead fusing a Fokl domain to the deactivated Cas9; see, *e.g.,* Tsai et al., Nature Biotech 32: 569-76 (2014); and Guilinger et al., Nature Biotech. 32: 577-82 (2014). Because Fokl must dimerize to become catalytically active, two guide RNAs are required to tether two Fokl fusions in close proximity to form the dimer and cleave DNA. This essentially doubles the number of bases in the combined target sites, thereby increasing the stringency of targeting by CRISPR-based systems.

As further example, fusion of the TALE DNA binding domain to a catalytically active HE, such as I-Tevl, takes advantage of both the tunable DNA binding and specificity of the TALE, as well as the cleavage sequence specificity of I-Tevl, with the expectation that off-target cleavage can be further reduced.

### On- and off-target mutation detection by sequencing

To sequence on-target sites and putative off-target sites, the appropriate amplification primers were identified and reactions were set up with these primers using the genomic DNA harvested using QuickExtract DNA extraction solution (Epicentre) from treated cells three days post-transfection. The amplification primers contain the gene specific portion flanked by adapters. The forward primer's 5' end includes a modified forward (read1) primer-binding site. The reverse primer's 5' end contains a combined modified reverse (read2) and barcode primer-binding site, in opposite orientation. The individual PCR reactions were validated by separating on agarose gels, then purified and re-amplified. The second round forward primers contain the Illumina P5 sequence, followed by a proportion of the modified forward (read1) primer binding site. The second round reverse primers contain the Illumina P7 sequence (at the 5' end), followed by the 6-base barcode and the combined modified reverse (read2) and barcode primer binding site. The second round amplifications were also checked on agarose gels, then purified, and quantitated using a NanoDrop spectrophotometer. The amplification products were pooled to match concentration and then submitted to the Emory Integrated Genomic core for library prepping and sequencing on an Illumina Miseq machine.

The sequencing reads were sorted by barcode and then aligned to the reference sequences supplied by bioinformatics for each product. Insertion and deletion rates in the aligned sequencing reads were detected in the region of the putative cut sites using software previously described; see, *e.g.,* Lin et al., Nucleic Acids Res., 42: 7473-7485 (2014). The levels of insertions and deletions detected in this window were then compared to the level seen in the same location in genomic DNA isolated from in mock transfected cells to minimize the effects of sequencing artifacts.

### Mutation detection assays

The on- and off-target cleavage activities of Cas9 and guide RNA combinations were measured using the mutation rates resulting from the imperfect repair of double-strand breaks by NHEJ.

On-target loci were amplified using AccuPrime Taq DNA Polymerase High Fidelity (Life Technologies, Carlsbad, CA) following manufacturer's instructions for 40 cycles (94°C, 30 s; 52-60°C, 30 s; 68°C, 60 s) in 50 µl reactions containing 1 µl of the cell lysate, and 1 µl of each 10 µM amplification primer. T7EI mutation detection assays were performed, as per manufacturers protocol [Reyon et al., Nat. Biotechnol., 30: 460-465 (2012)], with the digestions separated on 2% agarose gels and quantified using ImageJ [Guschin et al., Methods Mol. Biol., 649: 247-256 (2010)]. The assays determine the percentage of insertions/deletions ("indels") in the bulk population of cells.

### Methods and Compositions

Accordingly, the present disclosure relates in particular to the following non-limiting aspects: In a first method, Method 1, the present disclosure provides a method for editing a BCL11A gene in a human cell by genome editing, the method comprising the step of: introducing into the human cell one or more deoxyribonucleic acid (DNA) endonucleases to effect one or more single-strand breaks (SSBs) or double-strand breaks (DSBs) within or near the BCL11A gene or other DNA sequence that encodes a regulatory element of the BCL11A gene that results in a permanent deletion, modulation, or inactivation of a transcriptional control sequence of the BCL11A gene.

In another method, Method 2, the present disclosure provides a method for editing a BCL11A gene in a human cell by genome editing, as provided in Method 1, wherein the transcriptional control sequence is located within a second intron of the BCL11A gene.

In another method, Method 3, the present disclosure provides a method for editing a BCL11A gene in a human cell by genome editing, as provided in Methods 1 or 2, wherein the transcriptional control sequence is located within a +58 DNA hypersensitive site (DHS) of the BCL11A gene.

In another method, Method 4, the present disclosure describes an *ex vivo* method for treating a patient with a hemoglobinopathy, the method comprising the steps of: creating a patient specific induced pluripotent stem cell (iPSC); editing within or near a BCL11A gene or other DNA sequence that encodes a regulatory element of the BCL11A gene of the iPSC; differentiating the genome-edited iPSC into a hematopoietic progenitor cell; and implanting the hematopoietic progenitor cell into the patient.

In another method, Method 5, the present disclosure describes an ex *vivo* method for treating a patient with a hemoglobinopathy as described in Method 4 wherein the creating step comprises: isolating a somatic cell from the patient; and introducing a set of pluripotency-associated genes into the somatic cell to induce the somatic cell to become a pluripotent stem cell.

In another method, Method 6, the present disclosure described an *ex vivo* method for treating a patient with a hemoglobinopathy as described in Method 5, wherein the somatic cell is a fibroblast.

In another method, Method 7, the present disclosure describes an *ex vivo* method for treating a patient with a hemoglobinopathy as described in Methods 5 or 6, wherein the set of pluripotency-associated genes is one or more of the genes selected from the group consisting of OCT4, SOX2, KLF4, Lin28, NANOG and cMYC.

In another method, Method 8, the present disclosure describeddescribed an *ex vivo* method for treating a patient with a hemoglobinopathy as described in any one of Methods 4-7, wherein the editing step comprises introducing into the iPSC one or more deoxyribonucleic acid (DNA) endonucleases to effect one or more single-strand breaks (SSBs) or double-strand breaks (DSBs) within or near the BCL11A gene or other DNA sequence that encodes a regulatory element of the BCL11A gene that results in a permanent deletion, modulation, or inactivation of a transcriptional control sequence of the BCL11A gene.

In another method, Method 9, the present disclosure discloses an *ex vivo* method for treating a patient with a hemoglobinopathy as disclosed in any one of Methods 4-8, wherein the differentiating step comprises one or more of the following to differentiate the genome-edited iPSC into a hematopoietic progenitor cell: treatment with a combination of small molecules, delivery of master transcription factors, delivery of mRNA encoding master transcription factors, or delivery of mRNA encoding transcription factors.

In another method, Method 10, the present disclosure discloses an *ex vivo* method for treating a patient with a hemoglobinopathy as disclosed in any one of Methods 4-9, wherein the implanting step comprises implanting the hematopoietic progenitor cell into the patient by transplantation, local injection, systemic infusion, or combinations thereof.

In another method, Method 11, the present disclosure discloses an *ex vivo* method for treating a patient with a hemoglobinopathy, the method comprising the steps of: isolating a mesenchymal stem cell from the patient; editing within or near a BCL11A gene or other DNA sequence that encodes a regulatory element of the BCL11A gene of the mesenchymal stem cell; differentiating the genome-edited mesenchymal stem cell into a hematopoietic progenitor cell; and implanting the hematopoietic progenitor cell into the patient.

In another method, Method 12, the present disclosure discloses an *ex vivo* method for treating a patient with a hemoglobinopathy as disclosed in Method 11, wherein the mesenchymal stem cell is isolated from the patient's bone marrow or peripheral blood.

In another method, Method 13, the present disclosure disclose an *ex vivo* method for treating a patient with a hemoglobinopathy as disclosed in Methods 11 or 12, wherein the isolating step comprises: aspiration of bone marrow and isolation of mesenchymal cells using density gradient centrifugation media.

In another method, Method 14, the present disclosure discloses an *ex vivo* method for treating a patient with a hemoglobinopathy as disclosed in any one of Methods 11-13, wherein the editing step comprises introducing into the mesenchymal stem cell one or more deoxyribonucleic acid (DNA) endonucleases to effect one or more single-strand breaks (SSBs) or double-strand breaks (DSBs) within or near the BCL11A gene or other DNA sequence that encodes a regulatory element of the BCL11A gene that results in a permanent deletion, modulation, or inactivation of a transcriptional control sequence of the BCL11A gene.

In another method, Method 15, the present disclosure discloses an *ex vivo* method for treating a patient with a hemoglobinopathy as disclosed in any one of Methods 11-14, wherein the differentiating step comprises one or more of the following to differentiate the genome-edited mesenchymal stem cell into a hematopoietic progenitor cell: treatment with a combination of small molecules, delivery of master transcription factors, delivery of mRNA encoding master transcription factors, or delivery of mRNA encoding transcription factors.

In another method, Method 16, the present disclosure discloses an *ex vivo* method for treating a patient with a hemoglobinopathy as disclosed in any one of Methods 11-15, wherein the implanting step comprises implanting the hematopoietic progenitor cell into the patient by transplantation, local injection, systemic infusion, or combinations thereof.

In another method, Method 17, the present disclosure discloses an *ex vivo* method for treating a patient with a hemoglobinopathy, the method comprising the steps of: isolating a hematopoietic progenitor cell from the patient; editing within or near a BCL11A gene or other DNA sequence that encodes a regulatory element of the BCL11A gene of the hematopoietic progenitor cell; and implanting the genome-edited hematopoietic progenitor cell into the patient.

In another method, Method 18, the present disclosure discloses an *ex vivo* method for treating a patient with a hemoglobinopathy as provided in Method 17, wherein the method further comprises treating the patient with granulocyte colony stimulating factor (GCSF) prior to the isolating step.

In another method, Method 19, the present disclosure discloses an *ex vivo* method for treating a patient with a hemoglobinopathy as disclosed in Method 18, wherein the treating step is performed in combination with Plerixaflor.

In another method, Method 20, the present disclosure disclosed an *ex vivo* method for treating a patient with a hemoglobinopathy as disclosed in any one of Methods 17-19, wherein the isolating step comprises isolating CD34+ cells.

In another method, Method 21, the present disclosure discloses an *ex vivo* method for treating a patient with a hemoglobinopathy as disclosed in any one of Methods 17-20, wherein the editing step comprises introducing into the hematopoietic progenitor cell one or more deoxyribonucleic acid (DNA) endonucleases to effect one or more single-strand breaks (SSBs) or double-strand breaks (DSBs) within or near the BCL11A gene or other DNA sequence that encodes a regulatory element of the BCL11A gene that results in a permanent deletion, modulation, or inactivation of a transcriptional control sequence of the BCL11A gene.

In another method, Method 22, the present disclosure discloses an *ex vivo* method for treating a patient with a hemoglobinopathy as disclosed in any one of Methods 17-21, wherein the implanting step comprises implanting the genome-edited hematopoietic progenitor cell into the patient by transplantation, local injection, systemic infusion, or combinations thereof.

In another method, Method 23, the present disclosure discloses an *in vivo* method for treating a patient with a hemoglobinopathy, the method comprising the step of editing a BCL11A gene in a cell of the patient.

In another method, Method 24, the present disclosure discloses an *in vivo* method for treating a patient with a hemoglobinopathy as disclosed in Method 23, wherein the editing step comprises introducing into the cell one or more deoxyribonucleic acid (DNA) endonucleases to effect one or more single-strand breaks (SSBs) or double-strand breaks (DSBs) within or near the BCL11A gene or other DNA sequence that encodes a regulatory element of the BCL11A gene that results in a permanent deletion, modulation, or inactivation of a transcriptional control of the BCL11A gene.

In another method, Method 25, the present disclosure discloses an in vivo method for treating a patient with a hemoglobinopathy as provided in Methods 23 or 24, wherein the cell is a bone marrow cell, a hematopoietic progenitor cell, or a CD34+ cell.

In another method, Method 26, the present disclosure discloses a method according to any one of Methods 1, 8, 14, 21 and 24, wherein the one or more DNA endonucleases is a Cas1, Cas1B, Cas2, Cas3, Cas4, Cas5, Cas6, Cas7, Cas8, Cas9 (also known as Csn1 and Csx12), Cas100, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, or Cpf1 endonuclease; a homolog thereof, a recombination of the naturally occurring molecule thereof, codon-optimized thereof, or modified versions thereof, and combinations thereof.

In another method, Method 27, the present disclosure provides a method as disclosed in Method 26, wherein the method comprises introducing into the cell one or more polynucleotides encoding the one or more DNA endonucleases.

In another method, Method 28, the present disclosure provides a method as provided in Methods 26 or 27, wherein the method comprises introducing into the cell one or more ribonucleic acids (RNAs) encoding the one or more DNA endonucleases.

In another method, Method 29, the present disclosure discloses a method as disclosed in Methods 27 or 28, wherein the one or more polynucleotides or one or more RNAs is one or more modified polynucleotides or one or more modified RNAs.

In another method, Method 30, the present disclosure discloses a method as disclosed in Method 26, wherein the one or more DNA endonucleases is one or more proteins or polypeptides.

In another method, Method 31, the present disclosure provides a method as disclosed in Method 30, wherein the one or more proteins or polypeptides is flanked at the N-terminus, the C-terminus, or both the N-terminus and C-terminus by one or more nuclear localization signals (NLSs).

In another method, Method 32, the present disclosure provides a method as provided in Method 31, wherein the one or more proteins or polypeptides is flanked by two NLSs, one NLS located at the N-terminus and the second NLS located at the C-terminus.

In another method, Method 33, the present disclosure provides a method as provided in any one of Methods 31-32, wherein the one or more NLSs is a SV40 NLS.

In another method, Method 34, the present disclosure provides a method as provided in any one of Methods 1-33, wherein the method further comprises introducing into the cell one or more guide ribonucleic acids (gRNAs).

In another method, Method 35, the present disclosure provides a method as provided in Method 34, wherein the one or more gRNAs are single-molecule guide RNA (sgRNAs).

In another method, Method 36, the present disclosure provides a method as provided in Methods 34 or 35, wherein the one or more gRNAs or one or more sgRNAs is one or more modified gRNAs or one or more modified sgRNAs.

In another method, Method 37, the present disclosure provides a method as provided in Method 36, wherein the one or more modified sgRNAs comprises three 2'-O-methyl-phosphorothioate residues at or near each of its 5' and 3' ends.

In another method, Method 38, the present disclosure provides a method as provided in Method 37, wherein the modified sgRNA is the nucleic acid sequence of SEQ ID NO: 71,959.

In another method, Method 39, the present disclosure provides a method as provided in Methods 34-38, wherein the one or more DNA endonucleases is pre-complexed with one or more gRNAs or one or more sgRNAs to form one or more ribonucleoproteins (RNPs).

In another method, Method 40, the present disclosure provides a method as provided in Method 39, wherein the weight ratio of sgRNA to DNA endonuclease in the RNP is 1:1.

In another method, Method 41, the present disclosure provides a method as provided in Method 40, wherein the sgRNA comprises the nucleic acid sequence of SEQ ID NO: 71,959, the DNA endonuclease is a *S*. *pyogenes* Cas9 comprising a N-terminus SV40 NLS and a C-terminus SV40 NLS, wherein the weight ratio of sgRNA to DNA endonuclease is 1:1.

In another method, Method 42, the present disclosure provides a method as provided in any one of Methods 1-41, wherein the method further comprises introducing into the cell a polynucleotide donor template comprising a wild-type BCL11A gene or cDNA comprising a modified transcriptional control sequence.

In another method, Method 43, the present disclosure provides a method as provided in any one of Methods 1, 8, 14, 21, or 24, wherein the method further comprises introducing into the cell one guide ribonucleic acid (gRNA) and a polynucleotide donor template comprising a wild-type BCL11A gene or cDNA comprising a modified transcriptional control sequence, and wherein the one or more DNA endonucleases is one or more Cas9 or Cpf1 endonucleases that effect one single-strand break (SSB) or double-strand break (DSB) at a locus within or near the BCL11A gene or other DNA sequence that encodes a regulatory element of the BCL11A gene that facilitates insertion of a new sequence from the polynucleotide donor template into the chromosomal DNA at the locus that results in a permanent insertion, modulation, or inactivation of the transcriptional control sequence of the chromosomal DNA proximal to the locus, and wherein the gRNA comprises a spacer sequence that is complementary to a segment of the locus.

In another method, Method 44, the present disclosure provides a method as provided in Method 43, wherein proximal means nucleotides both upstream and downstream of the locus.

In another method, Method 45, the present disclosure provides a method as provided in any one of Methods 1, 8, 14, 21, or 24, wherein the method further comprises introducing into the cell one or more guide ribonucleic acid (gRNAs) and a polynucleotide donor template comprising a wild-type BCL11A gene or cDNA comprising a modified transcriptional control sequence, and wherein the one or more DNA endonucleases is one or more Cas9 or Cpf1 endonucleases that effect or create a pair of single-strand breaks (SSBs) or double-strand breaks (DSBs), the first break at a 5' locus and the second break at a 3' locus, within or near the BCL11A gene or other DNA sequence that encodes a regulatory element of the BCL11A gene that facilitates insertion of a new sequence from the polynucleotide donor template into the chromosomal DNA between the 5' locus and the 3' locus that results in a permanent insertion, modulation, or inactivation of the transcriptional control sequence of the chromosomal DNA between the 5' locus and the 3' locus.

In another method, Method 46, the present disclosure provides a method as provided in Method 45, wherein one gRNA creates a pair of SSBs or DSBs.

In another method, Method 47, the present disclosure provides a method as provided in Method 45, wherein one gRNA comprises a spacer sequence that is complementary to either the 5' locus or the 3' locus.

In another method, Method 48, the present disclosure provides a method as provided in Method 45, wherein the method comprises a first guide RNA and a second guide RNA, wherein the first guide RNA comprises a spacer sequence that is complementary to a segment of the 5' locus and the second guide RNA comprises a spacer sequence that is complementary to a segment of the 3' locus.

In another method, Method 49, the present disclosure provides a method as provided in any one of Methods 43-48, wherein the one or two gRNAs are one or two single-molecule guide RNA (sgRNAs).

In another method, Method 50, the present disclosure provides a method as provided in any one of Methods 43-49, wherein the one or two gRNAs or one or two sgRNAs is one or two modified gRNAs or one or two modified sgRNAs.

In another method, Method 51, the present disclosure provides a method as provided in Method 50, wherein the one modified sgRNA comprises three 2'-O-methyl-phosphorothioate residues at or near each of its 5' and 3' ends.

In another method, Method 52, the present disclosure provides a method as provided in Method 51, wherein the one modified sgRNA is the nucleic acid sequence of SEQ ID NO: 71,959.

In another method, Method 53, the present disclosure provides a method as provided in any one of Methods 43-52, wherein the one or more Cas9 endonucleases is pre-complexed with one or two gRNAs or one or two sgRNAs to form one or more ribonucleoproteins (RNPs).

In another method, Method 54, the present disclosure provides a method as provided in Method 53, wherein the one or more Cas9 endonuclease is flanked at the N-terminus, the C-terminus, or both the N-terminus and C-terminus by one or more nuclear localization signals (NLSs).

In another method, Method 55, the present disclosure provides a method as provided in Method 54, wherein the one or more Cas9 endonucleases is flanked by two NLSs, one NLS located at the N-terminus and the second NLS located at the C-terminus.

In another method, Method 56, the present disclosure provides a method as provided in any one of Methods 54-55, wherein the one or more NLSs is a SV40 NLS.

In another method, Method 57, the present disclosure provides a method as provided in Method 53, wherein the weight ratio of sgRNA to Cas9 endonuclease in the RNP is 1:1.

In another method, Method 58, the present disclosure provides a method as provided in Method 53, wherein the one sgRNA comprises the nucleic acid sequence of SEQ ID NO: 71,959, the Cas9 endonuclease is a *S. pyogenes* Cas9 comprising a N-terminus SV40 NLS and a C-terminus SV40 NLS, wherein the weight ratio of sgRNA to Cas9 endonuclease is 1:1.

In another method, Method 59, the present disclosure provides a method as provided in any one of Methods 43-58, wherein the donor template is either single or double stranded.

In another method, Method 60, the present disclosure provides a method as provided in any one of Methods 42-59, wherein the modified transcriptional control sequence is located within a second intron of the BCL11A gene.

In another method, Method 61, as provided in any one of Methods 42-59, wherein the modified transcriptional control sequence is located within a +58 DNA hypersensitive site (DHS) of the BCL11A gene.

In another method, Method 62, the present disclosure provides a method as provided in any one of Methods 42-61, wherein the insertion is by homology directed repair (HDR).

In another method, Method 63, the present disclosure provides a method as provided in any one of Methods 8, 14, 21, 24, 43, and 45, wherein the SSB, DSB, or 5' locus and 3' locus are located within a second intron of the BCL11A gene.

In another method, Method 64, the present disclosure provides a method as provided in any one of Methods 8, 14, 21, 24, 43, and 45, wherein the SSB, DSB, or 5' DSB and 3' DSB are located within a +58 DNA hypersensitive site (DHS) of the BCL11A gene.

In another method, Method 65, the present disclosure provides a method as provided in any one of Methods 1, 8, 14, 21, or 24, wherein the method further comprises introducing into the cell one or more guide ribonucleic acid (gRNAs), and wherein the one or more DNA endonucleases is one or more Cas9 or Cpf1 endonucleases that effect or create a pair of single-strand breaks (SSBs) or double-strand breaks (DSBs), a first SSB or DSB at a 5' locus and a second SSB or DSB at a 3' locus, within or near the BCL11A gene or other DNA sequence that encodes a regulatory element of the BCL11A gene that causes a deletion of the chromosomal DNA between the 5' locus and the 3' locus that results in a permanent deletion, modulation, or inactivation of the transcriptional control sequence of the chromosomal DNA between the 5' locus and the 3' locus.

In another method, Method 66, the present disclosure provides a method as provided in Method 65, wherein one gRNA creates a pair of SSBs or DSBs.

In another method, Method 67, the present disclosure provides a method as provided in Method 65, wherein one gRNA comprises a spacer sequence that is complementary to either the 5' locus or the 3' locus.

In another method, Method 68, the present disclosure provides a method as provided in Method 65, wherein the method comprises a first guide RNA and a second guide RNA, wherein the first guide RNA comprises a spacer sequence that is complementary to a segment of the 5' locus and the second guide RNA comprises a spacer sequence that is complementary to a segment of the 3' locus.

In another method, Method 69, the present disclosure provides a method as provided in Methods 65-68, wherein the one or more gRNAs are one or more single-molecule guide RNA (sgRNAs).

In another method, Method 70, the present disclosure provides a method as provided in Methods 65-69 wherein the one or more gRNAs or one or more sgRNAs are one or more modified gRNAs or one or more modified sgRNAs.

In another method, Method 71, the present disclosure provides a method as provided in Method 70, wherein the one modified sgRNA comprises three 2'-O-methyl-phosphorothioate residues at or near each of its 5' and 3' ends.

In another method, Method 72, the present disclosure provides a method as provided in Method 71, wherein the one modified sgRNA is the nucleic acid sequence of SEQ ID NO: 71,959.

In another method, Method 73, the present disclosure provides a method as provided in any one of Methods 65-72, wherein the one or more Cas9 endonucleases is pre-complexed with one or more gRNAs or one or more sgRNAs to form one or more ribonucleoproteins (RNPs).

In another method, Method 74, the present disclosure provides a method as provided in Method 73, wherein the one or more Cas9 endonuclease is flanked at the N-terminus, the C-terminus, or both the N-terminus and C-terminus by one or more nuclear localization signals (NLSs).

In another method, Method 75, the present disclosure provides a method as provided in Method 74, wherein the one or more Cas9 endonucleases is flanked by two NLSs, one NLS located at the N-terminus and the second NLS located at the C-terminus.

In another method, Method 76, the present disclosure provides a method as provided in any one of Methods 74-75, wherein the one or more NLSs is a SV40 NLS.

In another method, Method 77, the present disclosure provides a method as provided in Method 73, wherein the weight ratio of sgRNA to Cas9 endonuclease in the RNP is 1:1.

In another method, Method 78, the present disclosure provides a method as provided in Method 73, wherein the one sgRNA comprises the nucleic acid sequence of SEQ ID NO: 71,959, the Cas9 endonuclease is a *S*. *pyogenes* Cas9 comprising a N-terminus SV40 NLS and a C-terminus SV40 NLS, wherein the weight ratio of sgRNA to Cas9 endonuclease is 1:1.

In another method, Method 79, the present disclosure provides a method as provided in any one of Methods 65-78, wherein both the 5' locus and 3' locus are located within a second intron of the BCL11A gene.

In another method, Method 80, the present disclosure provides a method as provided in any one of Methods 65-78, wherein both the 5' locus and 3' locus are located within a +58 DNA hypersensitive site (DHS) of the BCL11A gene.

In another method, Method 81, the present disclosure provides a method as provided in any one of Methods 1, 8, 14, 21, or 24-80 wherein the Cas9 or Cpf1 mRNA, gRNA, and donor template are either each formulated into separate lipid nanoparticles or all co-formulated into a lipid nanoparticle.

In another method, Method 82, the present disclosure provides a method as provided in any one of Methods 1, 8, 14, 21, or 24-80, wherein the Cas9 or Cpf1 mRNA is formulated into a lipid nanoparticle, and both the gRNA and donor template are delivered to the cell by an adeno-associated virus (AAV) vector.

In another method, Method 83, the present disclosure provides a method as provided in any one of Methods 1, 8, 14, 21, or 24-80, wherein the Cas9 or Cpf1 mRNA is formulated into a lipid nanoparticle, and the gRNA is delivered to the cell by electroporation and donor template is delivered to the cell by an adeno-associated virus (AAV) vector.

In another method, Method 84, the present disclosure provides a method as provided in any one of Methods 1, 8, 14, 21, or 24-80, wherein the one or more RNP is delivered to the cell by electroporation.

In another method, Method 85, the present disclosure provides a method as provided in any one of Methods 1-84, wherein the BCL11A gene is located on Chromosome 2: 60,451,167 -60,553,567 (Genome Reference Consortium - GRCh38).

In another method, Method 86, the present disclosure provides a method as provided in any one of Methods 1-85, wherein the hemoglobinopathy is selected from a group consisting of sickle cell anemia and thalassemia (α, β, δ, γ, and combinations thereof).

In another method, Method 87, the present disclosure provides a method as provided in any one of Methods 1-86, wherein the editing within or near a BCL11A gene or other DNA sequence that encodes a regulatory element of the BCL11A gene can reduce BCL11A gene expression.

In a first composition, Composition 1, the present disclosure provides one or more guide ribonucleic acids (gRNAs) for editing a BCL11A gene in a cell from a patient with a hemoglobinopathy, the one or more gRNAs comprising a spacer sequence selected from the group consisting of nucleic acid sequences in SEQ ID NOs: 1 - 71,947 of the Sequence Listing.

In another composition, Composition 2, the present disclosure provides the one or more gRNAs of Composition 1, wherein the one or more gRNAs are one or more single-molecule guide RNAs (sgRNAs).

In another composition, Composition 3, the present disclosure provides the one or more gRNAs or sgRNAs of Compositions 1 or 2, wherein the one or more gRNAs or one or more sgRNAs is one or more modified gRNAs or one or more modified sgRNAs.

In another composition, Composition 4, the present disclosure provides the one or more sgRNAs of Composition 3, wherein the one or more modified sgRNAs comprises three 2'-O-methyl-phosphorothioate residues at or near each of its 5' and 3' ends.

In another composition, Composition 5, the present disclosure provides the one or more sgRNAs of Composition 3, wherein the one or more modified sgRNAs comprises the nucleic acid sequence of SEQ ID NO: 71,959.

In another composition, Composition 6, the present disclosure provides a single-molecule guide RNA (sgRNA) comprising the nucleic acid sequence of SEQ ID NO: 71,959.

### Definitions

The term "comprising" or "comprises" is used in reference to compositions, methods, and respective component(s) thereof, that are essential to the invention, yet open to the inclusion of unspecified elements, whether essential or not.

The term "consisting essentially of" refers to those elements required for a given aspect. The term permits the presence of additional elements that do not materially affect the basic and novel or functional characteristic(s) of that aspect of the invention.

The term "consisting of" refers to compositions, methods, and respective components thereof as described herein, which are exclusive of any element not recited in that description of the aspect.

The singular forms "a," "an," and "the" include plural references, unless the context clearly dictates otherwise.

Any numerical range recited in this specification describes all sub-ranges of the same numerical precision (i.e., having the same number of specified digits) subsumed within the recited range. For example, a recited range of "1.0 to 10.0" describes all sub-ranges between (and including) the recited minimum value of 1.0 and the recited maximum value of 10.0, such as, for example, "2.4 to 7.6," even if the range of "2.4 to 7.6" is not expressly recited in the text of the specification. Accordingly, the Applicant reserves the right to amend this specification, including the claims, to expressly recite any sub-range of the same numerical precision subsumed within the ranges expressly recited in this specification. All such ranges are inherently described in this specification such that amending to expressly recite any such sub-ranges will comply with written description, sufficiency of description, and added matter requirements, including the requirements under 35 U.S.C. § 112(a) and Article 123(2) EPC. Also, unless expressly specified or otherwise required by context, all numerical parameters described in this specification (such as those expressing values, ranges, amounts, percentages, and the like) may be read as if prefaced by the word "about," even if the word "about" does not expressly appear before a number. Additionally, numerical parameters described in this specification should be construed in light of the number of reported significant digits, numerical precision, and by applying ordinary rounding techniques. It is also understood that numerical parameters described in this specification will necessarily possess the inherent variability characteristic of the underlying measurement techniques used to determine the numerical value of the parameter.

### Examples

The invention will be more fully understood by reference to the following examples, which provide illustrative non-limiting aspects of the invention.

The examples describe the use of the CRISPR system as an illustrative genome editing technique to create defined genomic deletions, insertions, or replacements, termed "genomic modifications" herein, within or near the BCL11A gene or other DNA sequence that encodes a regulatory element of the BCL11A gene that lead to a permanent deletion, modulation, or inactivation of a transcriptional control sequence of the BCL11A gene. Introduction of the defined therapeutic modifications represents a novel therapeutic strategy for the potential amelioration of a hemoglobinopathy, as described and illustrated herein.

### Example 1 - CRISPR/SpCas9 target sites for the transcriptional control sequence of the BCL11A gene

Regions of the 12.4 kb transcriptional control sequence of the BCL11A gene were scanned for target sites. Each area was scanned for a protospacer adjacent motif (PAM) having the sequence NRG. gRNA 20 bp spacer sequences corresponding to the PAM were identified, as shown in SEQ ID NOs: 1 - 29,482 of the Sequence Listing.

### Example 2 - CRISPR/SaCas9 target sites for the transcriptional control sequence of the BCL11A gene

Regions of the 12.4 kb transcriptional control sequence of the BCL11A gene were scanned for target sites. Each area was scanned for a protospacer adjacent motif (PAM) having the sequence NNGRRT. gRNA 20 bp spacer sequences corresponding to the PAM were identified, as shown in SEQ ID NOs: 29,483 - 32,387 of the Sequence Listing.

### Example 3 - CRISPR/StCas9 target sites for the transcriptional control sequence of the BCL11A gene

Regions of the 12.4 kb transcriptional control sequence of the BCL11A gene were scanned for target sites. Each area was scanned for a protospacer adjacent motif (PAM) having the sequence NNAGAAW. gRNA 20 bp spacer sequences corresponding to the PAM were identified, as shown in SEQ ID NOs: 32,388 - 33,420 of the Sequence Listing.

### Example 4 - CRISPR/TdCas9 target sites for the transcriptional control sequence of the BCL11A gene

Regions of the 12.4 kb transcriptional control sequence of the BCL11A gene were scanned for target sites. Each area was scanned for a protospacer adjacent motif (PAM) having the sequence NAAAAC. gRNA 20 bp spacer sequences corresponding to the PAM were identified, as shown in SEQ ID NOs: 33,421 - 33,851 of the Sequence Listing.

### Example 5 - CRISPR/NmCas9 target sites for the transcriptional control sequence of the BCL11A gene

Regions of the 12.4 kb transcriptional control sequence of the BCL11A gene were scanned for target sites. Each area was scanned for a protospacer adjacent motif (PAM) having the sequence NNNNGHTT. gRNA 20 bp spacer sequences corresponding to the PAM were identified, as shown in SEQ ID NOs: 33,852 - 36,731 of the Sequence Listing.

### Example 6 - CRISPR/Cpf1 target sites for the transcriptional control sequence of the BCL11A gene

Regions of the 12.4 kb transcriptional control sequence of the BCL11A gene were scanned for target sites. Each area was scanned for a protospacer adjacent motif (PAM) having the sequence YTN. gRNA 22 bp spacer sequences corresponding to the PAM were identified, as shown in SEQ ID NOs: 36,732 - 71,947 of the Sequence Listing.

### Example 7 - Bioinformatics analysis of the guide strands

Candidate guides will be screened and selected in a multi-step process that involves both theoretical binding and experimentally assessed activity. By way of illustration, candidate guides having sequences that match a particular on-target site, such as a site within the transcriptional control sequence of the BCL11A gene, with adjacent PAM can be assessed for their potential to cleave at off-target sites having similar sequences, using one or more of a variety of bioinformatics tools available for assessing off-target binding, as described and illustrated in more detail below, in order to assess the likelihood of effects at chromosomal positions other than those intended. Candidates predicted to have relatively lower potential for off-target activity can then be assessed experimentally to measure their on-target activity, and then off-target activities at various sites. Preferred guides have sufficiently high on-target activity to achieve desired levels of gene editing at the selected locus, and relatively lower off-target activity to reduce the likelihood of alterations at other chromosomal loci. The ratio of on-target to off-target activity is often referred to as the "specificity" of a guide.

For initial screening of predicted off-target activities, there are a number of bioinformatics tools known and publicly available that can be used to predict the most likely off-target sites; and since binding to target sites in the CRISPR/Cas9/Cpf1 nuclease system is driven by Watson-Crick base pairing between complementary sequences, the degree of dissimilarity (and therefore reduced potential for off-target binding) is essentially related to primary sequence differences: mismatches and bulges, i.e. bases that are changed to a non-complementary base, and insertions or deletions of bases in the potential off-target site relative to the target site. An exemplary bioinformatics tool called COSMID (CRISPR Off-target Sites with Mismatches, Insertions and Deletions) (available on the web at crispr.bme.gatech.edu) compiles such similarities. Other bioinformatics tools include, but are not limited to, GUIDO, autoCOSMID, and CCtop.

Bioinformatics were used to minimize off-target cleavage in order to reduce the detrimental effects of mutations and chromosomal rearrangements. Studies on CRISPR /Cas9 systems suggested the possibility of high off-target activity due to nonspecific hybridization of the guide strand to DNA sequences with base pair mismatches and/or bulges, particularly at positions distal from the PAM region. Therefore, it is important to have a bioinformatics tool that can identify potential off-target sites that have insertions and/or deletions between the RNA guide strand and genomic sequences, in addition to base-pair mismatches. The bioinformatics-based tool, COSMID (CRISPR Off-target Sites with Mismatches, Insertions and Deletions) was therefore used to search genomes for potential CRISPR off-target sites (available on the web at crispr.bme.gatech.edu). COSMID output ranked lists of the potential off-target sites based on the number and location of mismatches, allowing more informed choice of target sites, and avoiding the use of sites with more likely off-target cleavage.

Additional bioinformatics pipelines were employed that weigh the estimated on- and/or off-target activity of gRNA targeting sites in a region. Other features that may be used to predict activity include information about the cell type in question, DNA accessibility, chromatin state, transcription factor binding sites, transcription factor binding data, and other CHIP-seq data. Additional factors are weighed that predict editing efficiency, such as relative positions and directions of pairs of gRNAs, local sequence features and micro-homologies.

### Example 8 - Testing of preferred guides in cells for on-target activity

The gRNAs predicted to have the lowest off-target activity will then be tested for on-target activity in K562 cells, and evaluated for indel frequency using TIDE.

TIDE is a web tool to rapidly assess genome editing by CRISPR-Cas9 of a target locus determined by a guide RNA (gRNA or sgRNA). Based on quantitative sequence trace data from two standard capillary sequencing reactions, the TIDE software quantifies the editing efficacy and identifies the predominant types of insertions and deletions (indels) in the DNA of a targeted cell pool. See Brinkman *et al,* Nucl. Acids Res. (2014) for a detailed explanation and examples. An alternative method is Next-generation sequencing (NGS), also known as high-throughput sequencing, which is the catch-all term used to describe a number of different modern sequencing technologies including: Illumina (Solexa) sequencing, Roche 454 sequencing, Ion torrent: Proton/PGM sequencing, and SOLiD sequencing. These recent technologies allow one to sequence DNA and RNA much more quickly and cheaply than the previously used Sanger sequencing, and as such have revolutionized the study of genomics and molecular biology.

Transfection of tissue culture cells, allows screening of different constructs and a robust means of testing activity and specificity. Tissue culture cell lines, such as K562 or HEK293T are easily transfected and result in high activity. These or other cell lines will be evaluated to determine the cell lines that match with CD34+ and provide the best surrogate. These cells will then be used for many early stage tests. For example, individual gRNAs for *S. pyogenes* Cas9 can be transfected into the cells using plasmids, such as, for example, CTx-1, CTx-2, or CTx-3 described in Figure 1A-1C, which are suitable for expression in human cells. Alternatively, commercially available vectors may also be used. For the Indel Freq assessment of the BCL11A gRNAs described herein, a commercially available Cas9 expression plasmid (GeneArt, Thermo Fisher) was employed. Several days later (48 hrs for this experiment), the genomic DNA was harvested and the target site amplified by PCR. The cutting activity was measured by the rate of insertions, deletions and mutations introduced by NHEJ repair of the free DNA ends. Although this method cannot differentiate correctly repaired sequences from uncleaved DNA, the level of cutting can be gauged by the amount of mis-repair. Off-target activity can be observed by amplifying identified putative off-target sites and using similar methods to detect cleavage. Translocation can also be assayed using primers flanking cut sites, to determine if specific cutting and translocations happen. Un-guided assays have been developed allowing complementary testing of off-target cleavage including guide-seq. The gRNA or pairs of gRNA with significant activity can then be followed up in cultured cells to measure the modulation or inactivation of the +58 DNA hypersensitive site (DHS) within the transcriptional control sequence of the BCL11A gene. Off-target events can be followed again. Similarly CD34+ cells can be transfected and the level of modulation or inactivation of the +58 DNA hypersensitive site (DHS) within the transcriptional control sequence of the BCL11A gene and possible off-target events measured. These experiments allow optimization of nuclease and donor design and delivery.

### Example 9 - Testing of preferred guides in cells for off-target activity

The gRNAs having the best on-target activity from the TIDE and next generation sequencing studies in the above example will then be tested for off-target activity using whole genome sequencing. Candidate gRNAs will be more completely evaluated in CD34+ cells or iPSCs.

### Example 10 - Testing of preferred gRNA combinations in cells

The gRNAs having the best on-target activity from the TIDE and next generation sequencing studies and lowest off-target activity will be tested in combinations to evaluate the size of the deletion resulting from the use of each gRNA combination. Potential gRNA combinations will be evaluated in primary human CD34+ cells.

For example, gRNA combinations will be tested for efficiency of deleting all or a portion of the transcriptional control sequence of the BCL1 1A gene. The gRNA combinations will also be tested for efficiency of deleting all or a portion of the +58 DNA hypersensitive site (DHS) of the BCL11A gene.

### Example 11 - Testing different approaches for HDR gene editing

After testing the gRNAs for both on-target activity and off-target activity, modulation/inactivation and knock-in strategies will be tested for HDR gene editing.

For the modulation/inactivation approach, donor DNA template will be provided as a short single-stranded oligonucleotide, a short double-stranded oligonucleotide (PAM sequence intact/PAM sequence mutated), a long single-stranded DNA molecule (PAM sequence intact/PAM sequence mutated) or a long double-stranded DNA molecule (PAM sequence intact/PAM sequence mutated). The donor DNA template will comprise either a wild-type BCL11A gene or cDNA comprising a modified transcriptional control sequence or a wild-type BCL11A gene or cDNA comprising a modified (e.g. mutated) +58 DNA hypersensitive site (DHS). In addition, the donor DNA template will be delivered by AAV.

For the cDNA knock-in approach, a single-stranded or double-stranded DNA may include more than 40 nt of the modified transcriptional control sequence of the BCL1 1A gene. The single-stranded or double-stranded DNA may include more than 80 nt of the modified transcriptional control sequence of the BCL1 1A gene. The single-stranded or double-stranded DNA may include more than 100 nt of the modified transcriptional control sequence of the BCL11A gene. The single-stranded or double-stranded DNA may include more than 150 nt of the modified transcriptional control sequence of the BCL11A gene. The single-stranded or double-stranded DNA may include more than 300 nt of the modified transcriptional control sequence of the BCL1 1A gene. The single-stranded or double-stranded DNA may include more than 400 nt of the modified transcriptional control sequence of the BCL11A gene. Alternatively, the DNA template will be delivered by AAV.

For the cDNA knock-in approach, a single-stranded or double-stranded DNA may include more than 40 nt of the modified +58 DNA hypersensitive site (DHS) of the BCL1 1A gene. The single-stranded or double-stranded DNA may include more than 80 nt of the modified +58 DNA hypersensitive site (DHS) of the BCL11A gene. The single-stranded or double-stranded DNA may include more than 100 nt of the modified +58 DNA hypersensitive site (DHS) of the BCL11A gene. The single-stranded or double-stranded DNA may include more than 150 nt of the modified +58 DNA hypersensitive site (DHS) of the BCL11A gene. The single-stranded or double-stranded DNA may include more than 300 nt of the modified +58 DNA hypersensitive site (DHS) of the BCL1 1A gene. The single-stranded or double-stranded DNA may include more than 400 nt of the modified +58 DNA hypersensitive site (DHS) of the BCL11A gene. Alternatively, the DNA template will be delivered by AAV.

### Example 12 - Re-assessment of lead CRISPR-Cas9/DNA donor combinations

After testing the different strategies for HDR gene editing, the lead CRISPR-Cas9/DNA donor combinations will be re-assessed in primary human cells for efficiency of deletion, recombination, and off-target specificity. Cas9 mRNA or RNP will be formulated into lipid nanoparticles for delivery, sgRNAs will be formulated into nanoparticles or delivered as AAV, and donor DNA will be formulated into nanoparticles or delivered as AAV.

### Example 13 - In vivo testing in relevant animal model

After the CRISPR-Cas9/DNA donor combinations have been re-assessed, the lead formulations will be tested *in vivo* in an animal model.

Culture in human cells allows direct testing on the human target and the background human genome, as described above.

Preclinical efficacy and safety evaluations can be observed through engraftment of modified mouse or human CD34+ cells in NSG or similar mice. The modified cells can be observed in the months after engraftment.

### Example 14- Editing Cells with various gRNAs

Mobilized human peripheral blood CD34+ cells from human donors 1-3 were cultured in serum free StemSpan Medium with CD34+ expansion supplement for two days. 100,000 cells were washed and electroporated using Cas9 mRNA with Corfu Large (CLO) gRNAs, Corfu Small (CSO) gRNAs, HPFH5 gRNAs, Kenya gRNAs, SD2 sgRNA, or SPY101 sgRNA. Cells were allowed to recover for two days before being switched to an erythroid differentiation medium (IMDM+Glutamax supplemented with 5% human serum, 10ug/ml insulin, 20ng/ml SCF, 5ng/ml IL-3, 3U/ml EPO, 1uM dexamethasone, 1uM β-estradiol, 330ug/ml holo-transferrin and 2U/ml heraprin). The percentage of insertions/deletions ("indels") was determined for each of the cells electroporated with Corfu Large (CLO) gRNAs, cells electroporated with Corfu Small (CSO) gRNAs, cells electroporated with HPFH5 gRNAs, cells electroporated with Kenya gRNAs, cells electroporated with SD2 sgRNA, and the cells electroporated with SPY101 sgRNA (Figure 3), as described in the "On- and off-target mutation detection by sequence" and "Mutation detection assays" sections described herein. After differentiating these cells for 12 days in erythroid differentiation medium, RNA was collected to assess hemoglobin levels by quantitative real-time-PCR (Figures 4A-4C).

Single erythroid progenitors were generated using flow cytometry one day later and cultured in the erythroid differentiation medium to expand and grow as colonies. Each colony was split and collected 12 days post-sorting for DNA and RNA analysis. The sister colonies were collected 15 days post-sorting for the analysis of hemoglobin proteins. Globin expression (ratio of y/18sRNA or ratio of γ/α) was determined by quantitative real-time PCR and compared for each of the edited erythroid colonies (Figures 5A-5B).

### Example 15 - Testing of SPY101 sgRNA

Three possible gene editing outcomes may occur within intron 2 of the BCL11A gene when using SPY101 sgRNA. The first gene editing outcome that may occur when using SPY101 sgRNA results in only indels in both alleles (Indel/Indel, Figure 6). The second gene editing outcome that may occur when using SPY101 sgRNA results in a clone with both indels and wild-type sequences in the two alleles (Indel/WT, Figure 6). The third gene editing outcome that may occur when using SPY101 sgRNA results in a colony with wild-type sequences in both alleles (WT/WT, Figure 6).

When using SPY101 sgRNA, 92% of the erythroid colonies were edited. For example, 92% of the erythroid colonies had alleles with indels (Figure 6).

γ-globin expression (γ/α globin mRNA ratio or γ/(γ+β) globin mRNA ratio) was measured in single erythroid colonies edited with SPY101 (Figures 7A-B). The single erythroid colonies included colonies with biallelic or homozygous indel (indel/indel), colonies with a monoallelic or heterozygous indel (indel/WT), and colonies with wild-type sequences in both allelles (WT/WT). The erythroid colonies having indels were able to express higher levels of gamma globin compared to the clones with wild-type sequences in both alleles (Figures 7A-B).

### Example 16 - Therapeutic Strategy for Sickle Cell Disease (SCD) and β-thalassemia

The following Table (Table 4) provides information related to the gRNAs used in Examples 16-17.

**Table 4**

| **gRNA** | **Name** | **Sequence** | **SEQ ID NO**. |
|---|---|---|---|
| gRNA A | CL01 | | 71950 |
| | | UAAGGCUAGUCCGUUAUCAACUUGAA AAAGUGGCACCGAGUCGGUGCususus U3' | |
| gRNA B | CL08 | | 71951 |
| gRNA C | CS02 | | 71952 |
| gRNA D | CS06 | | 71953 |
| gRNA E | HPFH5-15 | 5'csusgsUCUUAUUACCCUGUCAUGUUU UAGAGCUAGAAAUAGCAAGUUAAAAU AAGGCUAGUCCGUUAUCAACUUGAAA AAGUGGCACCGAGUCGGUGCusususU 3' | 71954 |
| gRNA F | HPFH5-4 | 5'ascsusGAGUUCUAAAUCAUCGGUUU UAGAGCUAGAAAUAGCAAGUUAAAAU AAGGCUAGUCCGUUAUCAACUUGAAA AAGUGGCACCGAGUCGGUGCusususU 3' | 71955 |
| gRNA G | Kenya 02 | | 71956 |
| gRNA H | Kenya 17 | | 71957 |
| gRNA I | SD2 | 5'csususGUCAAGGCUAUUGGUCAGUU UUAGAGCUAGAAAUAGCAAGUUAAAA UAAGGCUAGUCCGUUAUCAACUUGAA AAAGUGGCACCGAGUCGGUGCususus U3' | 71958 |
| gRNA J | SPY | 5'csusasACAGUUGCUUUUAUCACGUUU UAGAGCUAGAAAUAGCAAGUUAAAAU AAGGCUAGUCCGUUAUCAACUUGAAA AAGUGGCACCGAGUCGGUGCusususU 3' | 71959 |

| | | | |
|---|---|---|---|
| a, g, u: 2'-O-methyl residues s: phosphorothioate A, C, G, U: RNA residues | | | |

The following Table (Table 5) provides information related to the targets referred to in Examples 16-17.

**Table 5**

| | |
|---|---|
| Target 1 | Corfu Large |
| Target 2 | Corfu Small |
| Target 3 | HPFH5 |
| Target 4 | KENYA |
| Target 5 | SD2 |
| Target 6 | SPY101 |

A therapeutic strategy for SCD and β-thalassemia used CRISPR/Cas9 to re-create the same genetic mutations that occur naturally in HPFH patients. Patients' hematopoietic stem cells were isolated, these cells were treated *ex vivo* with CRISPR/Cas9 to create HPFH genetic edits, and then the edited cells were reintroduced into the patients. The genetically modified stem cells gave rise to erythrocytes that contain sufficient levels of HBF to significantly reduce the severity of disease symptoms. A number of genetic edits have been prioritized based on the degree of HBF upregulation seen in nature, the ability to re-create theses edits at high efficiency using CRISPR/Cas9, and the absence of off target editing.

Candidate guide RNA (gRNA) sequences were computationally selected and then screened for on-target editing efficacy in CD34+ cells. Shown in Figure 8 are the results of one such screen. gRNAs were identified with consistent, high (>70%) on target editing across multiple donor samples. Each CD34+ cell donor is represented by a unique symbol (▲, _{★}, ●) and on-target editing efficiency for each donor is measured twice.

Candidate gRNAs were screened in CD34+ cells for off-target activity by examining hundreds of sites computationally identified to be most similar in sequence to the intended on-target site, and thus have the highest potential for off-target activity. Figures 9A-B shows the experimental approach (Figure 9A) and results (Figure 9B) for each of the gRNAs tested in Figure 8. Most gRNAs displayed no detectable off-target activity, even at predicted sites. Only gRNA C and gRNA G show off-target activity. Multiple probes were used for each predicted site to increase assay sensitivity.

Candidate gRNAs were used to re-create specific HPFH or other modifications in erythroid cells obtained from SCD and β-thalassemia patients, as well as from healthy donors. After erythroid differentiation, globin transcript levels were measured to assess the increase in γ-globin relative to α- or β-globin. Shown in Figures 10A-B, greater than 30% γ-globin mRNA levels were observed in patient cells edited with gRNAs to re-create HPFH Target 5 and 6. SCD and β-thalassemia patient samples exhibited a larger absolute increase in γ-globin than those from healthy donors, consistent with the observation of higher HbF in patients than in heterozygote carriers with HPFH. The background level for mock treated cells from each donor was subtracted from the values shown. Data represent a single experiment, except for SCD patient data which represent the mean of 3 different donor samples. Editing efficiency was similar for all experiments.

To ensure that editing efficiencies in the bulk CD34+ population were representative of those in long-term repopulating HSCs (LT-HSC), bulk CD34+ cells were sorted into specific sub-populations and assayed for on-target editing efficiency as shown in Figures 11A-C. High editing efficiency in the LT-HSC population was observed. Experiments were done using SPY101 and Cas9 protein across 4 donors. Bars depict Mean ± SEM. LT-*HSC*, Long Term Hematopoietic Stem Cell; *MPP,* Multipotent Progenitor; *MLP,* Multilymphoid Progenitor; *CMP,* Common Myeloid Progenitor; *MEP,* Megakaryocyte Erythrocyte Progenitor; *GMP,* Granulocyte Macrophage Progenitor.

*In vivo* engraftment studies were performed in immunocompromised mice to confirm that the gene-edited HSPCs retain the potential for long-term repopulation of the hematopoietic system. Human CD34+ cells from healthy donors were untreated, unedited, or gene-edited using SPY101 gRNA and introduced into NSG mice. As shown in Figure 12, the presence of similar levels of hCD45RA+ cells (at 8-weeks post-engraftment) in mice injected with untreated/unedited HSPCs and mice injected with SPY101 gene-edited HSPCs confirmed that the SPY101 edited cells retained engraftment potential. Data points represent individual animals and depict the percentage of live cells that were human CD45RA+. Mean ± SD. "Untreated" represents HSPCs that were not electroporated and injected into immunocompromised mice. "Unedited" represents HSPCs that were electroporated, but not gene-edited and injected into immunocompromised mice. "SPY101" represents HSPCs that were electroporated with Cas9 and SPY101 gRNA and injected into immunocompromised mice.

Process development was initiated at a GMP-capable facility in preparation for clinical studies. As shown in Figure 13, no significant loss of gene editing efficacy was observed at clinical scale in a GMP-compatible process. Data was average across 4 or more experiments, ± SD.

GLP/toxicology studies have been initiated for our lead candidates, as shown in Figure 14. Two separate studies in NSG mice will allow for a comprehensive characterization of biodistribution and toxicology of edited CD34+ cells.

### Example 17 - Therapeutic Strategy for Sickle Cell Disease (SCD) and β-thalassemia

Results from recreation of six different HPFH variants, or editing "targets", in human mPB CD34+ cells are shown in Figures 16A-B and Figure 17. The CD34+ cells were treated with CRISPR/Cas9, differentiated into erythrocytes, and then assayed for HBF mRNA and protein expression in bulk (Figures 16A-B) and colonies (Figure 17), using an experimental process demonstrated in Figure 15.

The results presented in Figures 16A-B were from 3 different donors for targets 1-3, and 7 different donors for targets 4-6. The background level for mock treated cells had been subtracted. Data is mean ± SEM. Bulk analysis confirmed HBF upregulation and allowed for the prioritization of targets that demonstrated the highest levels of HBF.

Clonal analysis presented in Figure 17 allowed confirmation that genetic edits caused by CRISPR/Cas9 were indeed the cause of the increase in HBF at the individual cell level. Results were from a single donor, and 50-80 colonies per target. mRNA transcript levels were measured by qRT-PCR. Data is mean ± SEM.

Targets 5 and 6 displayed the highest HBF levels and were further analyzed in Figures 18A-B. Data is mean ± SEM. WT denotes colonies that do not show evidence of gene editing, Heterozygous or Het denotes colonies with one allele edited, and Homozygous or Homo denotes colonies with both alleles edited. The evidence in Figures 16A-B, 17, and 18A-B support the causal relationship between the genetic edits produced, and the desired upregulation of HBF, providing further validation for the proposed therapeutic strategy.

### Example 18 - Testing of preferred guide RNAs in cells for on-targeting activity

Mobilized human peripheral blood (mPB) CD34+ cells from four independent donors were cultured in serum free CellGro^{®} media including 100 ng/ml recombinant human stem cell factor (SCF), 100 ng/ml recombinant human Fit 3-Ligand (FLT3L), and 100 ng/ml Thrombopoietin (TPO). 200,000 cells per donor were washed and electroporated using Lonza electroporator without any CRISPR/Cas9 editing components (mock electroporation sample), with GFP gRNA and Cas9 protein as a negative control (GFP), with SPY101 gRNA and Cas9 protein (SPY), with SD2 gRNA and Cas9 protein (SD2), or dual BCL11A Exon 2 gRNAs and Cas9 protein (Ex2). The recombinant Cas9 protein encodes for *S. pyogenes* Cas9 flanked by two SV40 nuclear localization sequences (NLSs). These experiments were performed using a ribonucleoprotein (RNP) 1:1 weight ratio of gRNA to Cas9. The SPY101 gRNA creates an InDel disruption of DHS+58 Gata1 binding site in intron 2 of the BCL11a locus. The SD2 gRNA creates InDels and a 4.9 Kb deletion in the human beta globin locus. The 4.9 Kb deletion is located upstream of HBG1 and includes the entire HBG2 sequence. The 4.9 Kb deletion starts 168 bp 5' to the HBG2 coding sequence and ends 168 bp 5' to the HBG1 coding sequence. The Exon 2 gRNAs create a 196bp deletion on Exon 2 of the BCL11A locus and served as a positive control. Human mPB CD34+ cells that were not electroporated served as a negative control (no EP).

After electroporation, the gene-edited mPB CD34+ cells were allowed to recover for two days before being switched to an erythroid differentiation medium (IMDM+L-glutamine supplemented with 5% human serum, 10 ug/mL insulin, 20 ng/mL SCF, 5 ng/mL IL-3, 3 U/mL EPO, 1uM dexamethasone, 330ug/ml holo-transferrin and 2 U/mL heparin). The gene-edited mPB CD34+ cells were differentiated into erythrocytes and further tested via TIDE analysis, ddPCR analysis, quantitative real-time PCR analysis, FACS, and LC-MS (Figures 20A-B, 21A-D, 22A-B, and 23A-D). The overall experimental process is demonstrated in Figure 19.

### TIDE analysis / ddPCR analysis

Genomic DNA was isolated and tested for each of the gene-edited human mPB CD34+ cell samples grown in differentiation medium. Genomic DNA was isolated from the cells on days 1, 11, 13 and 15 post-differentiation. The genomic DNA was analyzed via TIDE analysis, which is a web tool to rapidly assess genome editing by CRISPR-Cas9 of a target locus determined by a guide RNA (gRNA or sgRNA). The results presented in Figures 20A-B were from 4 different donors and demonstrated that the percentage of gene editing was maintained throughout ex-vivo erythroid differentiation of mPB CD34+ cells edited with SD2 gRNA (Figure 20B) and mPB CD34+ cells edited with SPY101 gRNA (Figure 20A). Data is mean+SD.

The genomic DNA was also analyzed via ddPCR analysis to detect 4.9kb deletion frequency with SD2 treatment. The results presented in Figure 20B were from 4 different donors and demonstrated that the percentage of gene editing was maintained throughout ex-vivo erythroid differentiation of mPB CD34+ cells edited with SD2 gRNA (Figure 20B). Data is mean+SD.

### Quantitative Real-time PCR analysis

mRNA was isolated and tested for each of the gene-edited human mPB CD34+ cell samples grown in differentiation medium. mRNA isolation was performed on days 11 and 15 post-differentiation. Globin expression (ratio of γ/α and ratio of γ/(γ+β)) was determined by quantitative real-time PCR and compared for each of the human mPB CD34+ cells edited with SD2 gRNA and human mPB CD34+ cells edited with SPY101 gRNA (Figures 21A-D). The results presented in Figures 21A-D were from 4 different donors and demonstrated an increase in γ-globin transcript in human mPB CD34+ cells edited with SD2 gRNA and human mPB CD34+ cells edited with SPY101 gRNA compared to negative control. Data is mean+SD.

### FACS / LC-MS

Human mPB CD34+ cells edited with SD2 gRNA and human mPB CD34+ cells edited with SPY101 gRNA were grown in differentiation medium for 15 days. Human mPB CD34+ cells were also edited with dual BCL11A Exon 2 gRNAs (Ex2) or GFP gRNA and grown in differentiation medium for 15 days. Some human mPB CD34+ cells were not edited with any CRISPR/Cas9 editing components (mock electroporation sample) and some human mPB CD34+ cells were not electroporated (no EP). The live cells were stained with Glycophorin A, a erythroid maturation marker. The cells were then fixed and permeabilized. The fixed cells were stained with fluorophore-conjugated antibody for each globin subunit. The stained cells were then analyzed via FACS, an example of γ-globin represented in Figure 22A. The average median fluorescent intensity for γ-globin from 4 different donors are depicted in Figure 22B (mean±SEM) and demonstrated an upregulation in γ-globin in human mPB CD34+ cells edited with SD2 gRNA and human mPB CD34+ cells edited with SPY101 gRNA.

Human mPB CD34+ cells edited with SD2 gRNA and mPB CD34+ cells edited with SPY101 gRNA were grown in differentiation medium for 15 days. Human mPB CD34+ cells were also edited with dual BCL11A Exon 2 gRNAs (Ex2) or GFP gRNA and grown in differentiation medium for 15 days. Some human mPB CD34+ cells were not edited with any CRISPR/Cas9 editing components (mock electroporation sample) and some human mPB CD34+ cells were not electroporated (no EP). Liquid chromatography - mass spectrometry (LC-MS) was used to detect denatured globin monomers (Figures 23A-D). The results presented in Figures 23A-D were from 4 different donors and also demonstrated an upregulation in γ-globin in mPB CD34+ cells edited with SD2 gRNA and mPB CD34+ cells edited with SPY101 gRNA. Data is mean+SD.

### Example 19 - Testing of preferred guide RNAs in cells for off-targeting activity

While on-target editing of the genome is fundamental to a successful therapy, the detection of any off-target editing events is an important component of ensuring product safety. One method for detecting modifications at off-target sites involves enriching for regions of the genome that are most similar to the on-target site via hybrid capture sequencing and quantifying any indels that are detected.

Hybrid capture sequencing is a method that quantifies off-target edits in CRISPR-Cas9 edited cells and DNA. Details related to the hybrid capture sequencing method are as follows:

### MATERIALS AND METHODS

### Materials and Sources

### 1.1.1. Genomic DNA

As the purpose of this method is to determine if editing by CRISPR-Cas9 has occurred at off-target sites in the genome at least two input samples are typically used - treated and control (untreated, mock electroporated, etc.) samples. Each sample has genomic DNA (gDNA) extracted by an appropriated method and that gDNA is hybridized with the hybrid capture libraries (1.1.2) followed by the remainder of the protocol as described below.

### 1.1.2. Hybrid capture libraries

Hybrid capture libraries as described in (1.2.2) are generated by providing a list of up to 57,000 120-mer oligonucleotide bait sequences which are then synthesized as a custom SureSelect XT hybrid capture kit.

### 1.2 Methods

### 1.2.1. Off-target site detection algorithms

To determine the sites that are most likely to have off-target editing we use several algorithms with different features to ensure a wide-range of off-target sites were covered.

### 1.1.1.1. CCTop

For a given guide sequence CCTop uses the Bowtie 1 sequence mapping algorithm to search the genome for off-target sites with up to 5 mismatch between the site and the guide. We refer to these site as "homologous off-target sites" (rather than "predicted off-target sites") since only sequence homology is used to determine the potential off-target sites in the genome. These 5 mismatches are limited to no more than 2 mismatches in the 5 base alignment seed region closest to the PAM end of the sequences. The CRISPOR algorithm (1.2.1.2) does not have the limitation in the seed region and thus complements CCTop.

### 1.2.1.1. COSMID

Since some off-target Cas9 cleavage sites may have a short indels (also referred to as bulges) between themselves and the guide, we also search with the COSMID algorithm that can detect off-target sites with indels (typically limited to up to 2 indels) and thus complements the search done with CCTop.

### 1.2.1.2. CRISPOR

CRISPOR is a tool that implements many different published CRISPR on- and off-target scoring functions for the purpose of comparing various methods. It uses the BWA algorithm for searching guide sequences against the genome to find their off-target sites. This differs from Bowtie 1 algorithm used in CCTop and allows for a search that is slightly more permissive in that mismatches near the PAM region are not limited to 2 out of 5 bases as in CCTop.

### 1.2.1.3. PAMs

By default, screens are done with a search for guides with an NGG or NAG PAMs as they have some of the greatest activity. Later stage screens may include more PAMs to ensure that no off-target sites, even those with very low activity, are missed.

### 1.2.1.4. Combination of algorithms

The guides output by each algorithm are joined together to eliminate identical off-target sites and fed into the hybrid capture bait design component.

### 1.2.2. Hybrid capture baits

### 1.2.2.1 Design

The list of sites produced by the off-target site detection algorithms (1.2.1.) are then used to generate hybrid capture probes that will enrich for each of the off-target sites in the input gDNA samples. Although one bait may be sufficient to successfully enrich for a target DNA sequence, several baits are generally designed and tiled across the target site (Figure 24) in order to make it more likely that a bait specifically pulls down a target region even if it is flanked on a side by repetitive sequence that may be difficult to bind specifically. Hybrid capture baits (120-mers, dark colored portions) tiled across a bait (20-mer, light portion denoted by the *) (Figure 24).

### 1.2.3. Sequencing

After hybrid capture enrichment, sequencing is done on an Illumina HiSeq sequencer with paired-end 125 bp reads and a 175 bp insert size. Sequencing is typically done to target a depth of coverage that targets having 5 reads detected from a minimal frequency event. To detect for example 0.5% indel events, sequencing to 1000x coverage is performed so that an 0.5% event might have 5 reads.

### 1.2.4. Bait effectiveness

In a typical experiment we find that baits cover the large majority of the target sites with high levels of sequencing coverage. There are some limitations to the sequencing coverage that may be achieved by next-generation sequencing (NGS) methods due to: high or low %GC, low-complexity sequences, low bait affinity, bait non-specificity, and other reasons. The actual power to detect indels in an experiment is estimated by calculating the sampling power of different sequencing coverage for sites with different true indel frequencies. Generally, increased sequence coverage provides increased power to detect sites with low-frequency indels. For example, if a site has 2500x sequencing coverage, hybrid capture will have 99% power to see sites with 0.4% indel frequency, and 94% power to see sites with 0.3% indel frequency (Figure 25).

### 1.2.5. Quantification

Sequencing data is aligned with the BWA algorithm using default parameters to the human genome build hg38. For each potential off-target site, all indels within 3 bp of the potential Cas9 cleavage site are counted and divided by the coverage at the cut site and thus provides a quantity of indels at a particular cut site.

### 1.2.6. Statistical assessment of significant cut sites

Various events can lead to indels that are not a result of CRISPR-Cas9 being detected at sites throughout the genome: germline indel variants or polymorphisms, regions susceptible to genomic breaks, regions with homopolymer runs, and regions that are otherwise difficult to sequence

### 1.2.6.1. Sites excluded from analysis

We exclude from analysis: any sites with a "germline" indel on a donor-by-donor basis (donor has > 30% indel frequency in every sample), any chromosome Y sites in female samples, and any sites with 0 coverage.

### 1.2.6.2. Statistical test

To assess whether an indel seen at a potential off-target site is truly a CRISPR-Cas9 induced event, we test whether the samples treated with Cas9 and guide have a significantly higher frequency of indels than the untreated samples using both Mann-Whitney Wilcoxon test and Student's t-test. If either of these tests is significant (p < 0.05) we consider the site flagged for follow-up with PCR to determine if there is significant editing. To ensure that we flag sites for follow-up as aggressively as possible, we do not perform multiple hypothesis testing correction, which would decrease the number of sites that we find significant.

We also establish a negative control analysis, where we repeat the analysis, except we look for sites with higher frequency of indels in the untreated sample than the treated sample. Biologically, there is no reason we would expect to find "true hits" in this analysis, which provides us empirical information about the number of false positives we can expect to find in this dataset that can be attributable to background noise. Furthermore, we can expand this into an empirical null distribution by leveraging an additional two negative control samples, including cells electroporated with no Cas9 or guide, and cells electroporated with Cas9 and a GFP guide. By testing for hits in samples that are "less treated" compared to samples that are "more treated", we determine a conservative empirical null distribution of false positive hits, which can be used to inform the believability of the hits in our original analysis for treated vs untreated samples.

Human mPB CD34+ cells edited with SD2 gRNA and human mPB CD34+ cells edited with SPY101 gRNA were analyzed via a hybrid capture sequencing method described herein. The results presented in Figures 26-27 were from 3-4 different donors and demonstrated 0 off-target sites with evidence of cutting in gene-edited mPB CD34+ cells, which were edited with SD2 gRNA (Figure 27) and gene-edited mPB CD34+ cells edited with SPY101 gRNA (Figure 26). The indel frequency for round 1 is greater than (>) 0.5%. The indel frequencey for round 2 is greater than (>) 0.2%.

### Example 20 - Engraftment Experiments

Mobilized human peripheral blood (mPB) CD34+ cells were isolated from healthy donors using CliniMACS CD34 microbeads with the CliniMACS Prodigy (Miltenyi Biotec) and cultured in serum free CellGro^{®} media including 100 ng/ml recombinant human stem cell factor (SCF), 100 ng/ml recombinant human Fit 3-Ligand (FLT3L), and 100 ng/ml Thrombopoietin (TPO). The cells were then electroporated using a Maxcyte^{®} device following the manufacture's instructions with one of the following: an empty vector that does not contain any CRISPR/Cas9 editing components (mock electroporation sample), with GFP gRNA and Cas9 protein as a negative control (GFP), with SPY101 gRNA and Cas9 protein (SPY101), or with SD2 gRNA and Cas9 protein (SD2). The recombinant Cas9 protein encodes for *S. pyogenes* Cas9 flanked by two SV40 nuclear localization sequences (NLSs). These experiments were performed using a ribonucleoprotein (RNP) 1:1 weight ratio of gRNA to Cas9.

Each of the gene-edited mPB CD34+ human cells were injected via tail vein into 16 immunodeficient mice ("NSG" or NOD scid gamma -NOD) to demonstrate homing and engraftment capabilities. NSG is a strain of inbred laboratory mice and among the most immunodeficient described to date; see, *e.g.,* Shultz *et al., Nat. Rev. Immunol. 7*(2): 118-130 (2007). Details related to the engraftment experiment are presented in Figure 28. At 8-weeks post injection, the NSG mice were bled and the peripheral blood was analyzed via FACS for human CD45RA+ and mouse CD45+ live cells. At 16-weeks post injection, the NSG mice were sacrificed, and the bone marrow, spleen, and perifpheral blood were analyzed via FACS for human CD45RA+ and mouse CD45+ live cells. Engraftment of mPB CD34+ human cells in irradiated NSG mice in all threatment groups was observed from all three healthy donors. Human CD45RA+ cells were detected using FACS in all 3 hematopoietic organs from all donors. Untransfected CD34+ control cells exhibited slighlty better engraftment percentages. All transfected cell groups had similar engraftment percentages, including the mock transfected group across all 3 healthy donors. In general, the addition of Cas9-gRNA RNP did not affect engraftment as compared to the mock transfection control (Figures 29A-E and Figure 30). Data points in Figures 29A-E represent individual mice and depict the percentage of live cells that were human CD45RA+ cells. Data is mean+SEM.

### Example 21 - Assessing SPY101 editing efficiency and efficacy using Cas9 RNP

In order to achieve the highest efficacy using CRISPR-Cas9 for treating SCD and β-thalassemia using SPY101, we assessed two different Cas9 formats, Cas9 mRNA or Cas9 protein, for their editing efficiency, efficacy and toxicity in human CD34+ cells from mobilized peripheral blood (mPB). We compared various sources for Cas9 mRNA to Cas9 protein by electroporating Cas9 mRNA and SPY101 gRNA or Cas9 protein complexed with SPY101 gRNA (as ribonucleoprotein (RNP) complex) into human mPB CD34+ cells and assessed for their editing efficiency and cellular viability at 48 hours post-electroporation. We compared various sources for Cas9 mRNA to Cas9 protein and found that while we can achieve similar levels of editing efficiency between some Cas9 mRNA to Cas9 protein (Figure 31), most had significantly lower cell viability compared to control samples (No electroporation (No EP) or No substrate electroporation (Mock EP) controls) shown in Figures 32A-B, This indicates that Cas9 RNP is the best format to use for efficient delivery of Cas9 and gRNA into human mPB CD34+ cells.

We next compared different sources of Cas9 protein as well as Cas9 protein with varying number of nuclear localization signal (NLS) at either N or C-terminus as this can affect efficient localization of Cas9 into the nucleus to afford editing. Shown in Figures 33A-C, we found that Aldevron Cas9 protein with one NLS at both N and C terminus gave the best editing efficiency with no change in cell viability.

Next, we compiled SPY101 editing efficiency examined across various human mPB CD34+ donors using either Cas9 mRNA or Cas9 protein (Feldan or Aldevron chosen from previous example) and observed that Aldevron Cas9 protein resulted in the highest editing efficacy (Figure 34A). Furthermore, we were able to achieve similar rates of editing efficiency in GMP-compatible manufacturing at clinical scale using Cas9 protein (Figure 34B).

We next examined SPY101 efficacy across several CD34+ donors derived from mPB (Figures 35A-B) or bone marrow (BM, Figures 36A-B). We see that throughout our optimization process, we achieved better efficacy, measured as γ-globin expression to α-globin or to β-globin like globins (β-globin + γ-globin) by quantitative real-time PCR in erythroid differentiated CD34+ cells.

We then investigated whether SPY101 would be efficacious in cells obtained from SCD or β-thalassemia patients. Peripheral blood mononuclear cells from healthy donors or patients were electroporated with SPY101 Cas9 RNP and erythroid differentiated similar to examples above prior to extracting RNA to measure γ-globin expression. We see that SPY101 was indeed efficacious in γ-globin increase in patient samples (Figures 37A-B).

In order to better understand a genotype to phenotype relationship in SPY101 edited erythroid cells, we performed single colony analysis similar to Example 15 with Cas9 RNP and found that this increase a greater fraction of bi-allelic edited colonies using Cas9 RNP compared to Cas9 mRNA (Figures 38A-B). Furthermore, detailed breakdown of unedited colonies, mono-allelic disruption of GATA1 binding site targeted by SPY101 and bi-allelic disruption of GATA1 binding site revealed in dose-dependent efficacy of SPY101, measured as γ-globin increase compared to control GFP gRNA treated cells (Figures 39A-B).

To examine the percentage of cells expressing γ-globin, we performed FACS analysis in SPY101 Cas9 RNP edited CD34+ cells from human mPB. Compared to control GFP gRNA treated cells, we see a higher percentage of erythroid differentiated cells expressing γ-globin (Figures 40A-D), as well as an increase of γ-globin expression per cell (Figure 40E) in SPY101 treated cells.

## Claims

1. A single-molecule guide RNA (sgRNA) comprising the nucleic acid sequence of SEQ ID NO: 71,959, wherein the sgRNA comprises three 2'O-methyl-phosphorothioate residues at each of its 5' and 3' ends.

2. A single-molecule guide RNA (sgRNA) comprising the nucleic acid sequence of SEQ ID NO: 71,959, wherein bases 1-3 and 97-99 are modified 2'-O-methyl phosphorothioate nucleotides.

3. A nucleic acid comprising a nucleotide sequence encoding the sgRNA of claim 1 or 2.

4. A vector comprising the nucleic acid of claim 3.

5. The sgRNA of claim 1 or 2, the nucleic acid of claim 3, or the vector of claim 4, for use in treating a patient with hemoglobinopathy.

6. The sgRNA for use according to claim 5, the nucleic acid for use according to claim 5, or the vector for use of claim 5, wherein the hemoglobinopathy is sickle cell anemia or thalassemia.

7. The sgRNA for use according to claim 6, the nucleic acid for use according to claim 6, or the vector for use of claim 6, wherein the hemoglobinopathy is a thalassemia and the thalassemia is selected from the group consisting of α, β, δ, γ, and combinations thereof, further optionally wherein the thalassemia is β-thalassemia.

8. An *ex vivo* method of editing a B-cell lymphoma 11A (BCL11A) gene in a human cell by genome editing, the method comprising:
introducing into the human cell one or more deoxyribonucleic acid (DNA) endonucleases or a nucleic acid comprising a nucleotide sequence encoding the one or more DNA endonucleases or a vector comprising said nucleic acid to effect one or more single-strand breaks (SSBs) or double-strand breaks (DSBs) within or near the BCL11A gene or other DNA sequence that encodes a regulatory element of the BCL11A gene, that results in a permanent deletion, modulation, or inactivation of a transcriptional control sequence of the BCL11A gene,
wherein the method further comprises introducing into the cell the sgRNA of claim 1 or claim 2, a nucleic acid comprising a nucleotide sequence encoding the nucleic acid sequence of said sgRNA, or a vector comprising said nucleic acid, and wherein the one or more DNA endonucleases comprises a Cas9 endonuclease.

9. The method of claim 8, wherein the nucleic acid comprising a nucleotide sequence encoding the one or more DNA endonucleases is one or more ribonucleic acids (RNAs).

10. The method of claim 8 or 9, wherein the one or more DNA endonucleases each comprise, at the N-terminus, the C-terminus, or both the N-terminus and C-terminus, one or more nuclear localization signals (NLSs), optionally wherein the one or more NLSs comprises a SV40 NLS.

11. The method of claim 8 or 10, wherein the one or more DNA endonucleases is pre-complexed with the sgRNA to form one or more ribonucleoproteins (RNPs), optionally wherein the one or more DNA endonucleases is combined with the sgRNA in a weight ratio of 1:1 DNA endonuclease to sgRNA to form the one or more RNPs.

12. The method of claim 11, wherein the one or more RNPs are delivered to the human cell by electroporation, optionally wherein the one or more DNA endonucleases is a *S. pyogenes* Cas9 comprising a N-terminus SV40 NLS and a C-terminus SV40 NLS.

13. The method of any one of claims 8-12, wherein the human cell is a hematopoietic progenitor cell, optionally wherein the hematopoietic progenitor cell is a CD34+ cell.

## Patentansprüche

1. Single Molecule Guide RNA (sgRNA), umfassend die Nucleinsäuresequenz von SEQ ID NO:71.959, wobei die sgRNA drei 2'O-Methylphosphorothioat-Reste an jedem ihrer 5'- und 3'-Enden umfasst.

2. Single Molecule Guide RNA (sgRNA), umfassend die Nucleinsäuresequenz von SEQ ID NO:71.959, wobei die Basen 1-3 und 97-99 modfizierte 2'O-Methylphosphorothioat-Nucleotide sind.

3. Nucleinsäure, umfassend eine Nucleotidsequenz, die die sgRNA nach Anspruch 1 oder 2 codiert.

4. Vektor, umfassend die Nucleinsäure nach Anspruch 3.

5. sgRNA nach Anspruch 1 oder 2, Nucleinsäure nach Anspruch 3 oder Vektor nach Anspruch 4 zur Verwendung bei der Behandlung eines Patienten mit Hämoglobinopathie.

6. sgRNA zur Verwendung nach Anspruch 5, Nucleinsäure zur Verwendung nach Anspruch 5 oder Vektor zur Verwendung nach Anspruch 5, wobei die Hämoglobinopathie Sichelzellanämie oder Thalassämie ist.

7. sgRNA zur Verwendung nach Anspruch 6, Nucleinsäure zur Verwendung nach Anspruch 6 oder Vektor zur Verwendung nach Anspruch 6, wobei die Hämoglobinopathie eine Thalassämie ist und die Thalassämie ausgewählt ist aus der Gruppe bestehend aus α, β, δ, γ und Kombinationen davon, des Weiteren gegebenenfalls wobei die Thalassämie β-Thalassämie ist.

8. *Ex vivo*-Verfahren zum Editieren eines B-Zell-Lymphom 11A (BCL11A)-Gens in einer humanen Zelle durch Genom-Editierung, wobei das Verfahren umfasst:
Einbringen in die humane Zelle einer oder mehrerer Desoxyribonucleinsäure (DNA)-Endonuclease(n) oder einer Nucleinsäure, die eine Nucleotidsequenz umfasst, die die eine oder die mehreren DNA-Endonuclease(n) codiert, oder eines Vektors, der die Nucleinsäure umfasst, um einen oder mehrere Einzelstrangbrüche (single-strand breaks; SSBs) oder Doppelstrangbrüche (double-strand breaks; DSBs) innerhalb oder nahe des BCL11A-Gens oder einer anderen DNA-Sequenz, die ein regulatorisches Element des BCL11A-Gens codiert, zu bewirken, was zur permanenten Deletion, Modulation oder Inaktivierung einer transkriptionellen Kontrollsequenz des BCL11AGens führt,
wobei das Verfahren des Weiteren das Einbringen in die Zelle der sgRNA nach Anspruch 1 oder Anspruch 2, einer Nucleinsäure, die eine Nucleotidsequenz umfasst, die die Nucleinsäuresequenz der sgRNA codiert, oder eines die Nucleinsäure umfassenden Vektors umfasst, und wobei die eine oder die mehreren DNA-Endonuclease(n) eine Cas9-Endonuclease umfasst/umfassen.

9. Verfahren nach Anspruch 8, wobei die Nucleinsäure, die eine Nucleotidsequenz umfasst, die eine oder die mehreren DNA-Endonuclease(n) codiert, eine oder mehrere Ribonucleinsäuren (RNAs) ist/sind.

10. Verfahren nach Anspruch 8 oder 9, wobei die eine oder die mehreren DNA-Endonuclease(n) jeweils am N-Terminus, am C-Terminus oder sowohl am N-Terminus als auch am C-Terminus ein oder mehrere Kernlokalisierungssignal(e) (nuclear localization signals; NLSs) umfasst/umfassen, gegebenenfalls wobei das eine oder die mehreren NLSs ein SV40 NLS umfasst/umfassen.

11. Verfahren nach Anspruch 8 oder 10, wobei die eine oder die mehreren DNA-Endonuclease(n) mit der sgRNA prä-komplexiert ist/sind, um ein oder mehrere Ribonucleoprotein(e) (RNPs) zu bilden, gegebenenfalls wobei die eine oder die mehreren DNA-Endonuclease(n) mit der sgRNA in einem Gewichtsverhältnis von 1:1 DNA-Endonuclease zu sgRNA kombiniert ist/sind, um das eine oder die mehreren RNPs zu bilden.

12. Verfahren nach Anspruch 11, wobei das eine oder die mehreren RNPs durch Elektroporation an die humane Zelle abgegeben werden, gegebenenfalls wobei die eine oder die mehreren DNA-Endonuclease(n) eine *S*. *pyogenes-*Cas9 ist/sind, die ein N-Terminus-SV40 NLS und ein C-Terminus-SV 40 NLS umfasst.

13. Verfahren nach einem der Ansprüche 8 bis 12, wobei die humane Zelle eine hämatopoetische Vorläuferzelle ist, gegebenenfalls wobei die hämatopoetische Vorläuferzelle eine CD34+-Zelle ist.

## Revendications

1. ARN guide monomoléculaire (ARNsg) comprenant la séquence d'acide nucléique de la SEQ ID NO : 71 959, lequel ARNsg comprend trois résidus de 2'-O-méthyl-phosphorothioate à chacune de ses extrémités 5' et 3'.

2. ARN guide monomoléculaire (ARNsg) comprenant la séquence d'acide nucléique de la SEQ ID NO : 71 959, dans lequel les bases 1-3 et 97-99 sont des nucléotides 2'-O-méthyl-phosphorothioate modifiés.

3. Acide nucléique comprenant une séquence de nucléotides codant l'ARNsg de la revendication 1 ou 2.

4. Vecteur comprenant l'acide nucléique de la revendication 3.

5. ARNsg selon la revendication 1 ou 2, acide nucléique selon la revendication 3, ou vecteur selon la revendication 4, pour une utilisation dans le traitement d'un patient atteint d'une hémoglobinopathie.

6. ARNsg pour une utilisation selon la revendication 5, acide nucléique pour une utilisation selon la revendication 5, ou vecteur pour une utilisation selon la revendication 5, dans lequel l'hémoglobinopathie est une drépanocytose ou une thalassémie.

7. ARNsg pour une utilisation selon la revendication 6, acide nucléique pour une utilisation selon la revendication 6, ou vecteur pour une utilisation selon la revendication 6, dans lequel l'hémoglobinopathie est une thalassémie et la thalassémie est choisie dans le groupe constitué par les α, β, δ, γ et leurs combinaisons, en outre éventuellement dans lequel la thalassémie est une β-thalassémie.

8. Méthode *ex vivo* d'édition d'un gène de lymphome à cellules B 11A (BCL11A) dans une cellule humaine par édition génomique, la méthode comprenant :
l'introduction dans la cellule humaine d'une ou plusieurs acide désoxyribonucléique (ADN) endonucléases ou d'un acide nucléique comprenant une séquence de nucléotides codant une ou plusieurs ADN endonucléases ou un vecteur comprenant ledit acide nucléique pour réaliser une ou plusieurs cassures simple brin (SSB) ou cassures double brin (DSB) à l'intérieur ou à proximité du gène BCL11A ou d'une autre séquence d'ADN qui code un élément régulateur du gène BCL11A, ayant pour résultat une délétion, une modulation ou une inactivation permanente d'une séquence de contrôle de transcription du gène BCL11A,
laquelle méthode comprend en outre l'introduction dans la cellule de l'ARNsg de la revendication 1 ou 2, d'un acide nucléique comprenant une séquence de nucléotides codant la séquence d'acide nucléique dudit ARNsg, ou d'un vecteur comprenant ledit acide nucléique, et dans laquelle ladite une ou plusieurs ADN endonucléases comprennent une endonucléase Cas9.

9. Méthode selon la revendication 8, dans laquelle l'acide nucléique comprenant une séquence de nucléotides codant ladite une ou plusieurs ADN endonucléases est un ou plusieurs acides ribonucléiques (ARN).

10. Méthode selon la revendication 8 ou 9, dans laquelle chacune des une ou plusieurs ADN endonucléases comprend, à l'extrémité N, à l'extrémité C, ou à la fois à l'extrémité N et à l'extrémité C, un ou plusieurs signaux de localisation nucléaire (NLSs), éventuellement dans laquelle ledit un ou plusieurs NLSs comprennent un NLS SV40.

11. Méthode selon la revendication 8 ou 10, dans laquelle ladite une ou plusieurs ADN endonucléases sont pré-complexées avec l'ARNsg pour former une ou plusieurs ribonucléoprotéines (RNPs), éventuellement dans laquelle ladite une ou plusieurs ADN endonucléases sont combinées avec l'ARNsg en un rapport en poids d'ADN endonucléase à ARNsg de 1/1 pour former ladite une ou plusieurs RNPs.

12. Méthode selon la revendication 11, dans laquelle ladite une ou plusieurs RNPs sont délivrées à la cellule humaine par électroporation, éventuellement dans laquelle ladite une ou plusieurs ADN endonucléases sont une Cas9 de *S. pyogenes* comprenant un NLS SV40 à l'extrémité N et un NLS SV40 à l'extrémité C.

13. Méthode selon l'une quelconque des revendications 8 à 12, dans laquelle la cellule humaine est une cellule progénitrice hématopoïétique, éventuellement dans laquelle la cellule progénitrice hématopoïétique est une cellule CD34+.
